# EUROPEAN PATENT APPLICATION

(11) **EP 3 175 864 A1**
(43) Date of publication of application: **07.06.2017**
(21) Application number: 15810570.0
(22) Date of filing: 08.05.2015
(51) Int. Cl.: A61K 39/02, A61K 39/116, A61P 31/04

(54) **COMPLEX OF IMMUNOGENIC POLYPROTEINS OF M. HYOPNEUMONIAE, SYNTHETIC GENE ENCODING THE COMPLEX OF IMMUNOGENIC POLYPROTEINS OF M. HYOPNEUMONIAE, IMMUNOGENIC COMPOSITION, METHOD FOR PRODUCING A COMPLEX OF IMMUNOGENIC POLYPROTEINS OF M. HYOPNEUMONIAE, USE OF A COMPOSITION BASED ON THE COMPLEX OF IMMUNOGENIC POLYPROTEINS OF M. HYOPNEUMONIAE**

(30) Priority: 16.06.2014 BR 102014014727
(71) Applicant: Ouro Fino Saúde Animal Ltda., SP (São Paulo) (BR)
(72) Inventor: ROMERO RAMOS, Celso Raul, 14020-380 Ribeirão Preto - SP (BR); MARROQUIN QUELOPANA, Miryam, 14020-380 Ribeirão Preto - SP (BR)
(74) Representative: Herzog, Fiesser & Partner Patentanwälte PartG mbB
(86) International application number: PCT/BR2015/050056
(87) International publication number: WO 2015/192196

(57) **Abstract**

The present invention relates to a complex of polyproteins containing immunogenic regions derived from the P36, P46, HSP, NrdF, P97, MHP271 e P216 antigens of *M. hyopneumoniae,* which regions are fused with segments of the Rrp4. Rrp41 and Rrp42 carrier proteins of P. abyssi, forming a single complex. The present invention further provide the following objects: (i) a gene coding the polyprotein complex, which complex is based on the exosome of *P. abyssi* and carries copies of vaccine antigens of *M. hyopneumoniae;* (ii) an antigenic composition comprising the polyprotein complex of the invention, optionally one or more auxiliary agents and/or additives and a pharmacologically acceptable excipient; (iii) a method for producing said polyprotein complex; and (iv) uses of said complex and composition in the treatment and prevention of disease caused by *M. hyopneumoniae* infection.

SEQUENCE LISTING

<110>  OURO FINO SAUDE ANIMAL LTDA

<120>  Complex of immunogenic polyproteins of M. hyopneumoniae, Encoding
synthetic gene of complex of immunogenic polyproteins of M. hyopneumoniae,
antigenic composition, Process for obtaining
       a complex of a immunigenic polyprotein of M. hyopneumoniae,  Use of a
composition based on a complex of immnogenic polyproteins of M.
hyopneumoniae"

<130>  0003/0143-PAT

<150>  BR 10 2014 014727 6
<151>  2014-06-16

<160>  36

<170>  PatentIn version 3.5

<210>  1
<211>  314
<212>  PRT
<213>  Mycoplasma hyopneumoniae

<400>  1

Lys Pro Ile Lys Ile Ala Leu Ile Gly Ala Gly Asn Val Gly Asn Ser
1               5                   10                  15

Phe Leu Tyr Ala Ala Met Asn Gln Gly Leu Ala Ser Glu Tyr Gly Ile
            20                  25                  30

Ile Asp Ile Asn Pro Asp Phe Ala Asp Gly Asn Ala Phe Asp Phe Glu
        35                  40                  45

Asp Ala Ser Ala Ser Leu Pro Phe Pro Ile Ser Val Ser Arg Tyr Glu
    50                  55                  60

Tyr Lys Asp Leu Lys Asp Ala Asp Phe Ile Val Ile Thr Ala Gly Arg
65                  70                  75                  80

Pro Gln Lys Pro Gly Glu Thr Arg Leu Glu Leu Val Ala Asp Asn Ile
                85                  90                  95

Arg Ile Ile Arg Glu Ile Ala Leu Lys Val Lys Glu Ser Gly Phe Ser
            100                 105                 110

Gly Ile Ser Ile Ile Val Ala Asn Pro Val Asp Ile Ile Thr Arg Ala
        115                 120                 125

Tyr Arg Asp Ala Ser Gly Phe Ser Asp Gln Lys Val Ile Gly Ser Gly
    130                 135                 140

Thr Val Leu Asp Thr Ala Arg Leu Gln Phe Ala Ile Ala Lys Arg Ala
145                 150                 155                 160

Lys Val Ser Pro Asn Ser Val Gln Ala Tyr Val Met Gly Glu His Gly
                165                 170                 175

Asp Ser Ser Phe Val Ala Tyr Ser Asn Ile Lys Ile Ala Gly Glu Tyr
            180                 185                 190

Phe Cys Ala Tyr Ser Lys Leu Thr Gly Ile Asp Ser Ser Asn Tyr Glu
        195                 200                 205

Lys Glu Leu Glu Tyr Pro Val Ser Arg Arg Ala Tyr Glu Ile Ile Asn
    210                 215                 220

Arg Lys Arg Ala Thr Phe Tyr Gly Ile Gly Ala Ala Ile Ala Lys Ile
225                 230                 235                 240

Val Ser Asn Ile Ile Lys Asp Thr Lys Asn Ile Met Ile Ala Gly Ala
                245                 250                 255

Asn Leu Arg Gly Glu Tyr Gly Phe His Gly Val Asn Ile Gly Val Pro
            260                 265                 270

Val Val Leu Gly Ala Asn Gly Ile Glu Lys Ile Ile Glu Ile Ser Leu
        275                 280                 285

Asn Asp Lys Glu Lys Glu Lys Phe Ala Lys Ser Val Ala Ile Ile Asp
    290                 295                 300

Lys Ile Tyr Gln Asp Ala Ile Lys Asn Ile
305                 310

<210>  2
<211>  368
<212>  PRT
<213>  Mycoplasma hyopneumoniae

<400>  2

Lys His Lys Val Ser Asn Asp Ser Ile Arg Ile Ala Leu Thr Asp Pro
1               5                   10                  15

Asp Asn Pro Arg Trp Ile Ser Ala Gln Lys Asp Ile Ile Ser Tyr Val
            20                  25                  30

Asp Glu Thr Glu Ala Ala Thr Ser Thr Ile Thr Lys Asn Gln Asp Ala
        35                  40                  45

Gln Asn Asn Trp Leu Thr Gln Gln Ala Asn Leu Ser Pro Ala Pro Lys
    50                  55                  60

Gly Phe Ile Ile Ala Pro Glu Asn Gly Ser Gly Val Gly Thr Ala Val
65                  70                  75                  80

Asn Thr Ile Ala Asp Lys Gly Ile Pro Ile Val Ala Tyr Asp Arg Leu
                85                  90                  95

Ile Thr Gly Ser Asp Lys Tyr Asp Trp Tyr Val Ser Phe Asp Asn Glu
            100                 105                 110

Lys Val Gly Glu Leu Gln Gly Leu Ser Leu Ala Ala Gly Leu Leu Gly
        115                 120                 125

Lys Glu Asp Gly Ala Phe Asp Ser Ile Asp Gln Met Asn Glu Tyr Leu
    130                 135                 140

Lys Ser His Met Pro Gln Glu Thr Ile Ser Phe Tyr Thr Ile Ala Gly
145                 150                 155                 160

Ser Gln Asp Asp Asn Asn Ser Gln Tyr Phe Tyr Asn Gly Ala Met Lys
                165                 170                 175

Val Leu Lys Glu Leu Met Lys Asn Ser Gln Asn Lys Ile Ile Asp Leu
            180                 185                 190

Ser Pro Glu Gly Glu Asn Ala Val Tyr Val Pro Gly Trp Asn Tyr Gly
        195                 200                 205

Thr Ala Gly Gln Arg Ile Gln Ser Phe Leu Thr Ile Asn Lys Asp Pro
    210                 215                 220

Ala Gly Gly Asn Lys Ile Lys Ala Val Gly Ser Lys Pro Ala Ser Ile
225                 230                 235                 240

Phe Lys Gly Phe Leu Ala Pro Asn Asp Gly Met Ala Glu Gln Ala Ile
                245                 250                 255

Thr Lys Leu Lys Leu Glu Gly Phe Asp Thr Gln Lys Ile Phe Val Thr
            260                 265                 270

Gly Gln Asp Tyr Asn Asp Lys Ala Lys Thr Phe Ile Lys Asp Gly Asp
        275                 280                 285

Gln Asn Met Thr Ile Tyr Lys Pro Asp Lys Val Leu Gly Lys Val Ala
    290                 295                 300

Val Glu Val Leu Arg Val Leu Ile Ala Lys Lys Asn Lys Ala Ser Arg
305                 310                 315                 320

Ser Glu Val Glu Asn Glu Leu Lys Ala Lys Leu Pro Asn Ile Ser Phe
                325                 330                 335

Lys Tyr Asp Asn Gln Thr Tyr Lys Val Gln Gly Lys Asn Ile Asn Thr
            340                 345                 350

Ile Leu Val Ser Pro Val Ile Val Thr Lys Ala Asn Val Asp Asn Pro
        355                 360                 365

<210>  3
<211>  245
<212>  PRT
<213>  Mycoplasma hyopneumoniae

<400>  3

Gly Glu Ile Ser Asp Val Leu Leu Leu Asp Val Thr Pro Leu Thr Leu
1               5                   10                  15

Gly Ile Glu Thr Leu Gly Gly Ile Ala Thr Pro Leu Ile Pro Arg Asn
            20                  25                  30

Thr Thr Ile Pro Val Thr Lys Ser Gln Ile Phe Ser Thr Ala Glu Asp
        35                  40                  45

Asn Gln Thr Glu Val Thr Ile Ser Val Val Gln Gly Glu Arg Gln Leu
    50                  55                  60

Ala Ala Asp Asn Lys Met Leu Gly Arg Phe Asn Leu Ser Gly Ile Glu
65                  70                  75                  80

Ala Ala Pro Arg Gly Leu Pro Gln Ile Glu Val Ser Phe Ser Ile Asp
                85                  90                  95

Val Asn Gly Ile Thr Thr Val Ser Ala Lys Asp Lys Lys Thr Gly Lys
            100                 105                 110

Glu Gln Thr Ile Thr Ile Lys Asn Thr Ser Thr Leu Ser Glu Glu Glu
        115                 120                 125

Ile Asn Lys Met Ile Gln Glu Ala Glu Glu Asn Arg Glu Ala Asp Ala
    130                 135                 140

Leu Lys Lys Asp Lys Ile Glu Thr Thr Val Arg Ala Glu Gly Leu Ile
145                 150                 155                 160

Asn Gln Leu Glu Lys Ser Ile Thr Asp Gln Gly Glu Lys Ile Asp Pro
                165                 170                 175

Lys Gln Lys Glu Leu Leu Glu Lys Gln Ile Gln Glu Leu Lys Asp Leu
            180                 185                 190

Leu Lys Glu Asp Lys Thr Asp Glu Leu Lys Leu Lys Leu Asp Gln Ile
        195                 200                 205

Glu Ala Ala Ala Gln Ser Phe Ala Gln Ala Thr Ala Gln Gln Ala Asn
    210                 215                 220

Thr Ser Glu Ser Asp Pro Lys Ala Asp Asp Ser Asn Thr Ile Asp Ala
225                 230                 235                 240

Glu Ile Lys Gln Asp
                245

<210>  4
<211>  106
<212>  PRT
<213>  Mycoplasma hyopneumoniae

<400>  4

Lys Gln Asp Glu Ile Lys Lys Phe Thr Phe Asp Leu Leu Tyr Lys Leu
1               5                   10                  15

Ile Glu Leu Glu Lys Asp Tyr Leu Tyr Asp Leu Tyr Ser Glu Val Gly
            20                  25                  30

Leu Ala Glu Ser Ala Ile Lys Phe Ser Ile Tyr Asn Ala Gly Lys Phe
        35                  40                  45

Leu Gln Asn Leu Gly Tyr Asp Ser Pro Phe Ser Lys Glu Glu Thr Glu
    50                  55                  60

Ile Glu Pro Glu Ile Phe Ser Gln Leu Ser Ala Arg Ala Asp Glu Asn
65                  70                  75                  80

His Asp Phe Phe Ser Gly Asn Gly Ser Ser Tyr Val Met Ala Leu Ala
                85                  90                  95

Glu Glu Thr Glu Asp Glu Asp Trp Glu Phe
            100                 105

<210>  5
<211>  144
<212>  PRT

<213>  Mycoplasma hyopneumoniae

<400>  5

Ala Ala Lys Pro Glu Ala Ala Lys Pro Val Ala Ala Lys Pro Glu Thr
1               5                   10                  15

Thr Lys Pro Val Ala Ala Lys Pro Glu Ala Ala Lys Pro Glu Ala Ala
            20                  25                  30

Lys Pro Val Ala Ala Lys Pro Glu Ala Ala Lys Pro Val Ala Ala Lys
        35                  40                  45

Pro Glu Ala Ala Lys Pro Val Ala Ala Lys Pro Glu Ala Ala Lys Pro
    50                  55                  60

Val Ala Ala Lys Pro Glu Ala Ala Lys Pro Val Ala Thr Asn Thr Gly
65                  70                  75                  80

Phe Ser Leu Thr Asn Lys Pro Lys Glu Asp Tyr Phe Pro Met Thr Glu
                85                  90                  95

Gly Ser Ser Asn Gln Gly Lys Lys Ser Glu Gly Thr Pro Asn Gln Gly
            100                 105                 110

Lys Lys Ala Glu Gly Ala Pro Asn Gln Gly Lys Lys Ala Glu Gly Thr
        115                 120                 125

Pro Asn Gln Gly Lys Lys Ala Glu Gly Ala Pro Ser Gln Gln Ser Pro
    130                 135                 140

<210>  6
<211>  162
<212>  PRT
<213>  Mycoplasma hyopneumoniae

<400>  6

Ser Glu Phe Lys Glu Trp Arg Leu Asp Glu Ser Ala Phe Ser Ser Leu
1               5                   10                  15

Thr Phe Pro Lys Ser Gln Lys Ser Glu Gly Thr Gln Asn Gln Gly Lys
            20                  25                  30

Lys Ala Glu Gly Ala Pro Asn Gln Ser Lys Lys Ser Glu Glu Asn Ser
        35                  40                  45

Asn Lys Leu Thr Glu Tyr Ile Gln Glu Leu Gly Thr Lys Val Glu Lys
    50                  55                  60

Ser Leu Lys Ser Lys Gly Lys Asn Tyr Ser Ala Glu Val Glu Lys Ile
65                  70                  75                  80

Ile Glu Ala Phe Ser Gly Gly Tyr Lys Phe Leu Asp Phe Ala Leu Val
                85                  90                  95

Glu Gln Thr Pro Lys Pro Glu Thr Pro Lys Thr Glu Ala Ala Lys Pro
            100                 105                 110

Glu Thr Thr Lys Pro Val Ala Ala Arg Pro Glu Ala Ala Lys Val Ala
        115                 120                 125

Ala Lys Pro Ser Ala Ala Lys Pro Val Ser Ser Pro Ala Pro Lys Lys
    130                 135                 140

Ser Thr Leu Tyr Val Arg Val Leu Ile Arg Lys Lys Glu Asn Lys Gln
145                 150                 155                 160

Val Lys

<210>  7
<211>  25
<212>  PRT
<213>  Mycoplasma hyopneumoniae

<400>  7

Gln Lys Lys Ala Val Arg Lys Glu Glu Glu Leu Glu Thr Tyr Asn Pro
1               5                   10                  15

Lys Asp Glu Phe Asn Ile Leu Asn Pro
            20                  25

<210>  8
<211>  24
<212>  PRT
<213>  Mycoplasma hyopneumoniae

<400>  8

Val Asn Asn Asp Pro Asn Tyr Lys Ile Glu Asp Leu Lys Val Ile Lys
1               5                   10                  15

Asn Glu Ala Gly Asp His Gln Leu
            20

<210>  9
<211>  261
<212>  PRT
<213>  Pyrococcus abyssi

<400> 9

Lys Arg Ile Phe Val Gln Asn Arg Glu Leu Val Val Pro Gly Thr Leu
1               5                   10                  15

Leu Ala Gln Gly Pro Tyr Lys Asn Gly Arg Gly Thr Phe Arg Glu Gly
            20                  25                  30

Ser Arg Ile Tyr Ser Thr Val Ile Gly Leu Val Asp Ile Lys Gly Asn
        35                  40                  45

Thr Ile Arg Val Ile Pro Leu Glu Gly Pro Tyr Ile Pro Glu Val Gly
    50                  55                  60

Asp Asn Val Ile Gly Lys Ile Val Asp Val Lys Phe Ser Ser Trp Val
65                  70                  75                  80

Val Asp Ile Gly Ala Pro Tyr Pro Ala Asn Leu Lys Ile Gln Asp Phe
                85                  90                  95

Thr Asp Glu Lys Ile Asp Leu Leu Arg Thr Asp Leu Arg Lys Phe Phe
            100                 105                 110

Asp Ile Gly Asp Ile Ile Tyr Ala Lys Val Lys Ala Ile Thr Glu Val
        115                 120                 125

Asn Asn Ile Asp Leu Thr Thr Lys Gly Met Pro Phe Asn Gly Gly Pro
    130                 135                 140

Leu Lys Gly Gly Gln Ile Val Lys Ile Thr Pro Ser Arg Val Pro Arg
145                 150                 155                 160

Val Ile Gly Arg Gly Gly Ser Met Ile Asn Met Ile Lys Lys Leu Thr
                165                 170                 175

Met Thr Arg Ile Ile Val Gly Gln Asn Gly Trp Ile Trp Val Ser Gly
            180                 185                 190

Lys Asn Asp Ala Leu Glu Lys Leu Ala Ile Glu Ala Ile Leu Lys Ile
        195                 200                 205

Asp Lys Glu Ser His Thr Arg Gly Leu Thr Asp Arg Ile Lys Ala Leu
    210                 215                 220

Leu Leu Ser Arg Leu Gln Glu Leu Lys Glu Lys Gly Val Ile Glu Glu
225                 230                 235                 240

Ile Pro Lys Leu Glu Glu Glu Pro Gln Gly Glu Asp Glu Val Asn Gly

245                 250                 255

Asn Asp Gly Glu Ala
            260

<210>  10
<211>  244
<212>  PRT
<213>  Pyrococcus abyssi

<400>  10

Glu Lys Pro Glu Gly Leu Lys Leu Ile Asp Glu Asn Gly Arg Arg Ile
1               5                   10                  15

Asp Gly Arg Lys Lys Tyr Glu Leu Arg Pro Ile Lys Met Glu Val Gly
            20                  25                  30

Val Leu Lys Asn Ala Asn Gly Ser Ala Tyr Ile Glu Trp Gly Lys Asn
        35                  40                  45

Lys Ile Ile Ala Ala Val Tyr Gly Pro Arg Glu Leu His Pro Lys His
    50                  55                  60

Leu Gln Arg Pro Asp Arg Ala Ile Leu Arg Val Arg Tyr Asn Met Ala
65                  70                  75                  80

Pro Phe Ser Val Glu Glu Arg Lys Lys Pro Gly Pro Asp Arg Arg Ser
                85                  90                  95

Ile Glu Ile Ser Lys Val Ile Lys Gly Ala Leu Glu Pro Ala Leu Ile
            100                 105                 110

Leu Glu Met Phe Pro Arg Thr Ala Ile Asp Val Phe Ile Glu Val Leu
        115                 120                 125

Gln Ala Asp Ala Gly Thr Arg Val Ala Gly Ile Thr Ala Ala Ser Leu
    130                 135                 140

Ala Leu Ala Asp Ala Gly Ile Pro Met Arg Asp Leu Val Ala Ala Cys
145                 150                 155                 160

Ala Ala Gly Lys Ile Glu Gly Glu Ile Val Leu Asp Leu Asn Lys Glu
                165                 170                 175

Glu Asp Asn Tyr Gly Glu Ala Asp Val Pro Val Ala Ile Met Pro Leu
            180                 185                 190

Lys Asn Asp Ile Thr Leu Leu Gln Met Asp Gly Tyr Leu Thr Lys Asp

195                 200                 205

Glu Phe Ile Glu Ala Val Lys Leu Ala Ile Lys Gly Ala Lys Ala Val
    210                 215                 220

Tyr Gln Lys Gln Arg Glu Ala Leu Lys Glu Lys Tyr Leu Lys Ile Ala
225                 230                 235                 240

Gln Glu Val Glu

<210>  11
<211>  272
<212>  PRT
<213>  Pyrococcus abyssi

<400>  11

Ser Asp Asn Glu Ile Val Ala Gly Ile Met Arg Asp His Ile Ile Asn
1               5                   10                  15

Leu Leu Lys Glu Gly Lys Arg Ile Asp Asp Arg Gly Phe Glu Asp Tyr
            20                  25                  30

Arg Pro Ile Glu Ile Glu Val Gly Val Ile Glu Lys Ala Glu Gly Ser
        35                  40                  45

Ala Leu Val Lys Leu Gly Ser Thr Gln Val Leu Val Gly Ile Lys Thr
    50                  55                  60

Ser Leu Gly Glu Pro Phe Pro Asp Thr Pro Asn Met Gly Val Met Thr
65                  70                  75                  80

Thr Asn Val Glu Leu Val Pro Leu Ala Ser Pro Thr Phe Glu Pro Gly
                85                  90                  95

Pro Pro Asp Glu Arg Ala Ile Glu Leu Ala Arg Val Ile Asp Arg Gly
            100                 105                 110

Ile Arg Glu Ser Lys Ala Leu Asn Leu Glu Lys Met Val Ile Val Pro
        115                 120                 125

Gly Lys Ile Val Arg Val Val Phe Ile Asp Val His Val Leu Asp His
    130                 135                 140

Asp Gly Asn Leu Met Asp Ala Ile Gly Ile Ala Ala Ile Ala Ala Leu
145                 150                 155                 160

Leu Asn Ala Arg Val Pro Lys Val Arg Tyr Asn Glu Glu Thr Gly Glu

165                 170                 175

Val Glu Thr Leu Asp Glu Thr Glu Pro Leu Pro Val Glu Lys Ile Pro
            180                 185                 190

Val Pro Val Thr Phe Ala Lys Ile Gly Asn Ile Leu Val Val Asp Pro
        195                 200                 205

Ser Leu Asp Glu Glu Leu Val Met Asp Gly Lys Ile Thr Ile Thr Thr
    210                 215                 220

Asp Glu Thr Gly His Ile Ser Ala Val Gln Lys Ser Glu Gly Gly Ala
225                 230                 235                 240

Phe Lys Leu Glu Glu Val Met Tyr Ala Val Glu Thr Ala Phe Lys Lys
                245                 250                 255

Ala Glu Glu Ile Arg Lys Leu Ile Leu Glu Ala Val Glu Lys Ala Lys
            260                 265                 270

<210>  12
<211>  942
<212>  DNA
<213>  Artificial Sequence

<220>
<223>  Synthetic gene for P36 antigen

<400>  12
aaaccgatta agattgctct gattggtgca ggcaatgtag gcaactcttt cctgtacgcg      60

gcaatgaatc agggtctggc gtctgaatat ggtattattg atattaaccc ggatttcgct     120

gacggtaatg cattcgattt cgaggacgct tccgcttccc ttccgttccc aatttccgtt     180

tcccgttatg aatacaaaga cctgaaagat gcagacttta ttgttattac cgcgggccga     240

ccgcagaagc cgggtgagac ccgcctggaa ctggttgctg acaacattcg catcatccgc     300

gaaattgccc tgaaagtgaa agaaagcggt ttctctggta tttctattat tgtagctaac     360

ccggtagata ttatcacccg tgcttatcgc gatgcttccg gtttctccga ccaaaaagtt     420

atcggttccg gcaccgtgct ggacaccgcg cgtctgcaat tcgccattgc aaaacgcgcg     480

aaagttagcc cgaactcggt tcaagcatac gttatgggtg agcacggtga ctctagcttt     540

gtagcctaca gcaacattaa gattgcgggt gaatatttct ctgcgtacag caaactgacc     600

ggtattgaca gctctaacta cgaaaaagaa ctggaatacc cggtgtcccg tcgagcgtac     660

gaaattatta accgaaaacg tgcgactttc tatggtatcg gcgctgcaat tgcaaagatt     720

gtttctaaca tcattaaaga caccaagaac attatgattg ccggcgcgaa cctgcgcggt     780

gaatatggtt tccacggtgt aaacattggt gtgccggttg tactgggtgc aaacggcatc     840

gagaaaatta tcgaaatttc tctgaacgat aaagaaaaag aaaagttcgc aaaatccgta 900

gcgattattg ataaaatcta tcaggatgcg attaaaaaca tt 942

<210> 13
<211> 1104
<212> DNA
<213> Artificial Sequence

<220>
<223> Synthetic gene for P36 antigen

<400> 13
aaacacaaag tatctaacga ctccattcgc attgcactga ctgaccctga taacccgcgc 60

tggatttctg ctcagaaaga tattatttcc tatgttgatg aaaccgaggc ggctacttct 120

accattacca aaaaccagga tgcccagaac aactggctga cccagcaggc taacctgtcc 180

ccggcaccga aaggctttat tatcgctccg gaaaacggtt ctggtgtggg tactgccgtt 240

aacactattg ctgacaaagg tatccctatt gtagcttatg accgcctgat tactggtagc 300

gacaaatatg actggtacgt gtctttcgat aacgaaaagg ttggcgaact gcaaggcctg 360

tctctggcgg cgggtctgct gggtaaagag gacggtgctt ttgactctat tgaccagatg 420

aacgagtacc tgaaatctca catgccgcag gaaactatca gcttctacac cattgccggc 480

agccaggacg ataacaacag ccaatacttc tacaacggtg ccatgaaagt actgaaagag 540

ctgatgaaaa acagccagaa caaaattatt gacctgtctc cggaaggcga aaacgcggtt 600

tacgttccgg gttggaacta cggtactgcc ggtcaacgca ttcagtcttt cctgactatt 660

aacaaggacc cggccggtgg taataaaatt aaggctgtag gttccaagcc ggcgtctatc 720

tttaaaggtt tcctggcacc gaatgacggc atggcagagc aggcaattac caagctgaaa 780

ctggaaggct tcgataccca gaaaattttc gtaaccggtc aggattataa cgacaaagcg 840

aaaactttta ttaaagacgg cgaccagaac atgaccattt ataaaccgga caaagttctg 900

ggtaaagtgg cagtggaagt tctgcgtgtt ctgattgcga aaaaaaacaa agccagccga 960

agcgaagttg aaaacgagct gaaagcgaaa ctgccgaaca tctccttcaa gtatgataac 1020

cagacctaca aggtgcaagg caagaacatt aatactatcc tggtttctcc ggtgattgta 1080

accaaagcga atgtggacaa ccct 1104

<210> 14
<211> 735
<212> DNA
<213> Artificial Sequence

<220>
<223> Synthetic gene for the HSP antigen

<400> 14

ggtgaaattt ctgacgtgct gctgctggac gtaaccccgc tgaccctggg cattgagact 60

ctgggtggca ttgctacccc gctgattccg cgaaacacca ccattccagt gaccaaaagc 120

caaattttct ccaccgctga agataaccag accgaagtta ccatctccgt tgtgcaaggt 180

gaacgacagc tggcagccga caacaagatg ctgggtcgct ttaacctgag cggcatcgaa 240

gcggctccgc gtggtctgcc acaaattgaa gtgtccttca gcatcgatgt aaacggcatc 300

accactgttt ccgctaaaga caaaaaaacc ggtaaagaac agaccattac catcaagaat 360

acctccaccc tgtctgaaga agaaattaac aaaatgattc aggaagctga ggaaaaccgt 420

gaagccgatg cactgaaaaa agacaaaatt gaaaccaccg tacgtgcgga aggcctgatt 480

aaccagctgg aaaaatctat tactgaccag ggtgagaaaa ttgacccgaa acagaaagaa 540

ctgctggaaa agcagattca ggaactgaaa gacctgctga aagaggataa aaccgacgaa 600

ctgaaactga aactggacca gattgaagct gcggcccaat ccttcgcgca ggcaaccgcg 660

cagcaagcga acaccagcga atctgacccg aaagcggacg atagcaacac catcgacgcg 720

gagattaaac aagat 735

<210> 15
<211> 318
<212> DNA
<213> Artificial Sequence

<220>
<223> Synthetic gene for the NrdF antigen

<400> 15
aaacaggatg aaatcaaaaa attcaccttc gacctgctgt acaaactgat tgaactggaa 60

aaagattacc tgtacgacct gtactccgaa gtgggcctgg cggaaagcgc catcaagttc 120

agcatctaca acgctggcaa attcctgcaa aacctgggct acgattctcc gttttctaaa 180

gaagagaccg aaatcgagcc ggagattttc tcccagctga gcgctcgcgc agatgagaac 240

catgacttct tctctggtaa cggcagcagc tacgtgatgg cgctggcaga ggaaaccgaa 300

gacgaagatt gggagttc 318

<210> 16
<211> 441
<212> DNA
<213> Artificial Sequence

<220>
<223> Synthetic gene for the P97R1R2 antigen

<400> 16
gcggcgaaac cggaggcagc gaaaccggtt gcggctaaac cggagaccac caaaccggtg 60

gctgctaaac cggaagcggc caaaccggaa gctgcgaaac cggtggcggc gaagccggag 120

gctgccaaac cggtagctgc taagccggaa gcggctaagc cggtggcagc aaaaccggag 180

gcggcaaaac cggtggccgc caagccggaa gctgccaagc cggttgcaac caacactggt     240

ttttctctga ccaacaaacc gaaagaagac tactttccga tgaccgaagg ctccagcaac     300

cagggtaaaa agtccgaagg tactccgaac cagggtaaga aagcggaggg tgcaccgaac     360

cagggcaaaa aagcggaagg caccccgaac caaggtaaaa aagcagaggg tgcgccgagc     420

cagcagagcc cgaccaccga a                                               441

<210>  17
<211>  393
<212>  DNA
<213>  Artificial Sequence

<220>
<223>  Synthetic gene for the MHP271 antigen

<400>  17
ccgaaatccc aaaaatctga aggtactcaa aaccagggta aaaaagctga aggtgccccg      60

aatcagtcca aaaaaagcga ggaaaactcc aacaaactga ctgaatacat tcaagaactg     120

ggcactaaag tggagaagag cctgaaatct aaaggtaaaa actacagcgc agaagttgag     180

aagattattg aagcattcag cggcggctat aagttcctgg atttcgcgct ggttgaacag     240

accccgaaac cggaaacccc gaaaactgaa gctgctaaac cggaaactac taaaccggtg     300

gcggcgcgcc cggaagctgc gaaagttgct gcgaaaccgt ccgctgcgaa acctgtttcc     360

agcccggcgc cgaaaaaatc taccctgtac gtg                                  393

<210>  18
<211>  78
<212>  DNA
<213>  Artificial Sequence

<220>
<223>  Synthetic gene for the P216-A antigen

<400>  18
ggccagaaga aggcggtacg taaggaagag gaactggaaa cctacaaccc gaaagacgag      60

tttaatatcc tgaacccg                                                    78

<210>  19
<211>  72
<212>  DNA
<213>  Artificial Sequence

<220>
<223>  Synthetic gene for the P216-A antigen

<400>  19
gtgaacaacg acccgaacta caaaattgaa gacctgaagg tgattaagaa cgaggcaggt      60

gaccatcagc tt                                                          72

<210>  20
<211>  783
<212>  DNA
<213>  Artificial Sequence

<220>
<223>  Gene for the Rrp4 protein of the exosome of P. abyssi

<400>  20
aaacgtatct tcgtacagaa tcgtgaactg gtggtgccgg gtactctgct ggcccagggt      60

ccttataaaa acggtcgtgg cactttccgt gaaggtagcc gtatctactc taccgttatc     120

ggtctggtgg acatcaaagg caacaccatt cgtgtgattc ctctggaggg tccgtacatc     180

ccggaagtag gtgacaacgt tatcggtaaa attgtggacg ttaagttctc tagctgggtt     240

gtagacattg gtgcgccgta tccggccaac ttgaagattc aggacttcac tgatgaaaaa     300

attgatttgc tgcgtactga ccttcgtaag ttctttgata ttggtgacat tatctacgct     360

aaagtaaaag ccatcaccga agttaacaac attgacctga cgaccaaagg catgccgttt     420

aacggtggtc cgctgaaagg tggtcaaatc gtgaaaatta cgccgtctcg tgtgccgcgt     480

gttatcggtc gcggtggcag catgattaac atgattaaaa aactgaccat gactcgtatt     540

attgttggcc aaaacggctg gatttgggtg tctggcaaaa acgatgcgct ggaaaaactt     600

gctattgagg ccattctgaa aattgacaaa gagtctcata cccgtggtct gactgaccgt     660

attaaagcgc tgctgctgtc ccgcctgcaa gaactgaaag aaaaaggtgt tattgaagag     720

attccgaaac tggaagaaga gccgcagggc gaagatgaag taaacggcaa tgacggtgaa     780

gct                                                                   783


<210>  21
<211>  732
<212>  DNA
<213>  Artificial Sequence

<220>
<223>  Gene for Rrp41 protein of the exosome of the P. abyssi

<400>  21
gagaaaccgg aaggtctgaa actgattgat gaaaacggcc gccgtattga cggtcgtaaa      60

aaatacgaac tgcgtccgat taaaatggaa gtgggtgtac tgaaaaacgc taacggttcc     120

gcgtacatcg aatggggcaa aaacaaaatt atcgcagcgg tttacggtcc gcgtgaactc     180

cacccgaaac accttcagcg tccggaccgc gccatcctgc gtgtacgtta caacatggcc     240

ccgttctctg ttgaagagac taagagcccg ggtccggacc gtcgtagcat tgagatttct     300

aaagtaatta aaggtgcgct ggaaccggca cttattctgg aaatgttccc gcgtactgct     360

attgatgtat tcatcgaagt tctgcaagcg gatgccggta cgcgcgttgc aggtatcacc     420

gcggctagcc tggcactggc cgacgctggt attccgatgc gcgacctggt tgccgcttgt     480

gcggcgggta aaatcgaagg cgaaattgtt ctggacctga acaaagaaga agataactac 540

ggcgaagcgg acgtgccggt agctatcatg ccgctgaaga acgatattac cctgctgcaa 600

atggatggtt acctgaccaa ggacgagttc atcgaggcgg ttaaactggc tattaaaggt 660

gctaaggcgg tgtatcagaa acagcgtgaa gcactgaaag aaaagtatct gaaaattgcc 720

caggaagttg ag 732

<210> 22
<211> 819
<212> DNA
<213> Artificial Sequence

<220>
<223> Gene for the Rrp42 protein of the exosome of the P. abyssi

<400> 22
tctgataatg aaatcgtggc gggtattatg cgcgaccata ttattaacct gcttaaggaa 60

ggtaaacgta ttgacgaccg cggtttcgaa gattaccgcc cgattgaaat tgaggtaggt 120

gttattgaaa aagcggaggg tagcgcgctg gtgaaactgg gtagcactca ggttctggtg 180

ggtattaaaa cctctctggg cgagccgttt ccggacaccc cgaacatggg cgtgatgacc 240

accaatgttg aacttgttcc gctggcgtct ccgactttcg aaccgggccc gccggacgag 300

cgtgctatcg aactggcacg tgttattgac cgtggtattc gtgagtccaa agcactgaat 360

ctggaaaaaa tggttatcgt gccgggcaaa attgttcgcg tagtttttat cgacgttcac 420

gttcttgacc acgacggtaa cctgatggac gccatcggta ttgcagctat cgccgcactg 480

ctgaacgcac gtgttccgaa agttcgctac aacgaagaaa cgggtgaagt tgaaaccctg 540

gatgaaactg aaccgctgcc ggttgaaaag attccggttc cggttacttt cgctaaaatt 600

ggtaatatcc tggtagttga cccgagcctg gatgaagagc tcgtgatgga cggcaaaatt 660

accattacca ctgacgaaac cggccatatt tctgccgttc agaaatctga aggtggcgct 720

ttcaaactgg aagaagtaat gtacgcggtg gaaactgcat tcaagaaggc ggaagaaatc 780

cgtaaactga ttctggaagc tgttgagaag gccaaacag 819

<210> 23
<211> 145
<212> DNA
<213> Artificial Sequence

<220>
<223> Promotor for the gene of Rrp41

<400> 23
taactgcagg cttaagtcga acagaaagta atcgtattgt acacggccgc aacttcgaaa 60

ttaatacgac tcactatagg gagaccacaa cggtttccca tcttagtata ttagttaagt 120

ataagaagga gatataaaca tggca                                          145

<210>  24
<211>  144
<212>  DNA
<213>  Artificial Sequence

<220>
<223>  Promotor for the Rrp42 gene

<400>  24
taagaattcg cttaagtcga acagaaagta atcgtattgt acacggccgc ataatcgaaa      60

ttaatacgac tcactatagg gagaccacaa cggtttccca tcttagtata ttagttaagt     120

ataagaagga gatataccat gggt                                           144

<210>  25
<211>  6764
<212>  DNA
<213>  Artificial Sequence

<220>
<223>  Gene for the EXOMYC complex

<220>
<221>  CDS
<222>  (4)..(2217)
<223>  Polyprotein 1: Rrp4-P36-MHP271

<220>
<221>  CDS
<222>  (2354)..(4651)
<223>  Polyprotein 2: Rrp41-P46-P97R1R2

<220>
<221>  CDS
<222>  (4787)..(6760)
<223>  Polyprotein 3: Rrp42-HSP-NrdF

<400>  25
cat atg gcc aaa cgt atc ttc gta cag aat cgt gaa ctg gtg gtg ccg      48
    Met Ala Lys Arg Ile Phe Val Gln Asn Arg Glu Leu Val Val Pro
    1               5                   10                  15

ggt act ctg ctg gcc cag ggt cct tat aaa aac ggt cgt ggc act ttc      96
Gly Thr Leu Leu Ala Gln Gly Pro Tyr Lys Asn Gly Arg Gly Thr Phe
                20                  25                  30

cgt gaa ggt agc cgt atc tac tct acc gtt atc ggt ctg gtg gac atc     144
Arg Glu Gly Ser Arg Ile Tyr Ser Thr Val Ile Gly Leu Val Asp Ile
            35                  40                  45

aaa ggc aac acc att cgt gtg att cct ctg gag ggt ccg tac atc ccg     192
Lys Gly Asn Thr Ile Arg Val Ile Pro Leu Glu Gly Pro Tyr Ile Pro
        50                  55                  60

gaa gta ggt gac aac gtt atc ggt aaa att gtg gac gtt aag ttc tct     240
Glu Val Gly Asp Asn Val Ile Gly Lys Ile Val Asp Val Lys Phe Ser
    65                  70                  75

agc tgg gtt gta gac att ggt gcg ccg tat ccg gcc aac ttg aag att 288
Ser Trp Val Val Asp Ile Gly Ala Pro Tyr Pro Ala Asn Leu Lys Ile
80                  85                  90                  95

cag gac ttc act gat gaa aaa att gat ttg ctg cgt act gac ctt cgt 336
Gln Asp Phe Thr Asp Glu Lys Ile Asp Leu Leu Arg Thr Asp Leu Arg
                100                 105                 110

aag ttc ttt gat att ggt gac att atc tac gct aaa gta aaa gcc atc 384
Lys Phe Phe Asp Ile Gly Asp Ile Ile Tyr Ala Lys Val Lys Ala Ile
            115                 120                 125

acc gaa gtt aac aac att gac ctg acg acc aaa ggc atg ccg ttt aac 432
Thr Glu Val Asn Asn Ile Asp Leu Thr Thr Lys Gly Met Pro Phe Asn
        130                 135                 140

ggt ggt ccg ctg aaa ggt ggt caa atc gtg aaa att acg ccg tct cgt 480
Gly Gly Pro Leu Lys Gly Gly Gln Ile Val Lys Ile Thr Pro Ser Arg
    145                 150                 155

gtg ccg cgt gtt atc ggt cgc ggt ggc agc atg att aac atg att aaa 528
Val Pro Arg Val Ile Gly Arg Gly Gly Ser Met Ile Asn Met Ile Lys
160                 165                 170                 175

aaa ctg acc atg act cgt att att gtt ggc caa aac ggc tgg att tgg 576
Lys Leu Thr Met Thr Arg Ile Ile Val Gly Gln Asn Gly Trp Ile Trp
                180                 185                 190

gtg tct ggc aaa aac gat gcg ctg gaa aaa ctt gct att gag gcc att 624
Val Ser Gly Lys Asn Asp Ala Leu Glu Lys Leu Ala Ile Glu Ala Ile
            195                 200                 205

ctg aaa att gac aaa gag tct cat acc cgt ggt ctg act gac cgt att 672
Leu Lys Ile Asp Lys Glu Ser His Thr Arg Gly Leu Thr Asp Arg Ile
        210                 215                 220

aaa gcg ctg ctg ctg tcc cgc ctg caa gaa ctg aaa gaa aaa ggt gtt 720
Lys Ala Leu Leu Leu Ser Arg Leu Gln Glu Leu Lys Glu Lys Gly Val
    225                 230                 235

att gaa gag att ccg aaa ctg gaa gaa gag ccg cag ggc gaa gat gaa 768
Ile Glu Glu Ile Pro Lys Leu Glu Glu Glu Pro Gln Gly Glu Asp Glu
240                 245                 250                 255

gta aac ggc aat gac ggt gaa gct aga tct ggt gtg aac aac gac ccg 816
Val Asn Gly Asn Asp Gly Glu Ala Arg Ser Gly Val Asn Asn Asp Pro
                260                 265                 270

aac tac aaa att gaa gac ctg aag gtg att aag aac gag gca ggt gac 864
Asn Tyr Lys Ile Glu Asp Leu Lys Val Ile Lys Asn Glu Ala Gly Asp
            275                 280                 285

cat cag ctt ggc tcc aaa ccg att aag att gct ctg att ggt gca ggc 912
His Gln Leu Gly Ser Lys Pro Ile Lys Ile Ala Leu Ile Gly Ala Gly
        290                 295                 300

aat gta ggc aac tct ttc ctg tac gcg gca atg aat cag ggt ctg gcg 960
Asn Val Gly Asn Ser Phe Leu Tyr Ala Ala Met Asn Gln Gly Leu Ala
    305                 310                 315

tct gaa tat ggt att att gat att aac ccg gat ttc gct gac ggt aat 1008
Ser Glu Tyr Gly Ile Ile Asp Ile Asn Pro Asp Phe Ala Asp Gly Asn

320                 325                 330                 335

gca ttc gat ttc gag gac gct tcc gct tcc ctt ccg ttc cca att tcc      1056
Ala Phe Asp Phe Glu Asp Ala Ser Ala Ser Leu Pro Phe Pro Ile Ser
                340                 345                 350

gtt tcc cgt tat gaa tac aaa gac ctg aaa gat gca gac ttt att gtt      1104
Val Ser Arg Tyr Glu Tyr Lys Asp Leu Lys Asp Ala Asp Phe Ile Val
            355                 360                 365

att acc gcg ggc cga ccg cag aag ccg ggt gag acc cgc ctg gaa ctg      1152
Ile Thr Ala Gly Arg Pro Gln Lys Pro Gly Glu Thr Arg Leu Glu Leu
        370                 375                 380

gtt gct gac aac att cgc atc atc cgc gaa att gcc ctg aaa gtg aaa      1200
Val Ala Asp Asn Ile Arg Ile Ile Arg Glu Ile Ala Leu Lys Val Lys
    385                 390                 395

gaa agc ggt ttc tct ggt att tct att att gta gct aac ccg gta gat      1248
Glu Ser Gly Phe Ser Gly Ile Ser Ile Ile Val Ala Asn Pro Val Asp
400                 405                 410                 415

att atc acc cgt gct tat cgc gat gct tcc ggt ttc tcc gac caa aaa      1296
Ile Ile Thr Arg Ala Tyr Arg Asp Ala Ser Gly Phe Ser Asp Gln Lys
                420                 425                 430

gtt atc ggt tcc ggc acc gtg ctg gac acc gcg cgt ctg caa ttc gcc      1344
Val Ile Gly Ser Gly Thr Val Leu Asp Thr Ala Arg Leu Gln Phe Ala
            435                 440                 445

att gca aaa cgc gcg aaa gtt agc ccg aac tcg gtt caa gca tac gtt      1392
Ile Ala Lys Arg Ala Lys Val Ser Pro Asn Ser Val Gln Ala Tyr Val
        450                 455                 460

atg ggt gag cac ggt gac tct agc ttt gta gcc tac agc aac att aag      1440
Met Gly Glu His Gly Asp Ser Ser Phe Val Ala Tyr Ser Asn Ile Lys
    465                 470                 475

att gcg ggt gaa tat ttc tct gcg tac agc aaa ctg acc ggt att gac      1488
Ile Ala Gly Glu Tyr Phe Ser Ala Tyr Ser Lys Leu Thr Gly Ile Asp
480                 485                 490                 495

agc tct aac tac gaa aaa gaa ctg gaa tac ccg gtg tcc cgt cga gcg      1536
Ser Ser Asn Tyr Glu Lys Glu Leu Glu Tyr Pro Val Ser Arg Arg Ala
                500                 505                 510

tac gaa att att aac cga aaa cgt gcg act ttc tat ggt atc ggc gct      1584
Tyr Glu Ile Ile Asn Arg Lys Arg Ala Thr Phe Tyr Gly Ile Gly Ala
            515                 520                 525

gca att gca aag att gtt tct aac atc att aaa gac acc aag aac att      1632
Ala Ile Ala Lys Ile Val Ser Asn Ile Ile Lys Asp Thr Lys Asn Ile
        530                 535                 540

atg att gcc ggc gcg aac ctg cgc ggt gaa tat ggt ttc cac ggt gta      1680
Met Ile Ala Gly Ala Asn Leu Arg Gly Glu Tyr Gly Phe His Gly Val
    545                 550                 555

aac att ggt gtg ccg gtt gta ctg ggt gca aac ggc atc gag aaa att      1728
Asn Ile Gly Val Pro Val Val Leu Gly Ala Asn Gly Ile Glu Lys Ile
560                 565                 570                 575

atc gaa att tct ctg aac gat aaa gaa aaa gaa aag ttc gca aaa tcc      1776

Ile Glu Ile Ser Leu Asn Asp Lys Glu Lys Glu Lys Phe Ala Lys Ser
                580                 585                 590

gta gcg att att gat aaa atc tat cag gat gcg att aaa aac att ccg      1824
Val Ala Ile Ile Asp Lys Ile Tyr Gln Asp Ala Ile Lys Asn Ile Pro
            595                 600                 605

aaa tcc caa aaa tct gaa ggt act caa aac cag ggt aaa aaa gct gaa      1872
Lys Ser Gln Lys Ser Glu Gly Thr Gln Asn Gln Gly Lys Lys Ala Glu
        610                 615                 620

ggt gcc ccg aat cag tcc aaa aaa agc gag gaa aac tcc aac aaa ctg      1920
Gly Ala Pro Asn Gln Ser Lys Lys Ser Glu Glu Asn Ser Asn Lys Leu
    625                 630                 635

act gaa tac att caa gaa ctg ggc act aaa gtg gag aag agc ctg aaa      1968
Thr Glu Tyr Ile Gln Glu Leu Gly Thr Lys Val Glu Lys Ser Leu Lys
640                 645                 650                 655

tct aaa ggt aaa aac tac agc gca gaa gtt gag aag att att gaa gca      2016
Ser Lys Gly Lys Asn Tyr Ser Ala Glu Val Glu Lys Ile Ile Glu Ala
                660                 665                 670

ttc agc ggc ggc tat aag ttc ctg gat ttc gcg ctg gtt gaa cag acc      2064
Phe Ser Gly Gly Tyr Lys Phe Leu Asp Phe Ala Leu Val Glu Gln Thr
            675                 680                 685

ccg aaa ccg gaa acc ccg aaa act gaa gct gct aaa ccg gaa act act      2112
Pro Lys Pro Glu Thr Pro Lys Thr Glu Ala Ala Lys Pro Glu Thr Thr
        690                 695                 700

aaa ccg gtg gcg gcg cgc ccg gaa gct gcg aaa gtt gct gcg aaa ccg      2160
Lys Pro Val Ala Ala Arg Pro Glu Ala Ala Lys Val Ala Ala Lys Pro
    705                 710                 715

tcc gct gcg aaa cct gtt tcc agc ccg gcg ccg aaa aaa tct acc ctg      2208
Ser Ala Ala Lys Pro Val Ser Ser Pro Ala Pro Lys Lys Ser Thr Leu
720                 725                 730                 735

tac gtg taa ctgcaggctt aagtcgaaca gaaagtaatc gtattgtaca              2257
Tyr Val

cggccgcaac ttcgaaatta atacgactca ctatagggag accacaacgg tttcccatct    2317

tagtatatta gttaagtata agaaggagat ataaac atg gca gag aaa ccg gaa      2371
                                        Met Ala Glu Lys Pro Glu
                                                    740

ggt ctg aaa ctg att gat gaa aac ggc cgc cgt att gac ggt cgt aaa      2419
Gly Leu Lys Leu Ile Asp Glu Asn Gly Arg Arg Ile Asp Gly Arg Lys
    745                 750                 755

aaa tac gaa ctg cgt ccg att aaa atg gaa gtg ggt gta ctg aaa aac      2467
Lys Tyr Glu Leu Arg Pro Ile Lys Met Glu Val Gly Val Leu Lys Asn
760                 765                 770                 775

gct aac ggt tcc gcg tac atc gaa tgg ggc aaa aac aaa att atc gca      2515
Ala Asn Gly Ser Ala Tyr Ile Glu Trp Gly Lys Asn Lys Ile Ile Ala
                780                 785                 790

gcg gtt tac ggt ccg cgt gaa ctc cac ccg aaa cac ctt cag cgt ccg      2563
Ala Val Tyr Gly Pro Arg Glu Leu His Pro Lys His Leu Gln Arg Pro

            795                 800                 805

gac cgc gcc atc ctg cgt gta cgt tac aac atg gcc ccg ttc tct gtt 2611
Asp Arg Ala Ile Leu Arg Val Arg Tyr Asn Met Ala Pro Phe Ser Val
        810                 815                 820

gaa gag act aag agc ccg ggt ccg gac cgt cgt agc att gag att tct 2659
Glu Glu Thr Lys Ser Pro Gly Pro Asp Arg Arg Ser Ile Glu Ile Ser
    825                 830                 835

aaa gta att aaa ggt gcg ctg gaa ccg gca ctt att ctg gaa atg ttc 2707
Lys Val Ile Lys Gly Ala Leu Glu Pro Ala Leu Ile Leu Glu Met Phe
840                 845                 850                 855

ccg cgt act gct att gat gta ttc atc gaa gtt ctg caa gcg gat gcc 2755
Pro Arg Thr Ala Ile Asp Val Phe Ile Glu Val Leu Gln Ala Asp Ala
                860                 865                 870

ggt acg cgc gtt gca ggt atc acc gcg gct agc ctg gca ctg gcc gac 2803
Gly Thr Arg Val Ala Gly Ile Thr Ala Ala Ser Leu Ala Leu Ala Asp
            875                 880                 885

gct ggt att ccg atg cgc gac ctg gtt gcc gct tgt gcg gcg ggt aaa 2851
Ala Gly Ile Pro Met Arg Asp Leu Val Ala Ala Cys Ala Ala Gly Lys
        890                 895                 900

atc gaa ggc gaa att gtt ctg gac ctg aac aaa gaa gaa gat aac tac 2899
Ile Glu Gly Glu Ile Val Leu Asp Leu Asn Lys Glu Glu Asp Asn Tyr
    905                 910                 915

ggc gaa gcg gac gtg ccg gta gct atc atg ccg ctg aag aac gat att 2947
Gly Glu Ala Asp Val Pro Val Ala Ile Met Pro Leu Lys Asn Asp Ile
920                 925                 930                 935

acc ctg ctg caa atg gat ggt tac ctg acc aag gac gag ttc atc gag 2995
Thr Leu Leu Gln Met Asp Gly Tyr Leu Thr Lys Asp Glu Phe Ile Glu
                940                 945                 950

gcg gtt aaa ctg gct att aaa ggt gct aag gcg gtg tat cag aaa cag 3043
Ala Val Lys Leu Ala Ile Lys Gly Ala Lys Ala Val Tyr Gln Lys Gln
            955                 960                 965

cgt gaa gca ctg aaa gaa aag tat ctg aaa att gcc cag gaa gtt gag 3091
Arg Glu Ala Leu Lys Glu Lys Tyr Leu Lys Ile Ala Gln Glu Val Glu
        970                 975                 980

gga tcc cct aaa cac aaa gta tct aac gac tcc att cgc att gca ctg 3139
Gly Ser Pro Lys His Lys Val Ser Asn Asp Ser Ile Arg Ile Ala Leu
    985                 990                 995

act gac cct gat aac ccg cgc tgg att tct gct cag aaa gat att 3184
Thr Asp Pro Asp Asn Pro Arg Trp Ile Ser Ala Gln Lys Asp Ile
1000                1005                1010

att tcc tat gtt gat gaa acc gag gcg gct act tct acc att acc 3229
Ile Ser Tyr Val Asp Glu Thr Glu Ala Ala Thr Ser Thr Ile Thr
1015                1020                1025

aaa aac cag gat gcc cag aac aac tgg ctg acc cag cag gct aac 3274
Lys Asn Gln Asp Ala Gln Asn Asn Trp Leu Thr Gln Gln Ala Asn
1030                1035                1040

ctg tcc ccg gca ccg aaa ggc ttt att atc gct ccg gaa aac ggt 3319

Leu Ser Pro Ala Pro Lys Gly Phe Ile Ile Ala Pro Glu Asn Gly
1045                1050                1055

tct ggt gtg ggt act gcc gtt aac act att gct gac aaa ggt atc 3364
Ser Gly Val Gly Thr Ala Val Asn Thr Ile Ala Asp Lys Gly Ile
1060                1065                1070

cct att gta gct tat gac cgc ctg att act ggt agc gac aaa tat 3409
Pro Ile Val Ala Tyr Asp Arg Leu Ile Thr Gly Ser Asp Lys Tyr
1075                1080                1085

gac tgg tac gtg tct ttc gat aac gaa aag gtt ggc gaa ctg caa 3454
Asp Trp Tyr Val Ser Phe Asp Asn Glu Lys Val Gly Glu Leu Gln
1090                1095                1100

ggc ctg tct ctg gcg gcg ggt ctg ctg ggt aaa gag gac ggt gct 3499
Gly Leu Ser Leu Ala Ala Gly Leu Leu Gly Lys Glu Asp Gly Ala
1105                1110                1115

ttt gac tct att gac cag atg aac gag tac ctg aaa tct cac atg 3544
Phe Asp Ser Ile Asp Gln Met Asn Glu Tyr Leu Lys Ser His Met
1120                1125                1130

ccg cag gaa act atc agc ttc tac acc att gcc ggc agc cag gac 3589
Pro Gln Glu Thr Ile Ser Phe Tyr Thr Ile Ala Gly Ser Gln Asp
1135                1140                1145

gat aac aac agc caa tac ttc tac aac ggt gcc atg aaa gta ctg 3634
Asp Asn Asn Ser Gln Tyr Phe Tyr Asn Gly Ala Met Lys Val Leu
1150                1155                1160

aaa gag ctg atg aaa aac agc cag aac aaa att att gac ctg tct 3679
Lys Glu Leu Met Lys Asn Ser Gln Asn Lys Ile Ile Asp Leu Ser
1165                1170                1175

ccg gaa ggc gaa aac gcg gtt tac gtt ccg ggt tgg aac tac ggt 3724
Pro Glu Gly Glu Asn Ala Val Tyr Val Pro Gly Trp Asn Tyr Gly
1180                1185                1190

act gcc ggt caa cgc att cag tct ttc ctg act att aac aag gac 3769
Thr Ala Gly Gln Arg Ile Gln Ser Phe Leu Thr Ile Asn Lys Asp
1195                1200                1205

ccg gcc ggt ggt aat aaa att aag gct gta ggt tcc aag ccg gcg 3814
Pro Ala Gly Gly Asn Lys Ile Lys Ala Val Gly Ser Lys Pro Ala
1210                1215                1220

tct atc ttt aaa ggt ttc ctg gca ccg aat gac ggc atg gca gag 3859
Ser Ile Phe Lys Gly Phe Leu Ala Pro Asn Asp Gly Met Ala Glu
1225                1230                1235

cag gca att acc aag ctg aaa ctg gaa ggc ttc gat acc cag aaa 3904
Gln Ala Ile Thr Lys Leu Lys Leu Glu Gly Phe Asp Thr Gln Lys
1240                1245                1250

att ttc gta acc ggt cag gat tat aac gac aaa gcg aaa act ttt 3949
Ile Phe Val Thr Gly Gln Asp Tyr Asn Asp Lys Ala Lys Thr Phe
1255                1260                1265

att aaa gac ggc gac cag aac atg acc att tat aaa ccg gac aaa 3994
Ile Lys Asp Gly Asp Gln Asn Met Thr Ile Tyr Lys Pro Asp Lys
1270                1275                1280

gtt ctg ggt aaa gtg gca gtg gaa gtt ctg cgt gtt ctg att gcg 4039
Val Leu Gly Lys Val Ala Val Glu Val Leu Arg Val Leu Ile Ala
1285                1290                1295

aaa aaa aac aaa gcc agc cga agc gaa gtt gaa aac gag ctg aaa 4084
Lys Lys Asn Lys Ala Ser Arg Ser Glu Val Glu Asn Glu Leu Lys
1300                1305                1310

gcg aaa ctg ccg aac atc tcc ttc aag tat gat aac cag acc tac 4129
Ala Lys Leu Pro Asn Ile Ser Phe Lys Tyr Asp Asn Gln Thr Tyr
1315                1320                1325

aag gtg caa ggc aag aac att aat act atc ctg gtt tct ccg gtg 4174
Lys Val Gln Gly Lys Asn Ile Asn Thr Ile Leu Val Ser Pro Val
1330                1335                1340

att gta acc aaa gcg aat gtg gac aac cct ggg gcg gcg aaa ccg 4219
Ile Val Thr Lys Ala Asn Val Asp Asn Pro Gly Ala Ala Lys Pro
1345                1350                1355

gag gca gcg aaa ccg gtt gcg gct aaa ccg gag acc acc aaa ccg 4264
Glu Ala Ala Lys Pro Val Ala Ala Lys Pro Glu Thr Thr Lys Pro
1360                1365                1370

gtg gct gct aaa ccg gaa gcg gcc aaa ccg gaa gct gcg aaa ccg 4309
Val Ala Ala Lys Pro Glu Ala Ala Lys Pro Glu Ala Ala Lys Pro
1375                1380                1385

gtg gcg gcg aag ccg gag gct gcc aaa ccg gta gct gct aag ccg 4354
Val Ala Ala Lys Pro Glu Ala Ala Lys Pro Val Ala Ala Lys Pro
1390                1395                1400

gaa gcg gct aag ccg gtg gca gca aaa ccg gag gcg gca aaa ccg 4399
Glu Ala Ala Lys Pro Val Ala Ala Lys Pro Glu Ala Ala Lys Pro
1405                1410                1415

gtg gcc gcc aag ccg gaa gct gcc aag ccg gtt gca acc aac act 4444
Val Ala Ala Lys Pro Glu Ala Ala Lys Pro Val Ala Thr Asn Thr
1420                1425                1430

ggt ttt tct ctg acc aac aaa ccg aaa gaa gac tac ttt ccg atg 4489
Gly Phe Ser Leu Thr Asn Lys Pro Lys Glu Asp Tyr Phe Pro Met
1435                1440                1445

acc gaa ggc tcc agc aac cag ggt aaa aag tcc gaa ggt act ccg 4534
Thr Glu Gly Ser Ser Asn Gln Gly Lys Lys Ser Glu Gly Thr Pro
1450                1455                1460

aac cag ggt aag aaa gcg gag ggt gca ccg aac cag ggc aaa aaa 4579
Asn Gln Gly Lys Lys Ala Glu Gly Ala Pro Asn Gln Gly Lys Lys
1465                1470                1475

gcg gaa ggc acc ccg aac caa ggt aaa aaa gca gag ggt gcg ccg 4624
Ala Glu Gly Thr Pro Asn Gln Gly Lys Lys Ala Glu Gly Ala Pro
1480                1485                1490

agc cag cag agc ccg acc acc gaa taa gaattcgctt aagtcgaaca 4671
Ser Gln Gln Ser Pro Thr Thr Glu
1495                1500

gaaagtaatc gtattgtaca cggccgcata atcgaaatta atacgactca ctatagggag 4731

accacaacgg tttcccatct tagtatatta gttaagtata agaaggagat atacc atg 4789

Met

ggt tct gat aat gaa atc gtg gcg ggt att atg cgc gac cat att 4834
Gly Ser Asp Asn Glu Ile Val Ala Gly Ile Met Arg Asp His Ile
    1505                1510                1515

att aac ctg ctt aag gaa ggt aaa cgt att gac gac cgc ggt ttc 4879
Ile Asn Leu Leu Lys Glu Gly Lys Arg Ile Asp Asp Arg Gly Phe
    1520                1525                1530

gaa gat tac cgc ccg att gaa att gag gta ggt gtt att gaa aaa 4924
Glu Asp Tyr Arg Pro Ile Glu Ile Glu Val Gly Val Ile Glu Lys
    1535                1540                1545

gcg gag ggt agc gcg ctg gtg aaa ctg ggt agc act cag gtt ctg 4969
Ala Glu Gly Ser Ala Leu Val Lys Leu Gly Ser Thr Gln Val Leu
    1550                1555                1560

gtg ggt att aaa acc tct ctg ggc gag ccg ttt ccg gac acc ccg 5014
Val Gly Ile Lys Thr Ser Leu Gly Glu Pro Phe Pro Asp Thr Pro
    1565                1570                1575

aac atg ggc gtg atg acc acc aat gtt gaa ctt gtt ccg ctg gcg 5059
Asn Met Gly Val Met Thr Thr Asn Val Glu Leu Val Pro Leu Ala
    1580                1585                1590

tct ccg act ttc gaa ccg ggc ccg ccg gac gag cgt gct atc gaa 5104
Ser Pro Thr Phe Glu Pro Gly Pro Pro Asp Glu Arg Ala Ile Glu
    1595                1600                1605

ctg gca cgt gtt att gac cgt ggt att cgt gag tcc aaa gca ctg 5149
Leu Ala Arg Val Ile Asp Arg Gly Ile Arg Glu Ser Lys Ala Leu
    1610                1615                1620

aat ctg gaa aaa atg gtt atc gtg ccg ggc aaa att gtt cgc gta 5194
Asn Leu Glu Lys Met Val Ile Val Pro Gly Lys Ile Val Arg Val
    1625                1630                1635

gtt ttt atc gac gtt cac gtt ctt gac cac gac ggt aac ctg atg 5239
Val Phe Ile Asp Val His Val Leu Asp His Asp Gly Asn Leu Met
    1640                1645                1650

gac gcc atc ggt att gca gct atc gcc gca ctg ctg aac gca cgt 5284
Asp Ala Ile Gly Ile Ala Ala Ile Ala Ala Leu Leu Asn Ala Arg
    1655                1660                1665

gtt ccg aaa gtt cgc tac aac gaa gaa acg ggt gaa gtt gaa acc 5329
Val Pro Lys Val Arg Tyr Asn Glu Glu Thr Gly Glu Val Glu Thr
    1670                1675                1680

ctg gat gaa act gaa ccg ctg ccg gtt gaa aag att ccg gtt ccg 5374
Leu Asp Glu Thr Glu Pro Leu Pro Val Glu Lys Ile Pro Val Pro
    1685                1690                1695

gtt act ttc gct aaa att ggt aat atc ctg gta gtt gac ccg agc 5419
Val Thr Phe Ala Lys Ile Gly Asn Ile Leu Val Val Asp Pro Ser
    1700                1705                1710

ctg gat gaa gag ctc gtg atg gac ggc aaa att acc att acc act 5464
Leu Asp Glu Glu Leu Val Met Asp Gly Lys Ile Thr Ile Thr Thr
    1715                1720                1725

gac gaa acc ggc cat att tct gcc gtt cag aaa tct gaa ggt ggc 5509
Asp Glu Thr Gly His Ile Ser Ala Val Gln Lys Ser Glu Gly Gly
    1730                1735                1740

gct ttc aaa ctg gaa gaa gta atg tac gcg gtg gaa act gca ttc 5554
Ala Phe Lys Leu Glu Glu Val Met Tyr Ala Val Glu Thr Ala Phe
    1745                1750                1755

aag aag gcg gaa gaa atc cgt aaa ctg att ctg gaa gct gtt gag 5599
Lys Lys Ala Glu Glu Ile Arg Lys Leu Ile Leu Glu Ala Val Glu
    1760                1765                1770

aag gcc aaa cag ctc gag ggc cag aag aag gcg gta cgt aag gaa 5644
Lys Ala Lys Gln Leu Glu Gly Gln Lys Lys Ala Val Arg Lys Glu
    1775                1780                1785

gag gaa ctg gaa acc tac aac ccg aaa gac gag ttt aat atc ctg 5689
Glu Glu Leu Glu Thr Tyr Asn Pro Lys Asp Glu Phe Asn Ile Leu
    1790                1795                1800

aac ccg ggt gaa att tct gac gtg ctg ctg ctg gac gta acc ccg 5734
Asn Pro Gly Glu Ile Ser Asp Val Leu Leu Leu Asp Val Thr Pro
    1805                1810                1815

ctg acc ctg ggc att gag act ctg ggt ggc att gct acc ccg ctg 5779
Leu Thr Leu Gly Ile Glu Thr Leu Gly Gly Ile Ala Thr Pro Leu
    1820                1825                1830

att ccg cga aac acc acc att cca gtg acc aaa agc caa att ttc 5824
Ile Pro Arg Asn Thr Thr Ile Pro Val Thr Lys Ser Gln Ile Phe
    1835                1840                1845

tcc acc gct gaa gat aac cag acc gaa gtt acc atc tcc gtt gtg 5869
Ser Thr Ala Glu Asp Asn Gln Thr Glu Val Thr Ile Ser Val Val
    1850                1855                1860

caa ggt gaa cga cag ctg gca gcc gac aac aag atg ctg ggt cgc 5914
Gln Gly Glu Arg Gln Leu Ala Ala Asp Asn Lys Met Leu Gly Arg
    1865                1870                1875

ttt aac ctg agc ggc atc gaa gcg gct ccg cgt ggt ctg cca caa 5959
Phe Asn Leu Ser Gly Ile Glu Ala Ala Pro Arg Gly Leu Pro Gln
    1880                1885                1890

att gaa gtg tcc ttc agc atc gat gta aac ggc atc acc act gtt 6004
Ile Glu Val Ser Phe Ser Ile Asp Val Asn Gly Ile Thr Thr Val
    1895                1900                1905

tcc gct aaa gac aaa aaa acc ggt aaa gaa cag acc att acc atc 6049
Ser Ala Lys Asp Lys Lys Thr Gly Lys Glu Gln Thr Ile Thr Ile
    1910                1915                1920

aag aat acc tcc acc ctg tct gaa gaa gaa att aac aaa atg att 6094
Lys Asn Thr Ser Thr Leu Ser Glu Glu Glu Ile Asn Lys Met Ile
    1925                1930                1935

cag gaa gct gag gaa aac cgt gaa gcc gat gca ctg aaa aaa gac 6139
Gln Glu Ala Glu Glu Asn Arg Glu Ala Asp Ala Leu Lys Lys Asp
    1940                1945                1950

aaa att gaa acc acc gta cgt gcg gaa ggc ctg att aac cag ctg 6184
Lys Ile Glu Thr Thr Val Arg Ala Glu Gly Leu Ile Asn Gln Leu
    1955                1960                1965

gaa aaa tct att act gac cag ggt gag aaa att gac ccg aaa cag 6229
Glu Lys Ser Ile Thr Asp Gln Gly Glu Lys Ile Asp Pro Lys Gln
    1970                1975                1980

aaa gaa ctg ctg gaa aag cag att cag gaa ctg aaa gac ctg ctg 6274
Lys Glu Leu Leu Glu Lys Gln Ile Gln Glu Leu Lys Asp Leu Leu
    1985                1990                1995

aaa gag gat aaa acc gac gaa ctg aaa ctg aaa ctg gac cag att 6319
Lys Glu Asp Lys Thr Asp Glu Leu Lys Leu Lys Leu Asp Gln Ile
    2000                2005                2010

gaa gct gcg gcc caa tcc ttc gcg cag gca acc gcg cag caa gcg 6364
Glu Ala Ala Ala Gln Ser Phe Ala Gln Ala Thr Ala Gln Gln Ala
    2015                2020                2025

aac acc agc gaa tct gac ccg aaa gcg gac gat agc aac acc atc 6409
Asn Thr Ser Glu Ser Asp Pro Lys Ala Asp Asp Ser Asn Thr Ile
    2030                2035                2040

gac gcg gag att aaa caa gat ggt tcc ggg aaa cag gat gaa atc 6454
Asp Ala Glu Ile Lys Gln Asp Gly Ser Gly Lys Gln Asp Glu Ile
    2045                2050                2055

aaa aaa ttc acc ttc gac ctg ctg tac aaa ctg att gaa ctg gaa 6499
Lys Lys Phe Thr Phe Asp Leu Leu Tyr Lys Leu Ile Glu Leu Glu
    2060                2065                2070

aaa gat tac ctg tac gac ctg tac tcc gaa gtg ggc ctg gcg gaa 6544
Lys Asp Tyr Leu Tyr Asp Leu Tyr Ser Glu Val Gly Leu Ala Glu
    2075                2080                2085

agc gcc atc aag ttc agc atc tac aac gct ggc aaa ttc ctg caa 6589
Ser Ala Ile Lys Phe Ser Ile Tyr Asn Ala Gly Lys Phe Leu Gln
    2090                2095                2100

aac ctg ggc tac gat tct ccg ttt tct aaa gaa gag acc gaa atc 6634
Asn Leu Gly Tyr Asp Ser Pro Phe Ser Lys Glu Glu Thr Glu Ile
    2105                2110                2115

gag ccg gag att ttc tcc cag ctg agc gct cgc gca gat gag aac 6679
Glu Pro Glu Ile Phe Ser Gln Leu Ser Ala Arg Ala Asp Glu Asn
    2120                2125                2130

cat gac ttc ttc tct ggt aac ggc agc agc tac gtg atg gcg ctg 6724
His Asp Phe Phe Ser Gly Asn Gly Ser Ser Tyr Val Met Ala Leu
    2135                2140                2145

gca gag gaa acc gaa gac gaa gat tgg gag ttc taa gctt 6764
Ala Glu Glu Thr Glu Asp Glu Asp Trp Glu Phe
    2150                2155

<210> 26
<211> 737
<212> PRT
<213> Artificial Sequence

<220>
<223> Synthetic Construct

<400> 26

Met Ala Lys Arg Ile Phe Val Gln Asn Arg Glu Leu Val Val Pro Gly
1               5                   10                  15

Thr Leu Leu Ala Gln Gly Pro Tyr Lys Asn Gly Arg Gly Thr Phe Arg
            20                  25                  30

Glu Gly Ser Arg Ile Tyr Ser Thr Val Ile Gly Leu Val Asp Ile Lys
        35                  40                  45

Gly Asn Thr Ile Arg Val Ile Pro Leu Glu Gly Pro Tyr Ile Pro Glu
    50                  55                  60

Val Gly Asp Asn Val Ile Gly Lys Ile Val Asp Val Lys Phe Ser Ser
65                  70                  75                  80

Trp Val Val Asp Ile Gly Ala Pro Tyr Pro Ala Asn Leu Lys Ile Gln
                85                  90                  95

Asp Phe Thr Asp Glu Lys Ile Asp Leu Leu Arg Thr Asp Leu Arg Lys
            100                 105                 110

Phe Phe Asp Ile Gly Asp Ile Ile Tyr Ala Lys Val Lys Ala Ile Thr
        115                 120                 125

Glu Val Asn Asn Ile Asp Leu Thr Thr Lys Gly Met Pro Phe Asn Gly
    130                 135                 140

Gly Pro Leu Lys Gly Gly Gln Ile Val Lys Ile Thr Pro Ser Arg Val
145                 150                 155                 160

Pro Arg Val Ile Gly Arg Gly Gly Ser Met Ile Asn Met Ile Lys Lys
                165                 170                 175

Leu Thr Met Thr Arg Ile Ile Val Gly Gln Asn Gly Trp Ile Trp Val
            180                 185                 190

Ser Gly Lys Asn Asp Ala Leu Glu Lys Leu Ala Ile Glu Ala Ile Leu
        195                 200                 205

Lys Ile Asp Lys Glu Ser His Thr Arg Gly Leu Thr Asp Arg Ile Lys
    210                 215                 220

Ala Leu Leu Leu Ser Arg Leu Gln Glu Leu Lys Glu Lys Gly Val Ile
225                 230                 235                 240

Glu Glu Ile Pro Lys Leu Glu Glu Glu Pro Gln Gly Glu Asp Glu Val
                245                 250                 255

Asn Gly Asn Asp Gly Glu Ala Arg Ser Gly Val Asn Asn Asp Pro Asn
            260                 265                 270

Tyr Lys Ile Glu Asp Leu Lys Val Ile Lys Asn Glu Ala Gly Asp His
        275                 280                 285

Gln Leu Gly Ser Lys Pro Ile Lys Ile Ala Leu Ile Gly Ala Gly Asn
    290                 295                 300

Val Gly Asn Ser Phe Leu Tyr Ala Ala Met Asn Gln Gly Leu Ala Ser
305                 310                 315                 320

Glu Tyr Gly Ile Ile Asp Ile Asn Pro Asp Phe Ala Asp Gly Asn Ala
                325                 330                 335

Phe Asp Phe Glu Asp Ala Ser Ala Ser Leu Pro Phe Pro Ile Ser Val
            340                 345                 350

Ser Arg Tyr Glu Tyr Lys Asp Leu Lys Asp Ala Asp Phe Ile Val Ile
        355                 360                 365

Thr Ala Gly Arg Pro Gln Lys Pro Gly Glu Thr Arg Leu Glu Leu Val
    370                 375                 380

Ala Asp Asn Ile Arg Ile Ile Arg Glu Ile Ala Leu Lys Val Lys Glu
385                 390                 395                 400

Ser Gly Phe Ser Gly Ile Ser Ile Ile Val Ala Asn Pro Val Asp Ile
                405                 410                 415

Ile Thr Arg Ala Tyr Arg Asp Ala Ser Gly Phe Ser Asp Gln Lys Val
            420                 425                 430

Ile Gly Ser Gly Thr Val Leu Asp Thr Ala Arg Leu Gln Phe Ala Ile
        435                 440                 445

Ala Lys Arg Ala Lys Val Ser Pro Asn Ser Val Gln Ala Tyr Val Met
    450                 455                 460

Gly Glu His Gly Asp Ser Ser Phe Val Ala Tyr Ser Asn Ile Lys Ile
465                 470                 475                 480

Ala Gly Glu Tyr Phe Ser Ala Tyr Ser Lys Leu Thr Gly Ile Asp Ser
                485                 490                 495

Ser Asn Tyr Glu Lys Glu Leu Glu Tyr Pro Val Ser Arg Arg Ala Tyr

            500                 505                 510

Glu Ile Ile Asn Arg Lys Arg Ala Thr Phe Tyr Gly Ile Gly Ala Ala
        515                 520                 525

Ile Ala Lys Ile Val Ser Asn Ile Ile Lys Asp Thr Lys Asn Ile Met
    530                 535                 540

Ile Ala Gly Ala Asn Leu Arg Gly Glu Tyr Gly Phe His Gly Val Asn
545                 550                 555                 560

Ile Gly Val Pro Val Val Leu Gly Ala Asn Gly Ile Glu Lys Ile Ile
                565                 570                 575

Glu Ile Ser Leu Asn Asp Lys Glu Lys Glu Lys Phe Ala Lys Ser Val
            580                 585                 590

Ala Ile Ile Asp Lys Ile Tyr Gln Asp Ala Ile Lys Asn Ile Pro Lys
        595                 600                 605

Ser Gln Lys Ser Glu Gly Thr Gln Asn Gln Gly Lys Lys Ala Glu Gly
    610                 615                 620

Ala Pro Asn Gln Ser Lys Lys Ser Glu Glu Asn Ser Asn Lys Leu Thr
625                 630                 635                 640

Glu Tyr Ile Gln Glu Leu Gly Thr Lys Val Glu Lys Ser Leu Lys Ser
                645                 650                 655

Lys Gly Lys Asn Tyr Ser Ala Glu Val Glu Lys Ile Ile Glu Ala Phe
            660                 665                 670

Ser Gly Gly Tyr Lys Phe Leu Asp Phe Ala Leu Val Glu Gln Thr Pro
        675                 680                 685

Lys Pro Glu Thr Pro Lys Thr Glu Ala Ala Lys Pro Glu Thr Thr Lys
    690                 695                 700

Pro Val Ala Ala Arg Pro Glu Ala Ala Lys Val Ala Ala Lys Pro Ser
705                 710                 715                 720

Ala Ala Lys Pro Val Ser Ser Pro Ala Pro Lys Lys Ser Thr Leu Tyr
                725                 730                 735

Val

<210> 27
<211> 765
<212> PRT
<213> Artificial Sequence

<220>
<223> Synthetic Construct

<400> 27

Met Ala Glu Lys Pro Glu Gly Leu Lys Leu Ile Asp Glu Asn Gly Arg
1               5                   10                  15

Arg Ile Asp Gly Arg Lys Lys Tyr Glu Leu Arg Pro Ile Lys Met Glu
            20                  25                  30

Val Gly Val Leu Lys Asn Ala Asn Gly Ser Ala Tyr Ile Glu Trp Gly
        35                  40                  45

Lys Asn Lys Ile Ile Ala Ala Val Tyr Gly Pro Arg Glu Leu His Pro
    50                  55                  60

Lys His Leu Gln Arg Pro Asp Arg Ala Ile Leu Arg Val Arg Tyr Asn
65                  70                  75                  80

Met Ala Pro Phe Ser Val Glu Glu Thr Lys Ser Pro Gly Pro Asp Arg
                85                  90                  95

Arg Ser Ile Glu Ile Ser Lys Val Ile Lys Gly Ala Leu Glu Pro Ala
            100                 105                 110

Leu Ile Leu Glu Met Phe Pro Arg Thr Ala Ile Asp Val Phe Ile Glu
        115                 120                 125

Val Leu Gln Ala Asp Ala Gly Thr Arg Val Ala Gly Ile Thr Ala Ala
    130                 135                 140

Ser Leu Ala Leu Ala Asp Ala Gly Ile Pro Met Arg Asp Leu Val Ala
145                 150                 155                 160

Ala Cys Ala Ala Gly Lys Ile Glu Gly Glu Ile Val Leu Asp Leu Asn
                165                 170                 175

Lys Glu Glu Asp Asn Tyr Gly Glu Ala Asp Val Pro Val Ala Ile Met
            180                 185                 190

Pro Leu Lys Asn Asp Ile Thr Leu Leu Gln Met Asp Gly Tyr Leu Thr
        195                 200                 205

Lys Asp Glu Phe Ile Glu Ala Val Lys Leu Ala Ile Lys Gly Ala Lys

210                 215                 220

Ala Val Tyr Gln Lys Gln Arg Glu Ala Leu Lys Glu Lys Tyr Leu Lys
225                 230                 235                 240

Ile Ala Gln Glu Val Glu Gly Ser Pro Lys His Lys Val Ser Asn Asp
                245                 250                 255

Ser Ile Arg Ile Ala Leu Thr Asp Pro Asp Asn Pro Arg Trp Ile Ser
            260                 265                 270

Ala Gln Lys Asp Ile Ile Ser Tyr Val Asp Glu Thr Glu Ala Ala Thr
        275                 280                 285

Ser Thr Ile Thr Lys Asn Gln Asp Ala Gln Asn Asn Trp Leu Thr Gln
    290                 295                 300

Gln Ala Asn Leu Ser Pro Ala Pro Lys Gly Phe Ile Ile Ala Pro Glu
305                 310                 315                 320

Asn Gly Ser Gly Val Gly Thr Ala Val Asn Thr Ile Ala Asp Lys Gly
                325                 330                 335

Ile Pro Ile Val Ala Tyr Asp Arg Leu Ile Thr Gly Ser Asp Lys Tyr
            340                 345                 350

Asp Trp Tyr Val Ser Phe Asp Asn Glu Lys Val Gly Glu Leu Gln Gly
        355                 360                 365

Leu Ser Leu Ala Ala Gly Leu Leu Gly Lys Glu Asp Gly Ala Phe Asp
    370                 375                 380

Ser Ile Asp Gln Met Asn Glu Tyr Leu Lys Ser His Met Pro Gln Glu
385                 390                 395                 400

Thr Ile Ser Phe Tyr Thr Ile Ala Gly Ser Gln Asp Asp Asn Asn Ser
                405                 410                 415

Gln Tyr Phe Tyr Asn Gly Ala Met Lys Val Leu Lys Glu Leu Met Lys
            420                 425                 430

Asn Ser Gln Asn Lys Ile Ile Asp Leu Ser Pro Glu Gly Glu Asn Ala
        435                 440                 445

Val Tyr Val Pro Gly Trp Asn Tyr Gly Thr Ala Gly Gln Arg Ile Gln
    450                 455                 460

Ser Phe Leu Thr Ile Asn Lys Asp Pro Ala Gly Gly Asn Lys Ile Lys
465                 470                 475                 480

Ala Val Gly Ser Lys Pro Ala Ser Ile Phe Lys Gly Phe Leu Ala Pro
                485                 490                 495

Asn Asp Gly Met Ala Glu Gln Ala Ile Thr Lys Leu Lys Leu Glu Gly
            500                 505                 510

Phe Asp Thr Gln Lys Ile Phe Val Thr Gly Gln Asp Tyr Asn Asp Lys
        515                 520                 525

Ala Lys Thr Phe Ile Lys Asp Gly Asp Gln Asn Met Thr Ile Tyr Lys
    530                 535                 540

Pro Asp Lys Val Leu Gly Lys Val Ala Val Glu Val Leu Arg Val Leu
545                 550                 555                 560

Ile Ala Lys Lys Asn Lys Ala Ser Arg Ser Glu Val Glu Asn Glu Leu
                565                 570                 575

Lys Ala Lys Leu Pro Asn Ile Ser Phe Lys Tyr Asp Asn Gln Thr Tyr
            580                 585                 590

Lys Val Gln Gly Lys Asn Ile Asn Thr Ile Leu Val Ser Pro Val Ile
        595                 600                 605

Val Thr Lys Ala Asn Val Asp Asn Pro Gly Ala Ala Lys Pro Glu Ala
    610                 615                 620

Ala Lys Pro Val Ala Ala Lys Pro Glu Thr Thr Lys Pro Val Ala Ala
625                 630                 635                 640

Lys Pro Glu Ala Ala Lys Pro Glu Ala Ala Lys Pro Val Ala Ala Lys
                645                 650                 655

Pro Glu Ala Ala Lys Pro Val Ala Ala Lys Pro Glu Ala Ala Lys Pro
            660                 665                 670

Val Ala Ala Lys Pro Glu Ala Ala Lys Pro Val Ala Ala Lys Pro Glu
        675                 680                 685

Ala Ala Lys Pro Val Ala Thr Asn Thr Gly Phe Ser Leu Thr Asn Lys
    690                 695                 700

Pro Lys Glu Asp Tyr Phe Pro Met Thr Glu Gly Ser Ser Asn Gln Gly
705                 710                 715                 720

Lys Lys Ser Glu Gly Thr Pro Asn Gln Gly Lys Lys Ala Glu Gly Ala
                725                 730                 735

Pro Asn Gln Gly Lys Lys Ala Glu Gly Thr Pro Asn Gln Gly Lys Lys
            740                 745                 750

Ala Glu Gly Ala Pro Ser Gln Gln Ser Pro Thr Thr Glu
        755                 760                 765

<210>  28
<211>  657
<212>  PRT
<213>  Artificial Sequence

<220>
<223>  Synthetic Construct

<400>  28
   Met Gly Ser Asp Asn Glu Ile Val Ala Gly Ile Met Arg Asp His Ile
   1               5                   10                  15
   Ile Asn Leu Leu Lys Glu Gly Lys Arg Ile Asp Asp Arg Gly Phe Glu
               20                  25                  30
   Asp Tyr Arg Pro Ile Glu Ile Glu Val Gly Val Ile Glu Lys Ala Glu
           35                  40                  45
   Gly Ser Ala Leu Val Lys Leu Gly Ser Thr Gln Val Leu Val Gly Ile
       50                  55                  60
   Lys Thr Ser Leu Gly Glu Pro Phe Pro Asp Thr Pro Asn Met Gly Val
   65                  70                  75                  80
   Met Thr Thr Asn Val Glu Leu Val Pro Leu Ala Ser Pro Thr Phe Glu
                   85                  90                  95
   Pro Gly Pro Pro Asp Glu Arg Ala Ile Glu Leu Ala Arg Val Ile Asp
               100                 105                 110
   Arg Gly Ile Arg Glu Ser Lys Ala Leu Asn Leu Glu Lys Met Val Ile
           115                 120                 125
   Val Pro Gly Lys Ile Val Arg Val Val Phe Ile Asp Val His Val Leu
       130                 135                 140
   Asp His Asp Gly Asn Leu Met Asp Ala Ile Gly Ile Ala Ala Ile Ala
   145                 150                 155                 160
   Ala Leu Leu Asn Ala Arg Val Pro Lys Val Arg Tyr Asn Glu Glu Thr
                   165                 170                 175
   Gly Glu Val Glu Thr Leu Asp Glu Thr Glu Pro Leu Pro Val Glu Lys
               180                 185                 190
   Ile Pro Val Pro Val Thr Phe Ala Lys Ile Gly Asn Ile Leu Val Val
           195                 200                 205
   Asp Pro Ser Leu Asp Glu Glu Leu Val Met Asp Gly Lys Ile Thr Ile
       210                 215                 220
   Thr Thr Asp Glu Thr Gly His Ile Ser Ala Val Gln Lys Ser Glu Gly
   225                 230                 235                 240
   Gly Ala Phe Lys Leu Glu Glu Val Met Tyr Ala Val Glu Thr Ala Phe
                   245                 250                 255
   Lys Lys Ala Glu Glu Ile Arg Lys Leu Ile Leu Glu Ala Val Glu Lys
               260                 265                 270
   Ala Lys Gln Leu Glu Gly Gln Lys Lys Ala Val Arg Lys Glu Glu Glu
           275                 280                 285
   Leu Glu Thr Tyr Asn Pro Lys Asp Glu Phe Asn Ile Leu Asn Pro Gly
       290                 295                 300
   Glu Ile Ser Asp Val Leu Leu Leu Asp Val Thr Pro Leu Thr Leu Gly
   305                 310                 315                 320
   Ile Glu Thr Leu Gly Gly Ile Ala Thr Pro Leu Ile Pro Arg Asn Thr
                   325                 330                 335
   Thr Ile Pro Val Thr Lys Ser Gln Ile Phe Ser Thr Ala Glu Asp Asn
               340                 345                 350
   Gln Thr Glu Val Thr Ile Ser Val Val Gln Gly Glu Arg Gln Leu Ala
           355                 360                 365
   Ala Asp Asn Lys Met Leu Gly Arg Phe Asn Leu Ser Gly Ile Glu Ala
       370                 375                 380
   Ala Pro Arg Gly Leu Pro Gln Ile Glu Val Ser Phe Ser Ile Asp Val
   385                 390                 395                 400
   Asn Gly Ile Thr Thr Val Ser Ala Lys Asp Lys Lys Thr Gly Lys Glu
                   405                 410                 415
   Gln Thr Ile Thr Ile Lys Asn Thr Ser Thr Leu Ser Glu Glu Glu Ile
               420                 425                 430
   Asn Lys Met Ile Gln Glu Ala Glu Glu Asn Arg Glu Ala Asp Ala Leu
           435                 440                 445
   Lys Lys Asp Lys Ile Glu Thr Thr Val Arg Ala Glu Gly Leu Ile Asn
       450                 455                 460
   Gln Leu Glu Lys Ser Ile Thr Asp Gln Gly Glu Lys Ile Asp Pro Lys
   465                 470                 475                 480
   Gln Lys Glu Leu Leu Glu Lys Gln Ile Gln Glu Leu Lys Asp Leu Leu
                   485                 490                 495
   Lys Glu Asp Lys Thr Asp Glu Leu Lys Leu Lys Leu Asp Gln Ile Glu
               500                 505                 510
   Ala Ala Ala Gln Ser Phe Ala Gln Ala Thr Ala Gln Gln Ala Asn Thr
           515                 520                 525
   Ser Glu Ser Asp Pro Lys Ala Asp Asp Ser Asn Thr Ile Asp Ala Glu
       530                 535                 540
   Ile Lys Gln Asp Gly Ser Gly Lys Gln Asp Glu Ile Lys Lys Phe Thr
   545                 550                 555                 560
   Phe Asp Leu Leu Tyr Lys Leu Ile Glu Leu Glu Lys Asp Tyr Leu Tyr
                   565                 570                 575
   Asp Leu Tyr Ser Glu Val Gly Leu Ala Glu Ser Ala Ile Lys Phe Ser
               580                 585                 590
   Ile Tyr Asn Ala Gly Lys Phe Leu Gln Asn Leu Gly Tyr Asp Ser Pro
           595                 600                 605
   Phe Ser Lys Glu Glu Thr Glu Ile Glu Pro Glu Ile Phe Ser Gln Leu
       610                 615                 620
   Ser Ala Arg Ala Asp Glu Asn His Asp Phe Phe Ser Gly Asn Gly Ser
   625                 630                 635                 640
   Ser Tyr Val Met Ala Leu Ala Glu Glu Thr Glu Asp Glu Asp Trp Glu
                   645                 650                 655
   Phe

<210>  29
<211>  23
<212>  DNA
<213>  Artificial Sequence

<220>
<223>  Reverse primer for sequencing of inserts in T7 vector

<400>  29
tgtggtctcc ctatagtgag tcg                                               23


<210>  30
<211>  32
<212>  DNA
<213>  Artificial Sequence

<220>
<223>  Reverse primer for the amplification of the P36 antigen

<400>  30
ggctgcagtt aaatgttttt aatcgcatcc tg                                     32


<210>  31
<211>  27
<212>  DNA
<213>  Artificial Sequence

<220>
<223>  Foward primer for the amplification of the MHP271 antigen

<400>  31
ccagatctcc gaaatcccaa aaatctg                                           27


<210>  32
<211>  25
<212>  DNA
<213>  Artificial Sequence

<220>
<223>  Foward primer for the amplification of the Rrp41 protein

<400>  32
agcatatggc agagaaaccg gaagg                                             25


<210>  33
<211>  34
<212>  DNA
<213>  Artificial Sequence

<220>
<223>  Reverse primer for the amplification of P46 antigen

<400>  33
ccgaattctt aagggttgtc cacattcgct ttgg                                   34


<210>  34

<211>  31
<212>  DNA
<213>  Artificial Sequence

<220>
<223>  Foward primer for the amplification of P97R1R2 antigen

<400>  34
aaggatccgc gaaaccggag gcagcgaaac c                                    31


<210>  35
<211>  31
<212>  DNA
<213>  Artificial Sequence

<220>
<223>  Reverse primer for the amplification of HSP antigen

<400>  35
ccaagcttaa tcttgtttaa tctccgcgtc g                                    31


<210>  36
<211>  37
<212>  DNA
<213>  Artificial Sequence

<220>
<223>  Foward primer for the amplification of NrdF antigen

<400>  36
ggctcgaggg gaaacaggat gaaatcaaaa aattcac                              37

## Description

### Technical Field

This invention refers to a complex of polyproteins with immunogenic activity based on specific polypeptides selected from antigenic proteins of *Mycoplasma hyopneumoniae* which is the etiological agent of Enzootic Pneumonia of Swine (EPS). More precisely, the present invention relates to a complex of proteins, herein called Exomyc complex, based on exosome proteins of *Pyrococcus abyssi* carrying vaccine antigens of *M*. *hyopneumoniae.*

Other additional objects of this invention provide: (i) an encoding gene of polyproteins comprising a complex based exosome of *P. abyssi* which carries copies of vaccine antigens of *M*. *hyopneumoniae*; (ii) a composition comprising the complex of polyproteins of the invention, optionally one or more adjuvants and/or additives and a pharmaceutically acceptable vehicle; (iii) a process of obtaining said lipoprotein complex, and (iv) uses of said complex of polyproteins and said composition in the treatment and prevention of infections caused by *M. hyopneumoniae.*

### History of the Invention

*Mycoplasma hyopneumoniae* is the etiological agent of Enzootic Pneumonia of Swine (EPS), a contagious respiratory disease affecting swines worldwide and causing large economic losses.

Commercial vaccines (bacterins - inactivated bacteria) currently used for the control of this disease are not produced in Brazil and have a high cost of production. In addition, immunization with bacterins provides only partial protection and does not prevent the colonization of the epithelium by *M. hyopneumoniae* (Thacker EL, Thacker BJ, Kuhn M, Hawkins PA, Waters WR. (2000) "Evaluation of local and systemic immune responses induced by intramuscular injection of a Mycoplasma hyopneumoniae bacterin to pigs". Am J Vet Res. 61 :1384-1389). The patent document PI0210804, for example, describes a method of treating a disease or disorder caused by *M. hyopneumoniae* with a vaccine comprising cells of this partially or totally inactivated bacterium or, alternatively polypeptides or immunogenic proteins selected from antigens P46, P65, P97, P102, P70, P50 and P44 of *M*. *hyopneumoniae.* In this same line of proposals, the document WO200665841 can be cited.

Vaccine antigens are being tested in different immunization systems using the technology of recombinant DNA. However, such antigens individually provides only partial protection (Simionatto S, Marchioro SB, Maes D, Dellagostin OA. (2013) "Mycoplasma hyopneumoniae: from disease to vaccine development". Vet Microbiol. 165:234-242). An example of a vaccine based on antigens mixtures is disclosed in EP1245677 reporting the mixture of antigens encoded by the mhp3 gene of *M*. *hyopneumoniae* at least one polypeptide selected from P46, P65, P97, P102 of *M*. *hyopneumoniae.*

One of the main vaccine antigens tested so far is a protein called P97R1, containing the binding site to cilia of the main adhesion of *Mycoplasma hyopneumoniae,* the P97 (Wilton JL, Scarman AL, Walker MJ, Djordjevic SP. (1998)" Reiterated repeat region variability in the ciliary adhesin gene of Mycoplasma hyopneumoniae". Microbiology. 144:1931 -1943). Adherence to cilia is an important event for the pathogenicity of *M*. *hyopneumoniae.* Bacterial vaccine does not induce antibodies against P97, which would explain the fact that immunization with bacterins does not prevent the initial colonization of the lung tissue (Chen AY, Fry SR, Daggard GE, Mukkur TK. (2008) "Evaluation of immune response to recombinant potential protective antigens of Mycoplasma hyopneumoniae delivered as cocktail DNA and/or recombinant protein vaccines in mice". Vaccine. 26:4372-4378). An alternative to improve performance and effectiveness of a vaccine based on antigen P97 is described in the patent document PI0603619 which describes a process of vaccine preparation of recombinant subunit (rLTBFM) against PES, comprising the melting of B subunit of E. coli enterotoxin (LTB, adjuvant) with the repeating region R1 of adhesin P97 of *M*. *hyopneumoniae* (R1, immunogen).

The immunization with various antigens prepared separately, either as protein or as DNA vaccine, was able to induce the immune response both humoral and cellular against each of the proteins tested, that is, P42, P97R1, P46 and NrdF (Chen AY, Fry SR, Daggard GE, Mukkur TK. (2008) "Evaluation of immune response to recombinant potential protective antigens of Mycoplasma hyopneumoniae delivered as cocktail DNA and/or recombinant protein vaccines in mice". Vaccine. 26:4372-4378); as weel as P37, P42, P46 and P95 ; (Galli V, Simionatto S, Marchioro SB, Fisch A, Gomes CK, Conceição FR, Dellagostin OA. (2012) "Immunization of mice with Mycoplasma hyopneumoniae antigens P37, P42, P46 and P95 delivered as recombinant subunit or DNA vaccines". Vaccine. 31 :135-140).

However, the production of separate antigens is still an impediment for industrial production of a vaccine containing all these antigens, because it would be necessary to carry out several individual fermentations for each antigen, combining the antigens obtained in a final pool and only then use this pool for immunization. This process is extremely complex and costly, invalidating large-scale production.

Recently, it was proposed the merging of different antigens of *M*. *hyopneumoniae* with protective capacity at adjuvant unit (Marchioro, S. B. (2013) "Evaluation of vaccines against Mycoplasma hyopneumoniae in swines and mice". Thesis (Doctorate. Post Graduation Program in Biotechnology. Federal University of Pelotas). In said work, an encoding gene of a polyprotein was synthesized, which contains the antigens of *M*. *hyopneumoniae* P97R1, P42 and Nrdf attached to the protein rLTB (subunit B of thermolabile enterotoxin of Escherichia coli, used as adjuvant). The immunization with this recombinant protein stimulated the formation of antibodies against each of the three antigens of mycobacteria, showing it is possible to stimulate an immune response against different antigens expressed as a single protein (polyprotein).

Thus, the construction of synthetic genes encoding more than one vaccine antigen of *M*. *hyopneumoniae* represents the possibility of obtaining a recombinant vaccine effective against this bacterium, at low cost and with a simple fermentation process.

### Summary of the Invention

The present invention reveals new polyproteins containing immunogenic regions derived from antigens P36, P46, HSP, NrdF, P97, MHP271 and P216 of *M*. *hyopneumoniae,* such regions that are fused to segments of the carrier proteins Rrp4, Rrp41 and Rrp42 of exosome of *Pyrococcus abyssi.*

According to a first aspect of the present invention, it is provided a complex of three polyproteins characterized by amino acid sequences herein identified as SEQ ID NO:26, SEQ ID NO:27 e SEQ ID NO:28.

According to a second aspect of the present invention, there are provided amino acid sequences having conservative substitutions in the sequences SEQ ID NO:26, SEQ ID NO:27 and SEQ ID NO:28, as well as immunogenic fragments thereof comprising conservative substitutions.

According to a third aspect of the present invention, it is provided a sequence of nucleotides - SEQ ID NO:25 - comprising the encoding synthetic gene to the amino acid sequences of three polyproteins comprehended by Complex of Exomyc Proteins of the present invention, nominally, the sequences SEQ ID NO:26, SEQ ID NO:27 e SEQ ID NO:28, or of a sequence of nucleotides having at least 75% of identity related to SEQ ID NO:25.

In a fourth aspect, the present invention relates to na antigenic composition comprising (i) at least two polypeptides of complex of polyproteins, comprising SEQ ID NO:26, SEQ ID NO:27 and SEQ ID NO:28, their analogs, derivative or immunogenic fragments thereof, including conservative substitutions in SEQ ID NO:26, SEQ ID NO:27 and SEQ ID NO:28, (ii) optionally, additives or potentiating substances of immunogenic activity of polyproteins and (iii) a pharmacologically acceptable vehicle.

In a fifth aspect, the present invention refers to a process comprising the steps of: (i) inserting a fusion gene comprising antigenic and stable segments derived from antigens P36, P46, HSP, NrdF, P97, MHP271 and P216 of *M*. *hyopneumoniae* with carrier proteins Rrp4, Rrp41 and Rrp42 of *P. abyssi* in an expression region of a plasmid, preferably the pMRK between sites Ndel and Hind III; (ii) transforming the host cell, preferably E. coli, with said plasmid transformed by insertion of sequences: SEQ ID NO:1 (derived from P36 antigen), SEQ ID NO:2 (derived from P46 antigen), SEQ ID NO:3 (derived from HSP antigen (P42)), SEQ ID NO:4 (derived from NrdF antigen), SEQ ID NO:5 (derived from P97 antigen), SEQ ID NO:6 (derived from MHP271 antigen), SEQ ID NO:7 and SEQ ID NO:8 (derived from P216 antigen), SEQ ID NO:9 (derived from Rrp4 carrier protein), SEQ ID NO:10 (derived from Rrp41 carrier protein) and SEQ ID NO:1 1 (derived from Rrp42 carrier protein); and (iii) expressing the fused heterologous protein from host cell to obtain polyproteins of sequences SEQ ID NO:26, SEQ ID NO:27 e SEQ ID NO:28, or analogs thereof having conservative substitutions, or antigenic fragments thereof having conservative substitutions.

Also, according to a sixth aspect, the invention refers to the use of (a) complex of polyproteins of SEQ ID NO:26, SEQ ID NO:27 and SEQ ID NO:28, analogs, derivatives or antigenic fragments thereof, including conservative substitutions in SEQ ID NO:26, SEQ ID NO:27 and SEQ ID NO:28, and/or (b) an antigenic composition comprising said polyproteins of SEQ ID NO:26, SEQ ID NO:27 and SEQ ID NO:28, analogs, derivatives or immunogenic fragments thereof, including conservative substitutions in the SEQ ID NO:26, SEQ ID NO:27 and SEQ ID NO:28 in the treatment or prophylaxis of medical and/or veterinary conditions related to infection by *M. hyopneumoniae.*

### Brief Description of Figures

Figure 1 shows the characterization of P36 antigen of *M*. *hyopneumoniae.* Figure 1 A presents the amino acids sequence of this protein; highlighting in gray ( ) the sequence selected to include in the complex of the present invention. Figure 1 B illustrates "ribbons" Diagram of P36 structural model of M. *hyopneumoniae.*
Figure 2 shows the characterization of P46 antigen of *M*. *hyopneumoniae.* Figure 2A presents the amino acids sequence of this protein, showing the peptide segment highlighted in gray ( ) which was selected to include in the complex of the present invention. Figure 2B illustrates the "ribbons" Diagram of P46 structural model of *M*. *hyopneumoniae.*

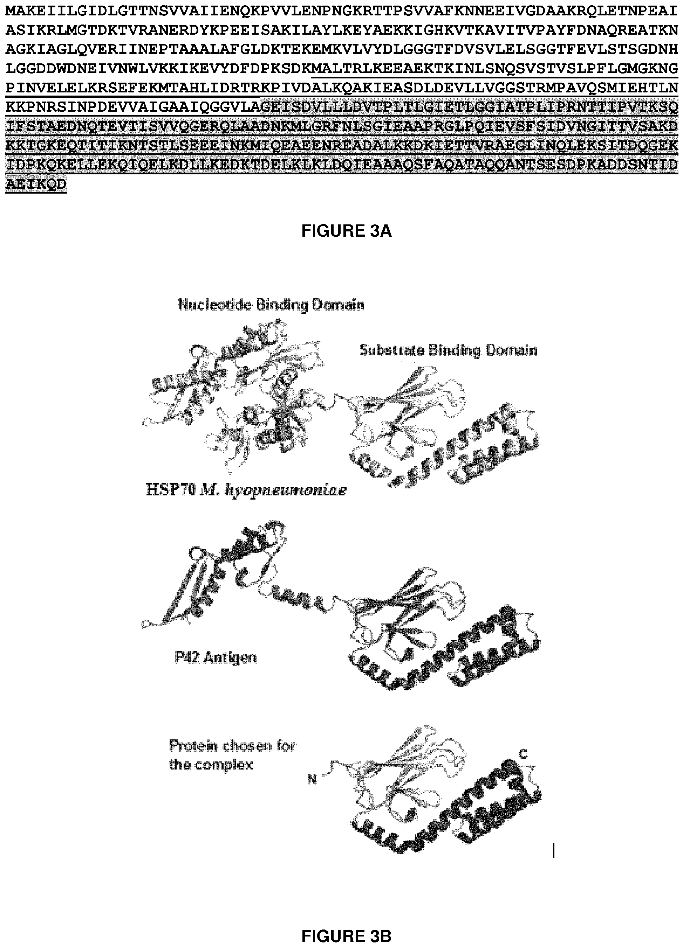

Figure 3 shows the characterization of P42 antigen (HSP) of *M*. *hyopneumoniae.* Figure 3A presents the amino acids sequence of this protein, showing the peptide segment highlighted in gray ( ) which was selected for inclusion in the complex of the present invention, the sequence used in other works as P42 antigen is underlined ( ). Figure 3B illustrates the "ribbons" Diagram of structural model of HSP70 protein of *M*. *hyopneumoniae,* (HSP70 *M. hyopneumoniae*), the P42 antigen, derived from HSP70, used in other works (P42 antigen), and, in the lower part of Figure 3B, the region of HSP70 of *M*. *hyopneumoniae* which was selected for inclusion in the complex of the present invention (protein chosen to the complex).

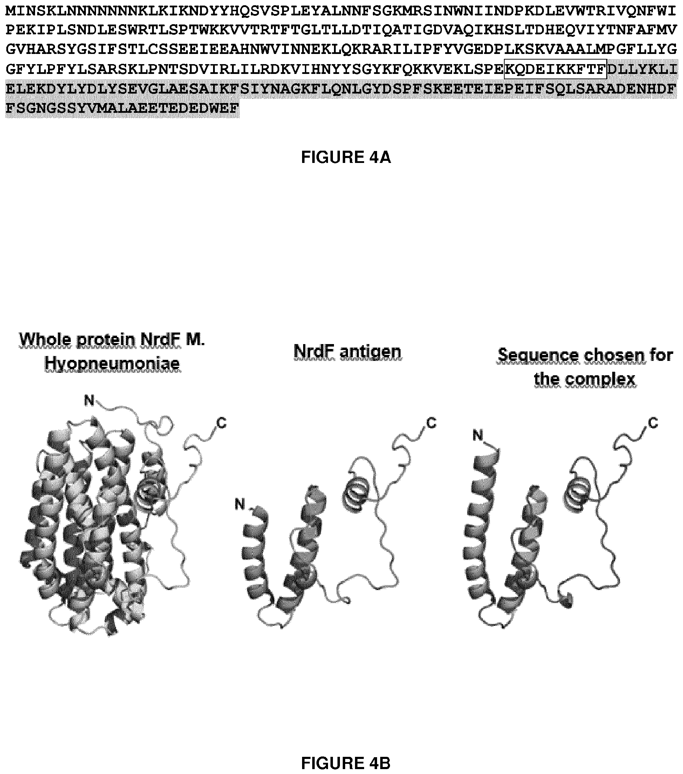

Figure 4 shows the characterization of NrdF antigen (corresponding to enzyme Ribonucleoside-diphosphate reductase) of *M*. *hyopneumoniae.* Figure 4A presents the amino acids sequence of this protein. The sequence used in other works as being the NrdF antigen is highlighted in grey ( ). For inclusion in the complex of the present invention, it was added to the sequence of NrdF antigen, highlighted in grey ( ), the outlined amino acids (| |), in order to improve the stability of antigen. Figure 4B illustrates the "ribbons" Diagram of whole NrdF protein (whole NrdF protein of *M*. *hyopneumoniae*), of NrdF antigen used in other works (NrdF antigen) and protein derived from NrdF of *M*. *hyopneumoniae* which was selected for inclusion in the complex of the present invention (sequence chosen for complex).

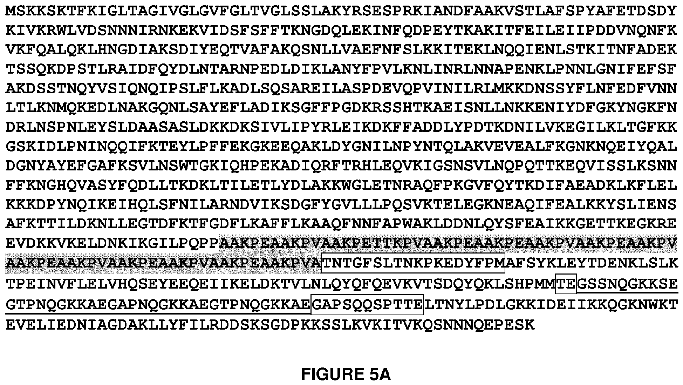

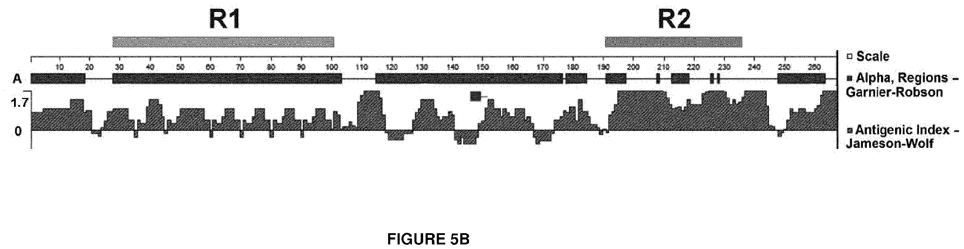

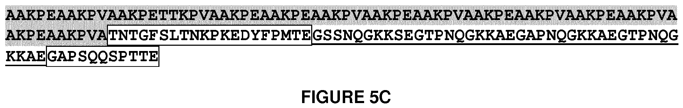

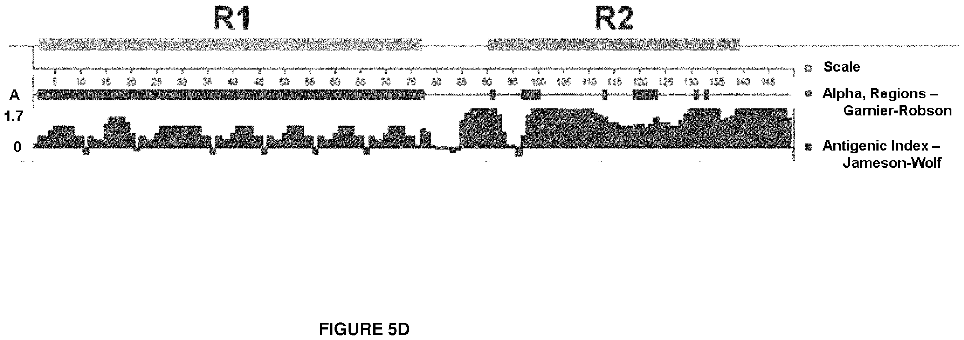

Figure 5 shows the characterization of antigen P97 de *M*. *hyopneumoniae.* Figure 5A presents the amino acids sequence of this protein, showing the segments highlighted in gray ( ) and marked with underline ( ), corresponding to the sequences R1 and R2, respectively, responsible for the function of adhesion to the epithelial tissue. Segments containing these sequences, and including the outlined segment (| |), were selected for inclusion in the complex of the present invention. Figure 5B illustrates the analysis of C-terminal sequence of P97 protein in the PROTEAN program, showing the prediction of secondary alpha-helix structures and antigenicity index. Figure 5C shows the amino acids sequence containing the regions R1 and R2, which was chosen for inclusion in the complex of invention and the Figure 5D illustrates the analysis of this sequence, by the PROTEAN program.
Figure 6 shows the characterization of antigen MHP271 de *M*. *hyopneumoniae.* Figure 6A presents the amino acids sequence of this protein, showing the sequences R1 and R2, responsible for adhesion, which are outlined (| |) or underlined ( ), respectively. The segments highlighted in grey ( ) were chosen for their inclusion in the protein complex of the present invention. Figure 6B illustrates the analysis of the sequence chosen for MHP271 protein in the PROTEAN program, showing the prediction of secondary alpha-helix structures and antigenicity index.
Figure 7 shows the characterization of P216 antigen of *M*. *hyopneumoniae.* Figure 7A shows the sequence of P216 protein; in which the region of adhesion to the platelets is highlighted in grey ( ). Inside this region, two antigen fragments were chosen with alpha-helix structure (predicted by the PROTEAN program) to be used as spacers ("linkers"), such fragments are marked with dashed ( ) or are underlined ( ). Figure 7B shows the derivatives segments of this antigen which were selected to be included in complex of the present invention as "linkers".
Figure 8 presents the organization of genes of *P. abyssi* exosome.
Figure 9 shows the complex surface diagram of *P. abysssi* exosome formed by the Rrp4, Rrp41 and Rrp42 proteins.
Figure 10 shows the sequence diagram of fusions that comprise the assembly/construction of complex of the invention, as well as polyproteins of Exomyc complex of the invention itself.
Figure 11 illustrates the cloning strategy of the Exomyc complex from pBluescript KS plasmid (pBSK) containing the synthetic gene in the pMRK expression vector by the sites of Ndel and Hindlll.

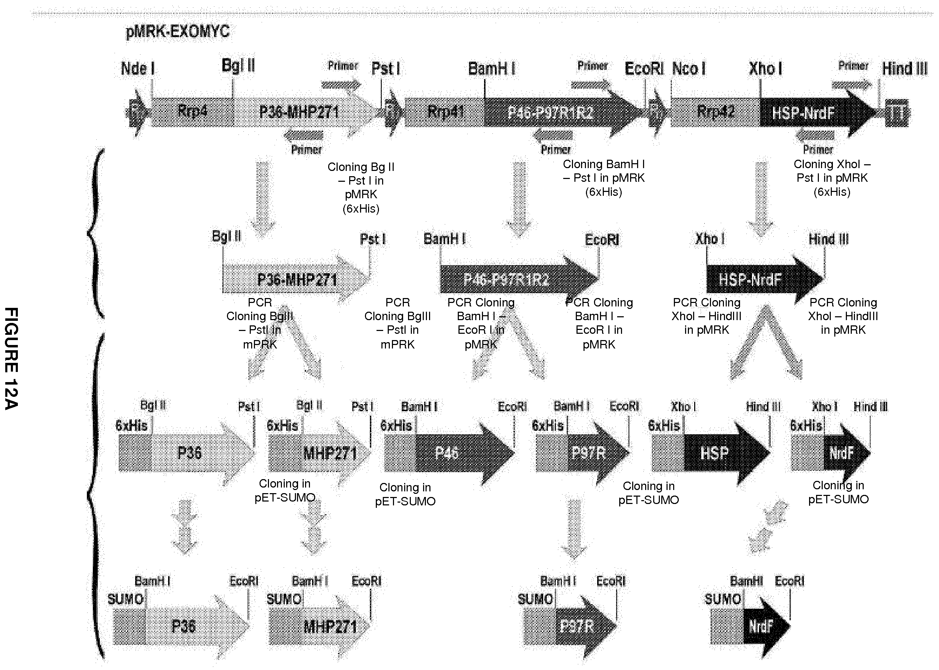

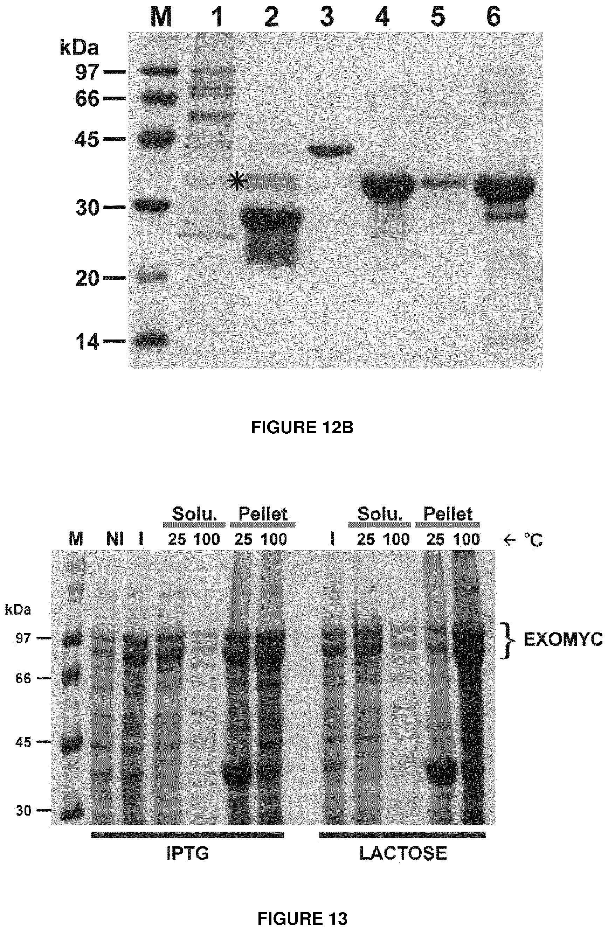

Figure 12 illustrates obtaining the antigens of *M*. *hyopneumoniae* separately recombinantly. Figure 12A shows the strategies for performing the antigens subcloning from the initial synthetic gene of the Exomyc complex. Plasmids were constructed for expressing the following recombinant proteins: 6xHis-SUMO-P36, 6xHis-SUMO-MHP271, 6xHis-P46, 6xHis-SUMO-P97R1 R2, 6xHis-HSP and 6xHis-SUMO-NrdF. Figure 12B shows the purified recombinant antigens: M.- Denotes the molecular mass marker, 1.- Denotes the protein 6xHis-SUMO-P36, 2.- Denotes the protein 6xHis-SUMO-MHP271 , 3.- Denotes the protein 6xHis-P46, 4.- Denotes the protein 6xHis-SUMO-P97R1 R2, 5.-Denotes the protein 6xHis-HSP, 6.- Denotes the protein 6xHis-SUMO-NrdF. An asterisk indicates the migration of protein 6xHis-SUMO-MHP271.
Figure 13 illustrates the expression of polyproteins of EXOMYX complex in Escherichia coli strain BL21 (DE3) transformed with the pMRK-EXOMYC plasmid. The bacterial lysate was incubated for 30 minutes at 25 and 100°C, cooled on ice for 5 minutes and subjected to centrifugation for 15 minutes at 12000xg. M.- Denotes the molecular mass marker; NI- denotes the non-induced material; I.- denotes the culture induced with IPTG (Isopropial thiogalactoside) or Lactose, Solu. - denotes the soluble protein after centrifugation. Pellet.- denotes the sedimented protein during centrifugation.
Figure 14 illustrates the purification result of EXOMYX complex expressed in *Escherichia coli*, by tangential filtration. M.- Denotes the molecular mass marker; 1.- denotes the analysis of 2 µL of purified protein; 2.- denotes the analysis of 4 µL of purified protein.
Figure 15 illustrates the analysis by ELISA of anti-P46 antibodies during the immunization of porcines with vaccines containing the Exomyc complex.
Figure 16 illustrates the analysis by ELISA of anti-HSP antibodies during the immunization of porcines with vaccines containing the Exomyc complex.
Figure 17 illustrates the analysis by ELISA of anti-P97R1 R2 antibodies during the immunization of porcines with vaccines containing the Exomyc complex.
Figure 18 illustrates the analysis by ELISA of anti-NrdF antibodies, C-terminal portion during the immunization of porcines with vaccines containing the Exomyc complex.
Figure 19 illustrates the analysis by Western Blot of anti-P36 antibodies during the immunization of porcines with vaccines containing the Exomyc complex. Group A.- Animals immunized with 1 ml of formulation (200 µg of Exomyc complex); Group B.- Animals immunized with 2 ml of formulation (400 µg of Exomyc complex).
Figure 20 shows image examples of lungs of animals sacrificed after 140 days of a single immunization, few (animal A6, of group immunized with 1 ml of the formula) or no injury (animal B7, of group immunized with 2 ml of formula).

### Detailed Description of the Invention

As described above, a first aspect of the invention is obtaining the three polyproteins corresponding to the complex based on *P. abyssi* exosome carrying copies of vaccine antigens of *M*. *hyopneumoniae.* To reach this goal, firstly the antigenic proteins of *M*. *hyopneumoniae* were selected, candidates to provide protection against infection caused by this bacterium. After the selection of candidate proteins, the great challenge was to choose the sequence fragments of each of these proteins that maintain the immunogenic activity and stability of the final polyprotein and that were able to merge with the *P. abyssi* exosome to obtain the aimed complex.

The term plasmid pMRK, as used herein, means a vector derived from the pAE vector, in which the following changes were performed: (a) substitution of the antibiotic resistance gene; and (b) introduction of the "parent" partition sequence of plasmid pSC101 (this paragraph was removed from the application of "Vectors" to give descriptive sufficiency to the employment of pMRK vector in obtaining the recombinant protein of the invention (EXOMYC).

The term "polyprotein" is used herein to name the only polypeptide sequence containing sequences of different proteins. The terms used as "complex of proteins" and "Exomyc complex of proteins" are used herein as synonyms with each other and means the complex resulting of binding or coupling of polyproteins identified as SEQ ID NO:26, SEQ ID NO:27 and SEQ ID NO:28, including the fragments and the conservatively modified variants having at least 85% of identity with the SEQ ID NO:26, SEQ ID NO:27 and SEQ ID NO:28. Said synonymous terms also cover the meaning of polyproteins encoded by synthetic gene of the invention identified by SEQ ID NO:25, or by a synthetic gene having at least 75% of identity with respect to SEQ ID NO:25.

### Selection of candidate antigenic proteins of M. hyopneumoniae

The present invention is based on the construction of a complex of proteins present in the *P. abyssi* exosome, said construction being formed from three copies of each specific antigen of *M*. *hyopneumoniae.* The study that resulted in the polyprotein construction of the invention was initiated by selection of antigens defined below:
P36 L-lactate dehydrogenase, whole protein.
P46 46kd surface antigen, whole protein.
HSP-2C two domains of C-terminal extreme of HSP70.
NrdF Three alpha-helices of C-terminal extreme of NrdF protein (Ribonucleoside-diphosphate reductase).
P97 R1 and R2 Fragments of P97, responsible for binding to cilia.
MHP271 R1 and R2 Fragments of MHP271, responsible for binding to cilia.
P216 Two segments of alpha-helices of domain of binding to platelets of P216, used as linkers (spacers between the proteins).

This selection was performed based on extensive bibliographic review. The amino acids sequences of antigens were obtained from GenBank, looking to select those belonging to pathogenic strains of *M*. *hyopneumoniae.*

### P36 (Swiss Prot: POCOJ2.1)

The P36 antigen is a cytoplasmic enzyme, lactate dehydrogenase that is highly immunogenic. Serum from infected animals recognizes with great intensity this antigen, why initially it was used for the diagnostic test, due to its specificity for *M*. *hyopneumoniae* (Stipkovits et al., Stipkovits L, Nicolet J, Haldimann A, Frey J. (1991) "Use of antibodies against the P36 protein of Mycoplasma hyopneumoniae for the Identification of M. hyopneumoniae strains". Mol Celi Probes. 5:451 -457). In pigs experimentally infected with *M*. *hyopneumoniae,* two peaks were detected in the production of antibodies against P36 (Frey J, Haldimann A, Kobisch M, Nicolet J. (1994) "Immune response against the L-lactate dehydrogenase of Mycoplasma hyopneumoniae in enzootic pneumonia of swine". Microb Pathog. 17:313-322). The first, a relatively low response was detected in the period between 5 and 10 weeks after infection, time that the clinical signs are apparent, including lung lesions. This response anti-P36 occurred simultaneously with a strong response of antibodies against membrane antigens of *M*. *hyopneumoniae* extracted with Tween 20. The second peak response happens from the twelfth week after infection, when the signs of the disease and the infectious agent have disappeared and do not have more antibodies against the proteins of membrane (Frey et al., 1994).

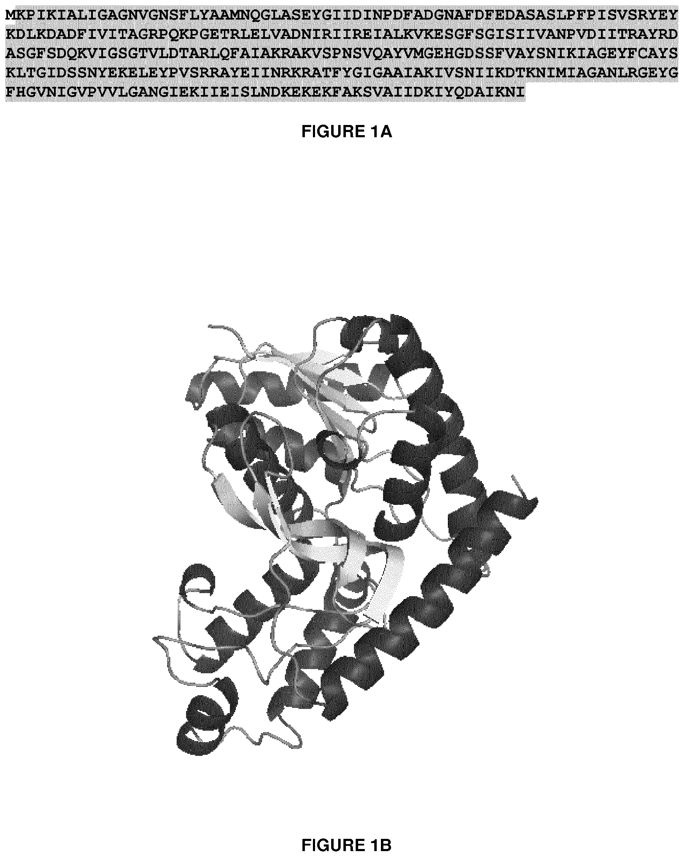

The amino acids sequence do P36 antigen, as filed in the database SwissProt (access P0C0J2.1) (shown in Figure 1 A) It was subjected to modeling the three-dimensional structure in the automatic mode on the server SWISS-MODEL (http://swissmodel.expasy.org). The chosen mold was the PDB 1 EZ4 structure which corresponds to the crystal structure of L-lactate dehydrogenase of *Lactobacillus pentoses.* The obtained structural model is shown in Figure 1 B.

In functional status, the lactate dehydrogenase forms a tetramer, which can dissociate depending on the pH and the presence of urea (Mayr U, Hensel R, Kandler O. (1980) "Factors affecting the quaternary structure of the allosteric L-lactate dehydrogenase from Lactobacillus casei and Lactobacillus curvatus as investigated by hybridization and ultracentrifugation". Eur J Biochem. 1 10:527-538). The tetramers are formed in pH 5.0 - 5.5, whereas the monomeric form prevails to basic pH values. This fact was taken into account to not promote protein aggregates of the assembled structure that forms the complex of the present invention.

As shown in Figure 1 B, this structure has the characteristics of a globular, compact conformation, without domains. In the immunization tests, this protein was used in its complete form, for this reason, the amino acids sequence corresponding to the complete P36 antigen, less the methionine of the beginning of translation, was selected to be included in the complex of the present invention. Thus, this sequence was renamed herein as SEQ ID NO:1 (Table 1).

### 2. P46 (GenBank: AAV27597.1)

P46 is a surface protein of *M*. *hyopneumoniae,* highly antigenic. Similarly to the P36, P46 is used in diagnostic tests to specific identification of this mycoplasma ((Futo S, Seto Y, Okada M, Sato S, Suzuki T, Kawai K, Imada Y, Mori Y. (1995) "Recombinant 46-kilodalton surface antigen (P46) of Mycoplasma hyopneumoniae expressed in Escherichia coli can be used for early specific diagnosis of mycoplasmal pneumonia of swine by enzyme-linked immunosorbent assay". J Clin Microbiol. 33:680-683; Cheikh Saad Bouh K, Shareck F, Dea S. (2003) "Monoclonal antibodies to Escherichia co//-expressed P46 and P65 membranous proteins for specific immunodetection of Mycoplasma hyopneumoniae in lungs of infected pigs". Clin Diagn Lab Immunol. 10:459-468; Okada M, Asai T, Futo S, Mori Y, Mukai T, Yazawa S, Uto T, Shibata I, Sato S. (2005) "Serological diagnosis of enzootic pneumonia of swine by a double-sandwich enzyme-linked immunosorbent assay using a monoclonal antibody and recombinant antigen (P46) of Mycoplasma hyopneumoniae". Vet Microbiol. 105:251 -259).

In proteome studies with respect to membrane proteins of *M*. *hyopneumoniae,* the protein P46, as well as the ciliary adhesin P97, was identified in different types of extracts, confirming its association with the membrane of mycoplasma (Tavares, CP. (2010) "Análise proteômica de proteins de membrana de Mycoplasma hyopneumoniae e Mycoplasma Synoviae". Dissertation (Masters in Biochemistry), Post Graduation Course in Biochemistry, Federal Unviersity of Santa Catarina, Florianópolis, Brazil).

In the joint immunization with many antigens in the form of DNA vaccines, only P46 could induce humoral response, whereas all other antigens, in this type of immunization, induced cellular response.

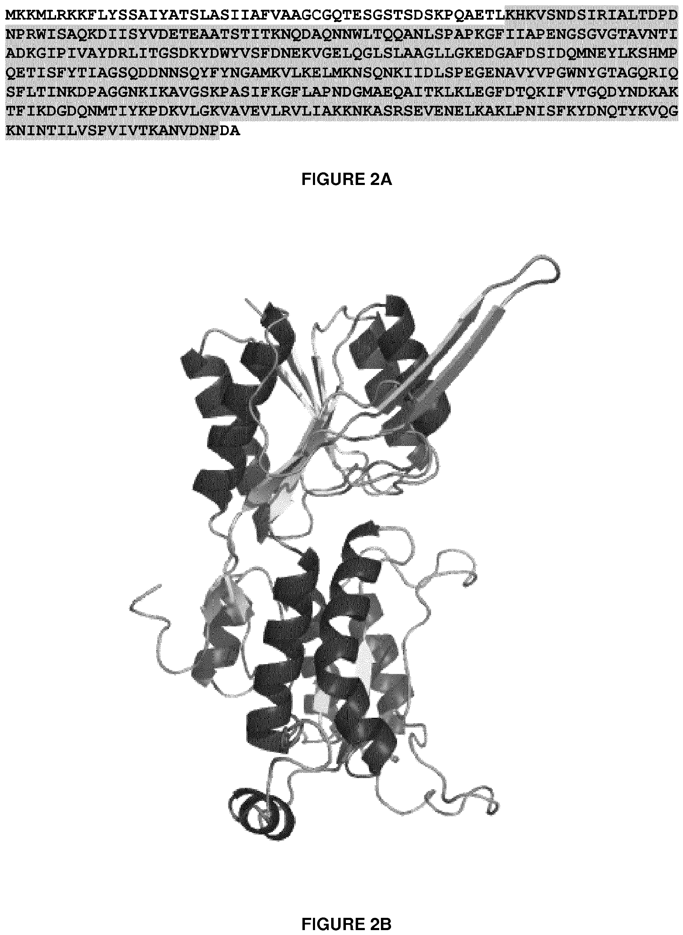

The amino acids sequence of P46 antigen, as filed in GenBank (access AAV27597.1) is shown in Figure 2A. This amino acids sequence was subjected to modeling the three-dimensional structure, in automatic mode on a server SWISS-MODEL. The chosen mold was the PDB 3MA0 structure, corresponding to the Crystal structure of Xylose Binding Protein of *Escherichia coli.* In other words, the P46 protein participate in the transport of sugars into the cells of the mycoplasma, which represents an important target for the development of vaccine, to the extent that the antibodies generated by the introduction of the P46 antigen in the host may inhibit the transport of sugars, which can be vital to the *M*. *hyopneumoniae.*

The model represented in Figure 2B presents two globular domains and a length of two beta sheets that leave the main structures. The amino acids sequences interweave between the two domains, which makes it impossible to clone only the domains separately. The N-terminal extreme of the model begins in the amino acid number 55. Probably, the first 54 amino acids correspond to the signal sequence, which determines the output of the bacterial cytoplasm, and to an anchoring region in the plasmatic membrane. For the hydrophobic nature of this kind of segments, in the process of the present invention for construction of complex of polyproteins of the invention, it was considered correct no to include these 54 amino acids in the recombinant protein. The amino acids of P46, highlighted in grey ( ) in the protein sequence of Figure 2A, correspond to the obtained structural model, and however, were chosen to be included in the construction of complex of the invention, passing this segment to be called herein SEQ ID NO:2 (Table 1).

### 3. P42 (HSP70) (Swiss Prot: Q49539.1)

P42 is derived from a major protein, P65, which is a cytoplasmic protein of *M*. *hyopneumoniae* belonging to the family of HSP70 (Proteína de Choque Térmico, Heat Shock Protein, of 70 kDa) (Chou SY, Chung TL, Chen RJ, Ro LH, Tsui PI, Shiuan D. (1997) "Molecular cloning and analysis of a HSP (heat shock protein)-like 42 kDa antigen gene of Mycoplasma hyopneumoniae". Biochem Mol Biol Int. 41:821-831). In experiments of immuno-screening with expression libraries constructed with genomic DNA of *M*. *hyopneumoniae,* an antigen of 42 kDa was identified (P42) which is a truncated derivative of the HSP70. This protein, P42, It is highly immunogenic and has also been used in diagnostic tests and as a vaccine antigen (Chou SY, Chung TL, Chen RJ, Ro LH, Tsui PI, Shiuan D. (1997) "Molecular cloning and analysis of a HSP (heat shock protein)-like 42 kDa antigen gene of Mycoplasma hyopneumoniae". Biochem Mol Biol Int. 41:821-831).

Vaccination with DNA formulation expressing P42 induces an immune response both humoral and cell. Serum from the immunized animals was able to inhibit the growth of *M*. *hyopneumoniae in vitro* (Chen YL, Wang SN, Yang WJ, Chen YJ, Lin HH, Shiuan D. (2003) "Expression and immunogenicity of Mycoplasma hyopneumoniae heat shock protein antigen P42 by DNA vaccination". Infect Immun. 71:1155-1160).

The amino acids sequence do HSP70 antigen of *M*. *hyopneumoniae* filed in Swiss Prot (access Q49539.1) is shown in Figure 3A. This amino acids sequence was submitted to modeling the three-dimensional structure, in automatic mode on a server SWISS-MODEL. The automatically chosen mold was the PDB 1 Q5L structure, the HPS70 of *Eschehchia coli*, representing the first model in Figure 3B.

Analyzing amino acids sequence, it was verified that P42 antigen (part of the underlined sequence ( ) in the amino acids sequence of Figure 3A) contains the truncated NBD domain (Nucleotide Binding Domain), as represented as in the second model in Figure 3B. The expression, recombinantly, of partial domains can result in proteins with low solubility, which is why it was included in the complex of the present invention, only the SBD domain (Substrate Binding Domain) together with the alpha-helices domain in extreme C-terminal (see model in the lower part of Figure 3B). These two domains make up a protein 27 kDa. The corresponding amino acids sequence is highlighted in grey ( ) (see Figure 3A).

The amino acids sequence corresponding to the segment containing said two domains was selected to be included in the complex of the invention, passing this segment to be called herein SEQ ID NO:3 (Table 1).

### 4. NrdF (Gen Bank AAV27433.1)

During the immuno-screening in an expression library, constructed from the genomic DNA of *M*. *hyopneumoniae* using a swine hyperimmune serum, it was identified and cloned a DNA fragment expressing a polypeptide of 11 kDa (Fagan PK, Djordjevic SP, Eamens GJ, Chin J, Walker MJ. (1996) "Molecular characterization of a ribonucleotide reductase (nrdF) gene fragment of Mycoplasma hyopneumoniae and assessment of the recombinant product as an experimental vaccine for enzootic pneumonia". Infect Immun. 64:1060-1064). The analysis done by sequencing showed that it is part of a subunit of the Ribonucleotide-diphosphate reductase enzyme of *M*. *hyopneumoniae.* Animals immunized by vaccination with this fragment of the protein and challenged with a virulent strain of *M*. *hyopneumoniae* had lung disease (measured by the method of mean Goodwin lung score) significantly reduced with respect to control of non-vaccinated pigs (Fagan PK, Djordjevic SP, Eamens GJ, Chin J, Walker MJ. (1996) "Molecular characterization of a ribonucleotide reductase (nrdF) gene fragment of Mycoplasma hyopneumoniae and assessment of the recombinant product as an experimental vaccine for enzootic pneumonia". Infect Immun. 64:1060-1064).

Since then, various immunization papers have been published using exactly the same sequence isolated in 1996 (example: Chen AY, Fry SR, Daggard GE, Mukkur TK. (2008) "Evaluation of immune response to recombinant potential protective antigens of Mycoplasma hyopneumoniae delivered as cocktail DNA and/or recombinant protein vaccines in mice". Vaccine. 26:4372-4378).

The amino acids sequence of NrdF antigen, as filed in GenBank (access AAV27433.1) is shown in Figure 4A. This amino acids sequence was subjected to modeling the three-dimensional structure, in automatic mode on the server SWISS-MODEL. The automatically chosen mold was the PDB 4DRO structure, corresponding to the crystal structure of dimanganese (II) NrdF of *Bacillus subtilis.*

As can be seen, the NrdF of *M*. *hyopneumoniae* (Figure 4B, first model) presents a structure consisting essentially of alpha-helices joined by small "beta-turn" segments.

The sequence isolated in 1996 corresponds to the last two alpha-helices of this structure, wherein, as was selected from a library obtained by random cuts in the genome, the N-terminal extreme of the cloned sequence - Figure 4B, segment correspondents to the amino acids highlighted in grey ( ) in the sequence of complete NrdF protein shown above - covers only part of one of the alpha-helices.

For the purpose of stabilizing said segment, it was selected the whole alpha-helix. Therefore, the outlined amino acids were added (| |) in the sequence shown in Figure 4A, corresponding to the third model of Figure 4B. The addition of amino acids to complete the alpha helix stabilizes structure to be expressed in the context of complex of the present invention.

Actually, the NrdF antigen corresponds to only a part of a major protein, globular and that does not present domains structure. Nevertheless, analyzing the surface of the protein to be expressed, it appears that this does not display hydrophobic regions which can influence the expression and purification of recombinant protein.

However, the amino acids sequence selected to be included in the complex of the present invention, correspondents to the outlined segment (| |) and highlighted in grey ( ) in Figure 4A, is the SEQ ID NO:4 (Table 1).

### 5. P97 (Gen Bank AAV27763.1)

The p97 adhesin of M. *hyopneumoniae,* first described in 1995 (Zhang Q, Young TF, Ross RF. (1995) "Identification and characterization of a Mycoplasma hyopneumoniae adhesion". Infect Immun. 63:1013-1019) (King KW, Faulds DH, Rosey EL, Yancey RJ Jr. (1997) "Characterization of the gene encoding Mhp1 from Mycoplasma hyopneumoniae and examination of MhpVs vaccine potential". Vaccine. 15:25-35) is considered the main adhesin of mycoplasma, responsible for the adhesion of bacterium to lung cilia.

The specific sequences of P97 protein known as R1 (also called P97R1) and R2 are responsible for this adhesion function (Minion FC, Adams C, Hsu T. (2000) "FM region of P97 mediates adherence of Mycoplasma hyopneumoniae to swine cilia". Infect Immun. 68:3056-3060) (Jenkins C, Wilton JL, Minion FC, Falconer L, Walker MJ, Djordjevic SP. (2006) "Two domains within the Mycoplasma hyopneumoniae cilium adhesin bind heparin". Infect Immun. 74:481 -487).

Immunization experiments with vectored vaccines of whole protein (Shimoji Y, Oishi E, Muneta Y, Nosaka H, Mori Y. (2003) "Vaccine efficacy of the attenuated Erysipelothrix rhusiopathiae YS-19 expressing a recombinant protein of Mycoplasma hyopneumoniae P97 adhesin against mycoplasmal pneumonia of swine". Vaccine. 21:532-537) or the C-terminal portion, which contains the R1 and R2 sequences (Okamba FR, Arella M, Music N, Jia JJ, Gottschalk M, Gagnon CA. (2010) "Potential use of a recombinant replication-defective adenovirus vector carrying the C-terminal portion of the P97 adhesin protein as a vaccine against Mycoplasma hyopneumoniae in swine". Vaccine. 28:4802-4809) resulted, after the challenge with *M. hyopneumoniae,* in significant reduction of lung lesions in swine.

Immunogenicity of recombinant vaccines containing the P97R1 sequence and R2 in lab animals is widely characterized in the literature, (Lee SH, Lee S, Chae C, Ryu DY. (2014) "A recombinant Chimera comprising the R1 and R2 repeat regions of M. hyopneumoniae P97 and the N-terminal region of A. pleuropneumoniae Apxlll elicits immune responses". BMC Vet Res. 10:43. doi: 10.1 186/1746-6148-10-43; Barate AK, Cho Y, Truong QL, Hahn TW. (2014) "Immunogenicity of IMS 1 1 13 plus soluble subunit and chimeric proteins containing Mycoplasma hyopneumoniae P97 C-terminal repeat regions". FEMS Microbiol Lett. doi: 10.1111/1574-6968.12389; Conceição FR, Moreira AN, Dellagostin OA. (2006) "A recombinant Chimera composed of R1 repeat region of Mycoplasma hyopneumoniae P97 adhesin with Escherichia coli heat-labile enterotoxin B subunit elicits immune response in mice". Vaccine. 24:5734-5743; Chen AY, Fry SR, Forbes-Faulkner J, Daggard G, Mukkur TK. (2006) "Evaluation of the immunogenicity of the P97R1 adhesin of Mycoplasma hyopneumoniae as a mucosal vaccine in mice". J Med Microbiol. 55:923-929). However, aP97 is an important target for the development of a recombinant vaccine against *M*. *hyopneumoniae.*

The immunization with the commercial vaccine (bacterial) does not induce the formation of antibodies against the P97 protein, that would explain the fact that immunization with bacterins does not prevent the initial colonization of lung tissue (Chen AY, Fry SR, Daggard GE, Mukkur TK. (2008) "Evaluation of immune response to recombinant potential protective antigens of Mycoplasma hyopneumoniae delivered as cocktail DNA and/or recombinant protein vaccines in mice". Vaccine. 26:4372-4378). Probably, the *M*. *hyopneumoniae* in culture does not express the genes which determine the virulance, as P97, whereby no response is generated against this protein, and probably against other important factors in the infection process, in the immunization with bacterin.

The amino acids sequence of P97 antigen (the P97 gene encodes a protein of 1108 amino acids), as filed in GenBank (access AAV27763.1) is shown in Figure 5A. The R1 and R2 sequences are highlighted in grey ( ) or marked with underline ( ), respectively. For the construction of complex of the present invention, it was decided to include the R1 and R2 sequences.

As the homologous proteins to P97 with solved structure are not known, it was not possible to model the structure of this protein. For this reason, only the amino acids sequence was analyzed using the PROTEAN program of Lasergene. This program allows to analyze and to visualize once the secondary structure, hydrophobicity, antigenicity, among other characteristics, using different algorithms.

In Figure 5B, it is evident that the R1 sequence corresponds to an alpha-helix (rectangles) in the P97 protein. R2 already has small segments in alpha-helix and several flexible "beta-turn". However, the antigenicity calculation (diagram) indicates that R2 is more antigenic than R1.

For the construction of complex of the present invention, it was employed alpha-helix containing the R1 protein, as well as the region of R2 protein, excluding the intermediate sequences which present poor immunogenicity (see Figure 5C).

As can be seen in Figure 5D, the sequences with low immunogenicity were removed with success. The analysis of "surface probability" (PROTEAN) indicates that the R2 region is more exposed than the R1, which is probably due to the fact that R1 has only one alpha-helix, which can help in a greater exposure of this region.

Thus, the amino acids sequence selected to be included in the complex of the present invention, corresponding to the P97R1 R2 segment, is SEQ ID NO:5 (Table 1).

### 6. MHP271 (Gen Bank AAV27492.1)

The MHP271 protein has recently been characterized as a paralogue of P97, which is able to bind to Heparin and to Fibronectin of lung cilia of swines (Deutscher AT, Jenkins C, Minion FC, Seymour LM, Padula MP, Dixon NE, Walker MJ, Djordjevic SP. (2010) "Repeat regions R1 and R2 in the P97 paralogue Mhp271 of Mycoplasma hyopneumoniae bind heparin, fibronectin and porcine cilia. Mol Microbiol. 78:444-458).

There is still no immunization data with this protein, however, the study clearly shows that it is associated to the membrane of *M*. *hyopneumoniae.* The R1 and R2 sequences, similar to those described for the P97 antigen, were also characterized and it was verified that they are involved in binding to host proteins, which makes MHP271 an excellent vaccine candidate (Deutscher AT, Jenkins C, Minion FC, Seymour LM, Padula MP, Dixon NE, Walker MJ, Djordjevic SP. (2010) "Repeat regions R1 and R2 in the P97 paralogue Mhp271 of Mycoplasma hyopneumoniae bind heparin, fibronectin and porcine cilia. Mol Microbiol. 78:444-458).

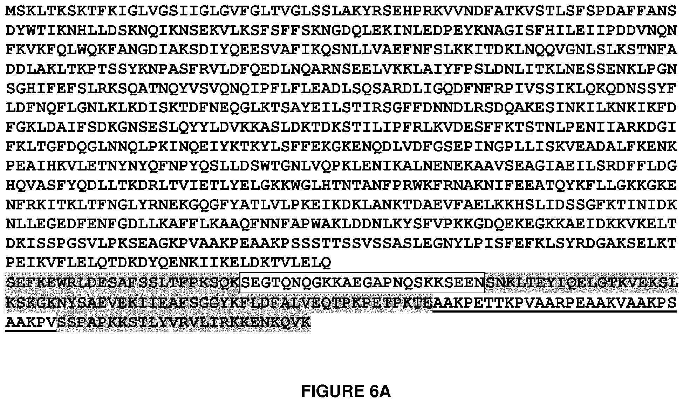

The amino acids sequence of MHP271 antigen (the MHP2717 gene encodes a protein which is also large, 1052 amino acids), as filed in GenBank (AAV27492.1), is the shown in Figure 6A. In Figure 6A, the R1 and R2 sequences are those outlined (| |) or marked with underline ( ), respectively.

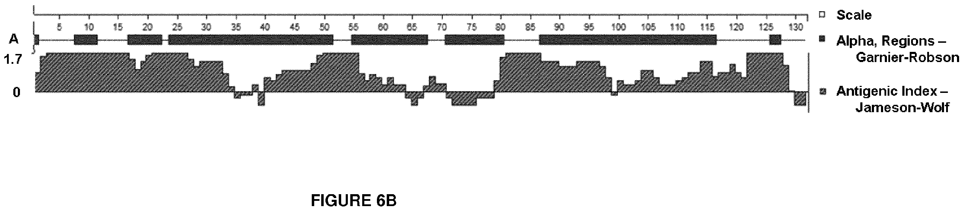

The analysis of amino acids sequence using the PROTEAN program of Lasergene, sown in Figure 6B, indicates that the N-terminal region of this protein, containing R1 and R2 sites, probably corresponds to a domain with secondary structures in alpha-helix. For this reason, all this region was separated with the aim to include the sequences with increased antigenicity, in addition to R1 and R2 proteins.

Thus, a amino acids sequence selected to be included in the complex of the present invention, corresponding to the MHP721 -C segment (see Figure 6C), is SEQ ID NO: 6 (Table 1).

### 7. P216 (Gen Bank AAZ53862.1)

*M. hyopneumoniae* has a variety of surface proteins of great molecular mass, with sequences homologous to the P97. However, it is possible to obtain redundant proteins for the function of binding to lung tissue cilia. Even if the P97 is the most abundant, its lock would not prevent colonization by the existence of other adhesins, as MHP271 mentioned above.

P216 is other protein characterized recently. Proteome studies showed that it is associated to the membrane and contributes significantly to the membrane architecture of *M*. *hyopneumoniae,* which makes this protein an excellent vaccine candidate (Wilton J, Jenkins C, Cordwell SJ, Falconer L, Minion FC, Oneal DC, Djordjevic MA, Connolly A, Barchia I, Walker MJ, Djordjevic SP. (2009) "Mhp493 (P216) is a proteolytically processed, cilium and heparin binding protein of Mycoplasma hyopneumoniae". Mol Microbiol. 71 :566-582). Sequences of R1 type or R2 type were not identified, as occurred in P97 and MHP271 proteins, but it was determined the region of the protein that would be responsible for adhesion (Bogema DR, Deutscher AT, Woolley LK, Seymour LM, Raymond BB, Tacchi JL, Padula MP, Dixon NE, Minion FC, Jenkins C, Walker MJ, Djordjevic SP. (2012) "Characterization of cleavage events in the multifunctional cilium adhesin Mhp684 (P146) reveals a mechanism by which Mycoplasma hyopneumoniae regulates surface topography". MBio. 3(2). pii: e00282-11).

There are no immunization experiments with this protein, However it is known that serum from pigs infected with the P216 of *M*. *hyopneumoniae* recognizes this protein (Wilton J, Jenkins C, Cordwell SJ, Falconer L, Minion FC, Oneal DC, Djordjevic MA, Connolly A, Barchia I, Walker MJ, Djordjevic SP. (2009) "Mhp493 (P216) is a proteolytically processed, cilium and heparin binding protein of Mycoplasma hyopneumoniae". Mol Microbiol. 71:566-582).

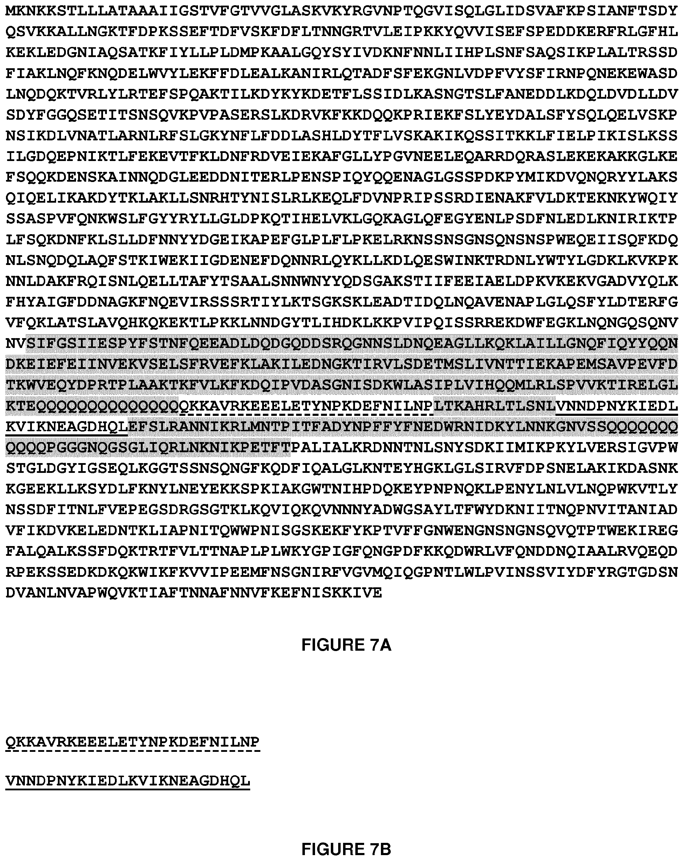

The amino acids sequence do P216 antigen (other protein as large as P97 and MHP271 protein, with 1885 amino acids), as filed in GenBank (access AAZ53862.1), is shown in Figure 7A).

The sequence highlighted in grey ( ), in Figure 7A, corresponds to the fragment of P216 responsible for binding to host molecules (Bogema DR, Deutscher AT, Woolley LK, Seymour LM, Raymond BB, Tacchi JL, Padula MP, Dixon NE, Minion FC, Jenkins C, Walker MJ, Djordjevic SP. (2012) "Characterization of cleavage events in the multifunctional cilium adhesin Mhp684 (P146) reveals a mechanism by which Mycoplasma hyopneumoniae regulates surface topography". MBio. 3(2). pii: e00282-1 1). With the aid of PROTEAN program, in this fragment, two predicted sequences were selected as alpha-helix and with high immunogenicity, marked, respectively, with dash ( ) or with underline ( ) in Figure 7A and shown separately in Figure 7B. These sequences were selected to serve as a linker (spacer) among the proteins in the complex of the present invention.

Thus, the amino acids sequences selected to be included in the complex of the present invention, corresponding to the chosen fragments in the sequence of P216 antigen (see Figure 7B), ate the sequences SEQ ID NO:7 and SEQ ID NO:8 (Table 1).

Table 1 contains the summary of antigenic proteins of *M*. *hyopneumoniae* and the amino acids sequences selected from these proteins for inclusion in the Exomyc complex of the present invention, as well as synthetic gene designed to encode said selected amino acids sequences.

**Table 1: M. hyopneumoniae antigens included in Exomyc complex**

| Protein | GenBank | N° aa | Consideration for synthesis | Synthetic Gene | Encoded Protein |
|---|---|---|---|---|---|
| P36 | SP POCOJ2.1 | 315 | 2-315 | SEQ ID NO: 12 | SEQ ID NO: 1 |
| P46 | BG AAV25597.1 | 419 | 50-417 | SEQ ID NO: 13 | SEQ ID NO: 2 |
| HSP70 | SP Q49539.1 | 600 | 356-600 | SEQ ID NO: 14 | SEQ ID NO: 3 |
| NrdF | GB AAV27433.1 | 353 | 248-353 | SEQ ID NO: 15 | SEQ ID NO: 4 |
| P97 | GB AVV27763.1 | 1108 | 815-907 (R1) | SEQ ID NO: 16 | SEQ ID NO: 5 |
| | | | 979-1031 (R2) | | |
| MHP271 | GB AAV27763.1 | 1052 | 891-1052 | SEQ ID NO: 17 | SEQ ID NO: 6 |
| P216 | GB AVV27492.1 | 1885 | 1272-1296 | SEQ ID NO: 18 | SEQ ID NO: 7 |
| | | | 1309-1332 | SEQ ID NO: 19 | SEQ ID NO: 8 |

### Pyrococcus abyssi Exosome

*Pyrococcus abyssi* is a hyperthermophilic archaebacterium, who lives near hydrothermal vents on the ocean floor. It supports temperatures above 100°C and pressure up to 200 atmospheres, not representing, however, danger to the health of humans, plants or animals.

To withstand such high temperatures and pressures, the evolution made *P. abyssi* to have equally stable proteins resistant to denaturation by temperature or pressure. A derivative complex of *P. abyssi,* called exosome, was characterized extensively (Ramos CR, et al. (2006) The Pyrococcus exosome complex: structural and functional characterization. J Biol Chem. 281 :6751 -6759; Navarro MV et al. 2008. Insights into the mechanism of progressive RNA degradation by the archaeal exosome. J Biol Chem. 283:14120-14131).

The complex consists of three copies of three different proteins (9 subunits in total). Its structural characterization allowed the identification of two amino acids in one of the subunits which is the most important for the enzymatic activity of the complex (Navarro MV et al. 2008. Insights into the mechanism of progressive RNA degradation by the archaeal exosome. J Biol Chem. 283:14120-14131). Substitutions of said amino acids by others inhibit the enzymatic activity of this complex, which participates in cell RNA processing and degradation.

The ease of assembly of this complex in the cytoplasm of E. coli, combined with its high stability and solubility, suggests that exosome can be used as peptides and proteins carrier, because the three-dimensional structure shows that the amino and carboxyl extremes of the subunits protrude out of the complex.

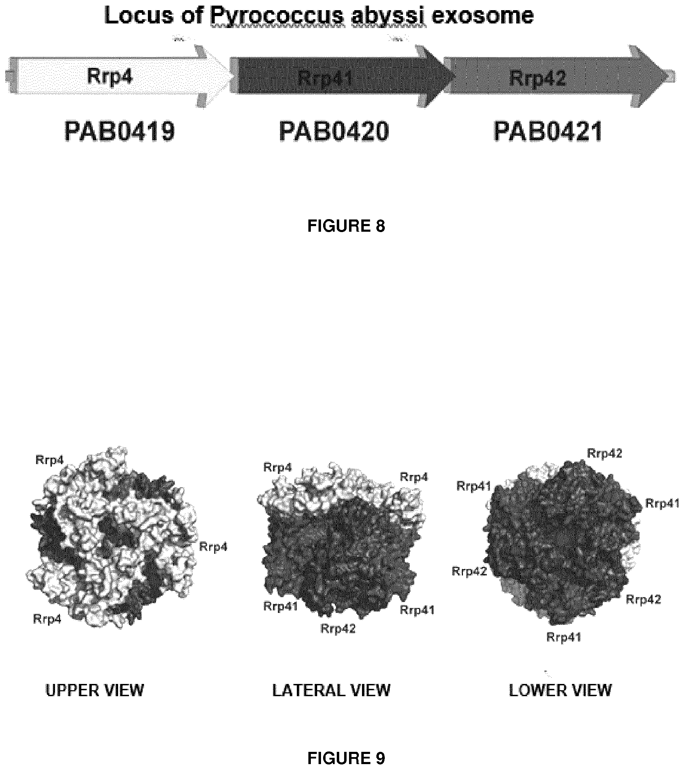

Genes encoding proteins that comprise the complex of *Pyrococcus abyssi* GE5 exosome, are located at a single locus in the genome of this archaebacterium (Koonin EV et al. (2001) "Prediction of the archaeal exosome and its connections with the proteasome and the translation and transcription machineries by a comparative-genomic approach". Genome Res. 11:240-252) (see Figure 8). This locus contains three genes, PAB0419, PAB0420 and PAB0421 encoding the Rrp4, Rrp41 and Rrp42 proteins, respectively.

The complex of proteins comprising the *P. abyssi* exosome is illustrated in Figure 9. The Rrp41 and Rrp42 proteins form a hexameric disc (ring) which contains three copies of each protein. Additionally, three copies of the Rrp4 protein bind to one of the disc surfaces, to form a complete exosomic complex (Ramos CR, et al. (2006) "The Pyrococcus exosome complex: structural and functional characterization". J Biol Chem. 281 :6751 -6759).

The complex of archaea has unique characteristics that can be exploited in the development of new generation of vaccines so-called "particles similar to pathogens", using biomimetics to improve the immune response. These characteristics are:
- capacity for specific self-association when expressed in *Escherichia coli* cells,
- high expression, solubility and thermostability of the complex, which facilitates purification and formation of recombinant proteins.
- thermostability also confers greater stability of the protein during the formulation and storage of finished products, important aspect in the development of biological products.
- ability to carry more than one antigen and immunomodulatory protein, because the amino and carboxyl terminal extremes of the three proteins are exposed to the solvent, as determined structure by crystallographic.

This new methodology represents a major advance regarding the use of unique recombinant antigens in vaccines formulation that can not afford the degree of protection that is achieved with whole organisms such as bacteria and viruses inactivated (Rosenthal J.A. et al. (2014) Pathogen-like particles: biomimetic vaccine carriers engineered at the nanoscale. Current Opinion in Biotechnology (Nanobiotechnology Systems biology) 28: 51 -58).

The expression of proteins in *Escherichia* coli facilitates the production, due to the complex being resistant to high temperatures, in which proteins from E. coli which is a mesophilic bacteria, completely denature. For example, the treatment at 80°C for 30 minutes, followed by centrifugation at 5000g, precipitates almost all the E. coli proteins, allowing to obtain the complex almost pure. The complex is resistant not only at high temperatures, but also to treatments with 8M urea and the high pressures, conditions in which E. coli protein (or of any mesophylous) denaturates. This quality, plus the fact that the complex has the capacity to carry other proteins and peptides, make the exosome complex is a good candidate to charger of multiple antigens simultaneously. The present inventors are unaware of the use of similar structures for this purpose.

The amino acids sequences of *P. abyssi* exosome were obtained from GenBank. The amino acids sequences used for the design of synthetic genes correspond to those reported in GenBank to *Pyrococcus abyssi* strain GE5:

**Table 2: Proteins of P. abyssi exosome included in Exomyc complex.**

| **Protein** | **GenBank** | **Nº aa** | **Considered for synthesis** | **Synthetic Gene** | **Encoded protein** |
|---|---|---|---|---|---|
| **Rrp4** | NP_126300.1 | 265 | 2-262 | SEQ ID NO: 20 | SEQ ID NO: 9 |
| **Rrp41** | NP_126301.1 | 249 | 3-246 | SEQ ID NO: 21 | SEQ ID NO: 10 |
| **Rrp42** | NP_126302.1 | 274 | 2-274 | SEQ ID NO: 22 | SEQ ID NO: 11 |

The Rrp41 protein contains the catalytical site responsible for the RNase activity of the exosome. Since this activity is not desired for the development of vaccines, the amino acids present in the active site were changed, based in published results (Navarro MV et al. 2008. Insights into the mechanism of progressive RNA degradation by the archaeal exosome. J Biol Chem. 283:14120-14131). In this work it is shown that the double mutation R89E and K91 E in Rrp41 abolished the RNase activity of the exosome. In the synthetic gene, R89T and K91 S exchanges were performed, resulting in a smaller exchange in charge of the molecule than that which occurs in the mutations reported in the literature.

Thus, the ssRNA-binding activity of protein Rrp4 (containing the binding domains to RNA S1 and KH) was maintained, as well as the ring structure was maintained (RING) formed by Rrp41 -Rrp42 proteins. It is believed that E. coli ssRNA which is co-purified along with the exosome can activate the TLR7 receptor, and thus act as an adjuvant in vaccines based on exosome.

### Design of synthetic genes for proteins of M. hyopneumoniae P36, P46, HSP, P97R1 R2, MHP271, segments of P216 protein; and the P. abyssi: Rrp4, Rrp41 and Rrp42

The amino acids sequences used for the design of synthetic genes corresponding to those reported in GenBank as indicated in Tables 1 and 2.

For the design of synthetic genes it took into account the theory known as "Codon Harmonization" (Angov E, et al. (2011) Adjustment of codon usage frequencies by codon harmonization improves protein expression and folding. Methods Mol Biol. 705:1-13). By matching theory codons, codons called rare (which are less commonly used than other codons in a given organism) have a lower amount of tRNAs and play an important role in the folding of proteins. They are located in the "loops" of proteins, among the segments of defined secondary structure. These codons may decrease the rate of protein synthesis at the ribosome, allowing the synthesized sequence to fold before the begining of synthesis of another protein fragment.

Rare codons in genes encoding the protein shown in Tables 1 and 2, were identified using the ANACONDA program (http://bioinformatics.ua.pt/software/anaconda) fed according to the codon usage table *M*. *hyopneumoniase* and *P. abyssi,* obtained in "Codon Usage Database" (http://www.kazusa.or.jp/codon/).

The location of the amino acids encoded by "rare codons" in the three-dimensional structures obtained for each of the proteins was performed using the Swiss-Pdb-Viewe 4.0.1 program (http://spdbv.vital-it.ch/), or in the predictions of secondary structure by the PROTEAN program, when there is no structural model. In the "harmonization" of synthetic gene, the rare codons were considered encoding amino acids located in the "loops" regions between the secondary structure, alpha-helix or beta-sheet.

For the design of the synthetic gene, it was used the Gene Designer 2.0 program (DNA2.0), manually selecting "rare" codons of E. coli for each of the identified amino acids with ANACONDA programs and located in the structure with Swiss-Pdb-Viewe 4.0.1, in order to improve the folding of proteins. The Gene Designer 2.0 program was fed with the table of codons use for highly expressed proteins in E. coli (Villalobos et al. (2006) Gene Designer: a synthetic biology tool for constructing artificial DNA segments. BMC Bioinformatics. 7:285). In order to improve stability of the gene to be synthesized in "Gene Design", sequences were avoided which promote recombination, such as "Chi" site, Cre-recombinase, bacterial invertase, Dam metilase, Tn3 Resolvase. In additio to these sequences to be avoided, the sequences of start of restriction enzime transcription and translation are also avoided, which are used in clonings and are added in the list of sequences that the program avoids in the synthesis of gene (see Table 3).

**Table 3: List of sequences to be avoided in the design of synthetic genes of the invention**

| **Name** | **Sequence** |
|---|---|
| Bacterial Invertase | AAACCAATTTGG |
| Chi Site | GCTGGTGG |
| Cre Recombinase | TACATAC |
| Cryptic Splice Acceptor | YYYYNTAGG |
| Cryptic Splice Donor | MAGGTRAGT |
| Dam Metilase | GATC |
| Lac Repressor Site | GTGGAATTGTGAGCGATAACAAT |
| Prokaryotic Ribosome Binding Site | AGGAGGTNNNNNDTG |
| Resolvase Tn3 | TGTGYNNTA |
| RNA Destabilizing Sequence | ATTTA |
| Shine-Dalgarno | AGGAGGTN(5,10)DTG |
| Sigma70 Promoter | TTGAC (N)18 TATAAT |

After the generation of sequences, they were subjected to analysis of the positions of rare codons in selected sites, as the CAI (Codon Adaptation Index) using again the ANACONDA program, this time with the codons table of E. coli.

The results were satisfactory with respect to the prediction of expression in E. coli (CAI) and the presence of rare codons, in the chosen sites, trying to simulate the original genes in their respective source organisms (M. *hyopneumoniae* e *P. Abyssi*)*.*

The identification of the DNA sequences designed for each protein is shown in Tables 1 and 2.

### Planning of fusions to be made in exosomo

In the present invention, proteins of high molecular mass are fused to the exosome. However, it was found to be preferable to fuse at the C-terminal extreme, so as to allow the exosome proteins primarily to fold, by the structure of the exosome, both in the N-terminal and in the C-terminal extreme, which are projected out of the complex of the present invention.

In the planning of fusions to modeling the complex of proteins of the present invention, all chosen amino acids sequences were analyzed in the Prot Param program (http://web.expasy.org/protparam). In Table 4, the results of this analysis are showed.

The chosen amino acids sequences for the M. *hyopneumoniae* antigens show a low instability index (I.I.), however, they are stable proteins, except the NrdF protein.

**Table 4: Analysis of physicochemical properties of proteins selected from the amino acids sequence**

| **N° (*)** | **Protein** | **MW (kDa)** | **pl** | **I.I (**)** | **Stability** |
|---|---|---|---|---|---|
| 1 | **NrdF** | 12,3 | 4,14 | 62,12 | Not stable |
| 2 | **HSP** | 26,8 | 4,53 | 35,98 | Stable |
| 3 | **Rrp42** | 30,0 | 4,72 | 37,33 | Stable |
| 4 | **P46** | 40,5 | 5,91 | 29,33 | Stable |
| 5 | **Rrp41** | 27,6 | 6,26 | 38,47 | Stable |
| 6 | **P36** | 34,2 | 7,91 | 25,75 | Stable |
| 7 | **Rrp4** | 29,3 | 9,08 | 23,24 | Stable |
| 8 | **MHP271** | 14,1 | 9,55 | 38,3 | Stable |
| 9 | **P97R1-R2** | 15,0 | 9,65 | 34,07 | Stable |

| | | | | | |
|---|---|---|---|---|---|
| ^{*} Proteins arranged according to its Ip (isoelectric point), in increasing order ^{**}Instability index | | | | | |

Proteins included in the same fusion were mainly chosen for their similarity in Isoelectric Point. Then, the most important antigens were associated with Rrp41 and Rrp42 proteins that make up the hexameric ring of exosome. It was determined that the largest protein (with improved folding and stability) of ***M. hyopneumoniae*** stay directly connected to the exosome proteins, while smaller proteins stay in the C-terminal extreme of the polyproteic fusions:
1. - Rrp4- P36 - MHP271
2. - Rrp41 - P46 - P97R1 R2
3. - Rrp42 - HSP - NrdF

### Panning of synthetic gene to express the "Exomyc" complex of the invention

After establishing the fusions, it was performed planning to group synthetic genes appropriate to encode the segments/fragments included in the Exomyc complex of the invention. For this planning the following criteria were considered:
1. a single sequence to express three fusions (polyproteins) in the same vector.
2. succession of the fusions identical to the sequence of the gene locus of exosome (Figure 8).
3. inserting restriction sites that enables expression of the antigens jointly and separately (for the ELISA tests).
4. inserting restriction sites that enables the fusion of other protein or peptide sequences, to the complex of exosome, in such a way that the Exomyc complex of the invention can be used in the formation of similar complexes, including those in which there are constitutive substitutions or that the complexes are obtained in the synthetic genes that, due to "degeneration" of genetic code, encode complexes like Exomyc, including those with constitutive substitutions.

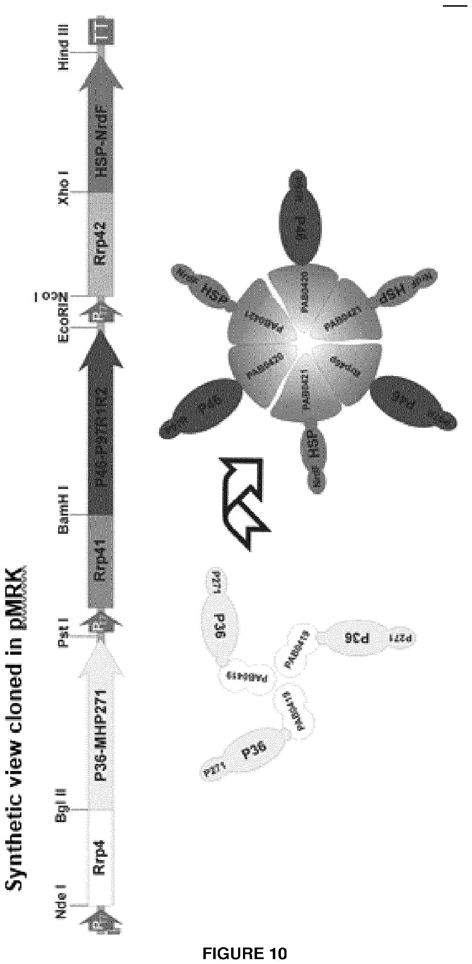

Figure 10 shows the planning result of the synthetic gene of "EXOMYC" complex of the invention, and a representation of encoded polyprotein forming the complex.

The construction shown in Figure 10 is designed for the cloning in the pMRKA vector by the Ndel and Hindlll sites, so that only the first gene (Rrp4) is under promoter control 17 of vector. For genes Rrp41 and Rrp42, sequences were designed containing the promoter 17, similarly to the co-expression vector pETDuet-1 and pACYCDuet-1 (EMD Millipore), but without the operator sequence of operon lac, present in these commercial vectors:
MHP271 stop, Rrp41 Promoter and start (SEQ ID NO:23) 
PstI
TAACTGCAGGCTTAAGTCGAACAGAAAGTAATCGTATTGTACACGGCCGCAACTTCGAAATTAATA
Stop   MHP271

Promotor T7                                                    RBS
CGACTCACTATAGGGAGACCACAACGGTTTCCCATCTTAGTATATTAGTTAAGTATAAGAAGGAGA

TATAAACATGGCA
       M  A  -> Rrp41
P7R1 R2 Stop, Rrp42 promoter and start (SEQ ID NO:24)

EcoRI
TAAGAATTCGCTTAAGTCGAACAGAAAGTAATCGTATTGTACACGGCCGCA
Stop  P97R1R2

Promotor T7
TAATCGAAATTAATACGACTCACTATAGGGAGACCACAACGGTTTCCCATC

RBS                    NcoI
TTAGTATATTAGTTAAGTATAAGAAGGAGATATACCATGGGT
M  G  -> Rrp42

The genes designed for each proteins of Exomyc complex do not contain sequences of translation start or ending, or restriction sites. Similarly to the promoter sequences, small sequences were designed separately to be introduced into the gene of Exomyc complex (SEQ ID NO:25):

NdeI                    BglII
CATATGGCC SEQ ID No: 20 AGATCTGGT SEQ ID No: 19 GGCTCC SEQ ID No: 12
   MetAla                ArgSerGly                GlySer

                                                BamHI
SEQ ID No: 17  SEQ ID No: 23  SEQ ID No: 21 GGATCCCCT SEQ ID No: 13
                                                GlySerPro

                                                   XhoI
GGG SEQ ID No: 16 SEQ ID No: 24 SEQ ID No: 22 CTCGAGGGC SEQ ID No: 18
Gly                                              LeuGluGly

                                          HindIII
SEQ ID No: 14 GGTTCCGGG SEQ ID No: 15 TAA GCTT
              GlySerGly               Stop

The first inserted fragment contains the cleavage site for the Ndel enzyme, the translation initiation codon (highlighted in the square) for the Rrp41 protein (SEQ ID NO:20) and a codon to the Alanine amino acid.

The second fragment contains the cleavage site for the Bglll enzyme to the cloning in C-terminal fusion of Rrp4 protein, and a codon to the amino acid Glycine, before "Hnker P216-B (SEQ ID NO:19). Between this sequence and the P36 protein (SEQ ID NO:12), it was introduced the dipeptide Glycine-Serine, to give greater flexibility to the protein.

After the sequence encoding the Rrp41 protein (SEQ ID NO:21 ), has been introduced the cleavage site for the BamHl enzyme for the cloning in the C-terminal fusion of Rrp41 protein, and the codon for the amino acid Proline, before the sequence of P46 protein (SEQ ID NO:13). Between this sequence and the P97R1 R2 protein (SEQ ID NO:16), it was introduced the Glycine codon, to give greater flexibility to the protein.

After the sequence of Rrp42 protein (SEQ ID NO:22), it was introduced the cleavage site to the Xhol enzyme to cloning in C- terminal fusion of Rrp42 protein, and a codon amino acid Glycine, before the sequence of "Hnkef P216-A (SEQ ID NO:18). Between the HSP protein (SEQ ID NO:14) and the NrdF (SEQ ID NO:15), It was inserted tripeptide (Glycine-Serine-Glycine), to increase the flexibility of the molecule. And, finally, it was placed a translation termination site and the cleavage site for Hindlll.

The complex of Exomyc proteins of the present invention, encoded by synthetic gene of the invention, characterized by the sequence of nucleotides of SEQ ID NO:25, encodes the polyproteins listed in Table 5.

**Table 5: Analysis of physicochemical of encoded polyproteins by gene of Exomyc complex**

| **Polyprotein** | **Composition** | **aa** | **MW (kDa)** | **pL** | **I.I(*)** | **Stability** | **SEQ ID NO:** |
|---|---|---|---|---|---|---|---|
| 1 | **Rrp4-P216B-P36-MHP271** | 737 | 80,5 | 9,05 | 27,88 | Stable | SEQ ID NO: 26 |
| 2 | **Rrp41-P46-97R1 R2** | 765 | 82,7 | 8,81 | 33,31 | Stable | SEQ ID NO: 27 |
| 3 | **Rrp42-P216A-HSP-NrdF** | 657 | 72,6 | 4,53 | 40,59 | Not stable | SEQ ID NO: 28 |

| | | | | | | | |
|---|---|---|---|---|---|---|---|
| instability index | | | | | | | |

To evaluate the immune response stimulated by Exomic complex for individual antigens, it is necessary to separate cloning thereof in fusion with peptides or proteins different from proteins of the *P. abyssi* exosome. For this task, specific primers can be used for insertion of restriction sites by PCR amplification, and taking advantage of the existing sites in the synthetic DNA, as illustrated in Figure 12.

Then, the drawn sequences are provided to this end:
**T7Rev - SEQ ID NO:29**
   5' TGTGGTCTCCCTATAGTGAGTCG 3'
Gen1 BRev (P36 reverse) - SEQ ID NO:30 
PstI
5' GGCTGCAGTTAAATGTTTTTAATCGCATCCTG 3'
Gen1CFor (MHP2171 Forward) - SEQ ID NO:31 
BglII
5' CCAGATCTCCGAAATCCCAAAAATCTG 3'
Gen2AFor (Rrp41 forward) - SEQ ID NO:32 
NdeI
5' AGCATATGGCAGAGAAACCGGAAGG 3'
Gen2BRev (P46 reverse) - SEQ ID NO:33 
EcoRI
5' CCGAATTCTTAAGGGTTGTCCACATTCGCTTTGG 3'
Gen2CFor (P97R1 R2 forward) - SEQ ID NO:34 
BamHI
5' AAGGATCCGCGAAACCGGAGGCAGCGAAACC 3'
Gen3BRev (HSP reverse) - SEQ ID NO:35 
HindIII
5' CCAAGCTTAATCTTGTTTAATCTCCGCGTCG 3'
Gen3CNFor (NrdF Forward) - SEQ ID NO:36

XhoI
5' GGCTCGAGGGGAAACAGGATGAAATCAAAAAATTCAC 3'

The synthetic gene of the present invention, comprising the sequence of nucleotides SEQ ID NO:25, encoding of the complex of Exomyc proteins of the invention has the sequence of nucleotides shown in the attached Sequence Listing and integrating the present description. the coding regions of the segments based on the corresponding antigens of M. *hyopneumoniae* and carrier proteins are listed in Tables 1, 2 and 5.

Considering that it is able to put together the complex shown in Figure 10, we will have three copies of each polyprotein in complex, with a molecular mass of approximately 700 kDa. It is expected that by the size, the Exomyc complex can act as "particle like pathogen" (Rosenthal J.A. et al. (2014) Pathogen-like particles: biomimetic vaccine carriers engineered at the nanoscale. Current Opinion in Biotechnology (Nanobiotechnology · Systems biology) 28:51-58).

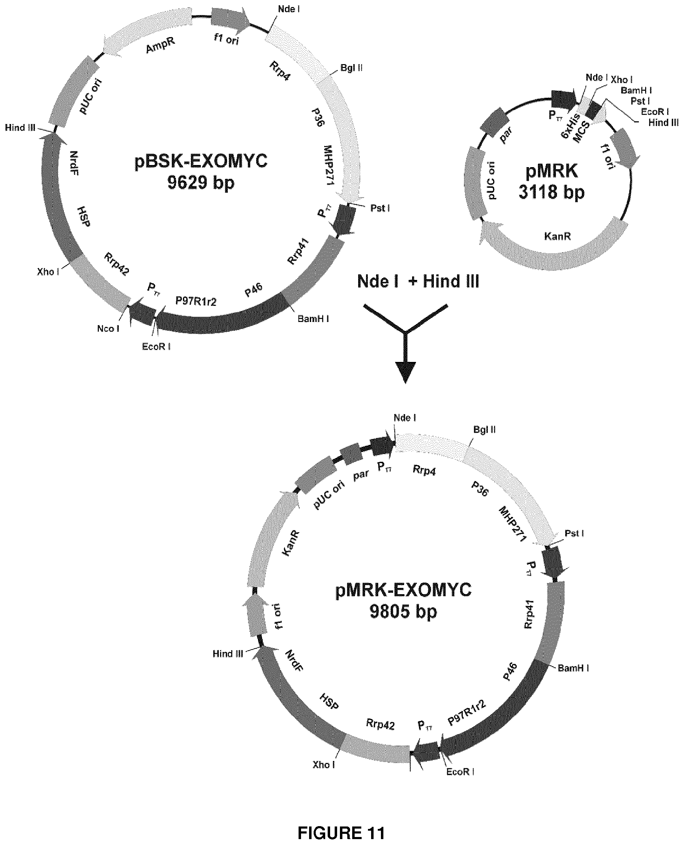

After synthesis of the designed gene, it was cloned into an appropriate vector which contemplates the insertion of a relatively large gene. Numerous expression vectors are commercially available for the construction of encoding synthetic gene of the Exomyc complex of proteins of the invention, such as the vectors of pET series (Novagen) and pRSET (Life Technologies). The person skilled in the art will be able to, from the teachings of the present Invention and from commercially available expression systems, select the appropriate vector for use in the production of the Exomyc complex de proteins of the present invention. More preferably, the synthetic gene of the invention was cloned in the pMRK vector between the Ndel and Hindi 11 sites (Figure 11).

The expression system for industrial production of Exomyc complex de proteins of the invention can be any commercially available system. An example is the *Escherichia* coli expression. system, strains BL21 (DE3) and Rosetta (DE3), available from the company Novagen (see Novagen Catalog 2009/2010, pages 121 e 123, available on the Web in www.merckmillichina.com/promart/library/Novagen-pET-E-coli-expression-system.pdf). With the use of this expression system and an expression vector containing the T7 phage promoter, such vector that carries the synthetic gene of the invention, it is possible to obtain the Exomyc complex of proteins of the invention by fermentation as extensive knowledge of the skilled in the art, why there is no need to provide further details of the conditions and steps employed, which may be determined without the need of many experiments. Examples of plasmids for expression of the recombinant protein of the Invention are those of the pET series, such as pRSET, being employed more preferably the PMK vector as defined herein.

It is widely known in the art of chemistry that certain amino acids substitutions can be made in sequences of proteins without affecting the function or activity thereof. Generally, conservative substitutions of amino acids or similar amino acids substitutions can occur naturally or intentionally without modification of the biological activity, for example, immunogenicity of the protein or fused protein. The similar amino acids can be those with size and/or similar charging properties. For example, aspartate and glutamate and isoleucine and valine are pairs of similar amino acids. The similarity between pairs of amino acids can be evaluated by several ways known by the skilled in the art. For example, Dayhoff et al. (1978) in Atlas of Protein Sequence and Structure, Volume 5, Supplement 3, Chapter 22, pp. 345-352, which is incorporated herein by reference, provides frequency tables for amino acids substitutions that can be used as a measure of similarity. The tables of frequency of Dayhoff et al. are based in comparisons of amino acids sequences to proteins having the same function, from a variety of different evolution sources.

Also known is the fact that the "percentage of sequence identity" for polynucleotides and polypeptides is determined by comparing two sequences aligned for optimal manner over a comparison window, wherein the portion of the sequence of polynucleotides or polypeptides, in the comparison window may comprise additions or deletions when compared to a reference sequence (that does not comprise deletions or additions) for optimal alignment of the two sequences. The percentage is calculated by determining the number of nucleic acid base positions or identical amino acid residue which occurs in both sequences to yield the number of matched positions/coincidents by the total number of positions in the comparison window and multiplying the result by 100 to yield the percentage. Optimal alignment of sequences for comparison may be conducted by computerized implementation of known algorithms (for example, GAP, BESTFIT, FASTA and TFASTA presents in Wisconsin Genetics Software Package, Genetics Computer Group (GCG), 575 Science Dr., Madison, Wis., or BlastN and BlastX available from National Center for Biotechnology Information), or by inspection. Sequences are typically compared using BlastN or BlastX with default parameters.

Substantial identity of polynucleotide sequences means that a polynucleotide or gene comprises a sequence having at least 75% of sequence identity, preferably at least 80% and more preferably at least 90% of sequence identity when compared to a reference sequence. Typically, two polypeptides or proteins are considered substantially identical if at least 40%, preferably at least 60%, more preferably 90% and more preferably 95% are identical sequences or have conservative substitutions. The sequences are preferably compared using GAP with default parameters.

It is important to note that the Exomyc complex of proteins of the present invention may contain conservative substitutions, which allow obtaining analogues of said complex, whose analogues preserving the antigenicity of antigen of *M*. *hyopenumoniae* which were the basis for the assembly of the complex and, however, such analogues are appropriate to the same uses, should therefore be considered as included within the scope of the present invention. Table 6 shows the examples of said substitutions:

**Table 6: Examples of conservative substitutions**

| **Residue** | **Conservative Substitution** |
|---|---|
| Ala | Ser |
| Arg | Lys |
| Asn | Gln, His |
| Asp | Glu |
| Gln | Asn |
| Gly | Pro |
| His | Asn, Gln |
| Leu | Ile, Val |
| Met | Leu, Ile |
| Ser | Thr, Gly |
| Thr | Ser, Val |
| Val | Ile, Leu |

Also included in the present Invention are Exomyc complex proteins fragments of the present Invention that maintain the antigenicity of the full complex as represented by amino acids sequences SEQ ID NO:26, SEQ ID NO:27 and SEQ ID NO:28.

Briefly, the method for producing a polyprotein which corresponds to the fusion and antigenic and stable segments of antigens P36, P46, HSP, NrdF, P97, MHP271 and P216 of *M*. *hyopneumoniae* with segments of carrier proteins Rrp4, Rrp41 and Rrp42 of *P. abyssi,* herein called Exomyc Complex de proteins of the present invention comprises the steps of: (i) inserting a gene fusion in a coding region of a plasmid, preferably the pMRK among the Ndel and Hind III sites; (ii) transforming the host cell, preferably E. coli, with the plasmid; and (iii) expression of the fused heterologous protein from the host cell by methods well known in the art. more particularly, the production method of fused heterologous protein of the present Invention comprises inserting cDNA which is encoded for expression of Exomyc Complex de proteins of the invention, or a fragment thereof, or a conservatively modified variant thereof, in a vector containing the coding information for the production of a carrier protein, preferably the proteins Rrp4, Rrp41 and Rrp42 of the *Pyrococcus abyssi* archaebacterium. After inserting the vector into the expression system, the fused heterologous protein is expressed by the system.

The present invention also refers to an antigenic composition comprising a polyprotein, corresponding to an immunogenic complex of the invention, or a analogue thereof, as active ingredient and pharmaceutically acceptable non-active ingredients.

The composition of the invention can be in the form of a solid, semisolid or liquid preparation. Preferably, the preparation is in the liquid form for oral administration, nasal spray or parenteral solution/suspension. The immunogenic polypeptides/polyproteins forming the Exomyc complex of the invention, active ingredient of the composition of the invention, can be in the neutral form or salts, the latter may be addition salts or salts formed with free carboxyl groups, the important being the fact that the said salts are pharmaceutically acceptable. The skilled in the art are able to, from the Invention disclosed herein and the common knowledge of polypeptides / polyproteins salts, those suitable for being included in the composition of the Invention.

According to the invention, the term "pharmaceutically acceptable vehicle" means one or more non-active ingredients which have the function, in the composition, of carrying the active ingredient until the local where its pharmacological activity is required and should include substances suitable for use in humans and animals and be compatible with the active ingredient of the invention, that is, with the complex of immunogenic polyproteins/polypeptides of the invention. Examples of pharmaceutically acceptable vehicles include saline, buffered saline, dextrose, water, glycerol, sterile isotonic solution, and combinations thereof.

The composition of the present invention can comprise, in addition to Exomyc Complex of polyproteins of the invention and pharmaceutically acceptable vehicle, other non-active ingredients, such as, adjuvants, preservatives, diluents, emulsifiers, stabilizers and other pharmaceutically acceptable ingredients which are employed in immunogenic preparations for human and animal use. Non-limiting examples of adjuvants include incomplete Freund's adjuvant, complete Freund's adjuvant, polydispersed acetylated mannan β-(1,4) linked, adjuvants of polyoxyethylene-polyoxypropylene copolymer, modified lipid adjuvants, adjuvants derived from saponine, dextran sulfate, aluminum hydroxide and aluminum phosphate.

The composition of present invention can be administered to an animal, including man, by means known in the art. For example, the composition may be administered parenterally, for example, subcutaneous, intraperitoneal, or intramuscular, or by oral route or nasal spray and may be administered in one or more daily doses.

The amount of the composition of the invention administred to an animal varies according to the species of animal. For cattle, the amount of Exomyc complex of protein per dose may vary from 50 to 500 micrograms. The dose of the composition of the Invention to be administered to the subject contains the Exomyc Complex of proteins of the invention, or conservatively modified variant or immunogenic fragments thereof, in a amount smaller than 1.0 mg, preferably at most 0.5 mg, more preferably still in the range of 0.2 until 0.4 mg. The skilled in the art, based on the teachings of the present invention may be able to determine by routine testing the amount of the Exomyc Complex of proteins of the invention, or a conservatively modified variant or immunogenic fragments thereof which will be necessary to develop an adequate immune response to said protein by the animal.

The following examples are provided which illustrate and represent specific embodiments of the invention. The following examples should not be construed as limiting the present Invention.

### EXAMPLES

### Example 1: Cloning of synthetic gene of the complex Exomyc in the pMRK- expression vector

The sequence of the designed gene, corresponding to SEQ ID NO:25, encodes sequences SEQ ID NO: 26, SEQ ID NO: 27 and SEQ ID NO 28 which comprise the EXOMYC complex of the present invention.

The synthetic gene was cloned into a vector without the multiple cloning site derived from pBlueScript II SK cut with Sma called pBSK-EXOMYC. The sequence of synthetic gene (SEQ ID NO:25) was confirmed by sequencing by technique known by the skilled in the art.

In one of the embodiments of the present invention, the expression of recombinant proteins was performed in the pMRK expression vector of E. coli. Figure 11 shows the cloning strategy. The synthetic gene was cut from plasmid pBSK-IniOF using the Ndel and Hind III enzymes and cloned in the corresponding sites of pMRK vector. The resulting plasmid was named pMRK-EXOMYC (Figure 11). The successful cloning was verified by restriction analysis, as widely known in the art.

### Example 2: Cloning and expression of M. hyopneumoniae antigens included in the EXOMYC complex

For immunological characterization of vaccination with Exomyc complex, it is necessary to separately obtain the antigen included in the complex. The strategy used in this work is illustrated in Figure 12. Initially, the coding sequences for the polyprotein P36-MHP271, P46-P97R1 R2 and HSP-NrdF were separated from pMRK-Exomyc plasmids, using cuts with the enzymes BgIII-Pstl, BamHI-EcoRI and Xhol-Hindlll, respectively, to be cloned into the respective sites of the pMRK vector.

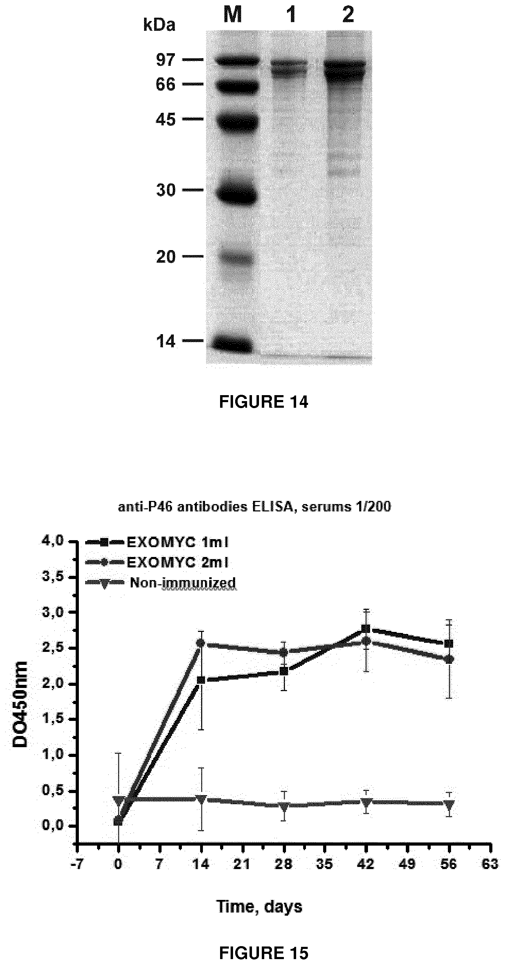

Then, the gene of each antigen was amplified by PCR using a specific primer, and a sequencing primer T7 and T7rev. Thus, 6 plasmides were obtained (pMRK-P36, pMRK-MHP271, pMRK-P46, pMRK-P97R1 R2, pMRK-HSP and pMRK-NrdF) expressing the antigen with fusion of 6xHis. Of these constructions only pMRK-P46 and the pMRK-HSP express the 6xHis-P46 and 6xHis-HSP proteins at a high level and in soluble form. Other antigens were cloned into the pET-SUMO vector, to its fusion with SUMO protein, obtaining the pET-SUMO-P36, pET-SUMO-MHP271, pET-SUMO-P97R1 R2, pET-SUMO-NrdF plasmids. Except the construction pET-SUMO-P36, all expressed in high level and in soluble form, thus obtaining the SUMO-MHP271, SUMO-P97R1 R2 e SUMO-NrdF proteins. Figure 14 shows the result of purification of antigens separately. All proteins except P36 were able to be purified with high degree of purity by affinity chromatography on immobilized metal (IMAC) and gel-filtration. Only the SUMO-MHP271 protein proved to be unstable during storage to 4°C, passing to show a smaller protein (in Figure 12A with asterisk indicates the migration of the main band after purification).

### Example 3: Expression of EXOMYC complex in E. coli cells

The *Escherichia coli* BL21 strain (DE3) transformed with the pMRK-EXOMYC plasmid was grown in 5 ml of LB Kanamycin medium (25 ug/ml) and incubated with the material of a seed bank cryotube (selected product clone, consistent in *Escherichia coli* cells transformed with the pMRK-EXOMYC plasmid, frozen at -80°C in the presence of glycerol). The culture was incubated at 37°C, at 200 rpm, by 16 hours.

One milliliter of culture was inoculated into 25 ml of LB Kanamycin medium (25 ug/ml) (A) and 25 ml of LB Kanamycin medium (25 ug/ml) + Lactose 0,8%, Glycerol 0.5% and MgSO4 1 mM (self-induction). The cultures were incubated at 37°C, at 200 rpm.

Culture A.- After the culture reaches DO₆₀₀ₙₘ of 0.5, it was added ITPG in the final concentration of 1 mM and it was incubated additional 4 hours at 28°C.

Cultura B.- After 4 hours at 37°C, it was incubated at 28°C for additional 6 hours. Aliquots of the cultures were analyzed in gel of SDS-PAGE (Figure 13) before (NI) and after induction (I).

Cells were collected by centrifugation at 6000 rpm for 30 minutes and re-suspended in lysis buffer (50 mM Tris-HCl, pH 8.0; 0.1 % Triton X-100; 0.25% Sarcozyl) and lysed in a high pressure homogenizer (800 bar-80x10³ kPa).

Aliquots of the lysates were incubated at 25 and 100°C for 30 minutes, cooled on ice and centrifuged for 15 minutes 12000 rpm. Both the supernatant and the pellet were analyzed by SDS-PAGE (Figure 13). For this experiment, it was determined that Exomic complex is expressed both in soluble form and in inclusion bodies, and the proportion of soluble recombinant protein is greater in the induction with lactose. The treatment with temperature of 100°C showed that the immunogenic proteins are fused to the proteins of the *P. abyssi* exosome make complex which is not thermostable, the difference of the exosome with or without fusions and smaller confusion.

### Example 4: Production of EXOMYC complex in E. coli cells

The strain of *Escherichia coli* BL21 (DE3) transformed with the pMRK-EXOMYC plasmid was grown in 200 ml of LB Kanamycin medium (25 ug/ml) and incubated with the material of a seed band cryotube. The culture was incubated at 37°C, in 200 rpm, for 16 hours.

The 200 ml of culture were inoculated in 5 liters of MCHD medium (Complex Medium of High Density) in fermenter of 15 liters. The fermentation was performed in steady-fed batch, at 37°C, 30% of dissolved oxygen having glycerol as carbon source. In the DO₆₀₀ₙₘ = 15, feeding of glycerol was replaced by lactose (induction) and the fermentation continued for 12 hours at 28°C.

Cells were collected by centrifugation at 6000 rpm for 30 minutes and re-suspended in lysis buffer (50 mM Tris-HCl, pH 8.0; 0.1 % Triton X- 100; 0,25% Sarcozyl) and lysed in a high pressure homogenizer.

The lysed and homogenized cells were subjected to filtration through a membrane of 0,2 µm (millipore *"end-filtration"* system) at room temperature.

It was added MgCl₂ for final concentration of 5mM and Benzonase 90u/ml (final concentration), and the mixture was incubated at room temperature for 6 hours.

The material treated with Benzonase was concentrated and diafiltrated in the tangential filtration system using a hollow fiber membrane of 500 kDa of MWCO, in the buffer 20 mM Tris-HCl, pH 8.0; 100mM NaCl and 0,1 mM EDTA. The protein retained is sterilized by microfiltration on membrane of 0.2 µm and stored at 4°C. Figure 14 shows the result of co-filtration of polyproteins comprising the Exomyc complex.

### Example 5: Formulation with EXOMYC complex for animals

The complex of Exomyc polyproteins was formulated in double emulsion oil (Montanide ISA 206) with saponin as adjuvant, with the components shown in Table 7.

**Table 7: Composition of Exomyc vaccine, for 1 ml.**

| **Component** | **Amount** |
|---|---|
| EXOMYC | 200 µg |
| Saponine | 1 mg |
| Timerosal | 0.01 in aqueous phase |
| Moontanide ISA 206 | 0.5 ml |

The animals (swines) were grouped in 8. The first group A received a single dose of 1 ml of vaccine (200 µg of antigen), the second group B received a single dose of 2ml of vaccine (400 µg of antigen) and the third group C was not vaccinated. The folowed chronogram was as follows:

| | |
|---|---|
| **Day 0** | Serum collection and first and single dose |
| **Day 14** | Serum collection |
| **Day 28** | Serum collection |
| **Day 42** | Serum collection |
| **Day 56** | Serum collection |
| **Day 140** | Sacrifice |

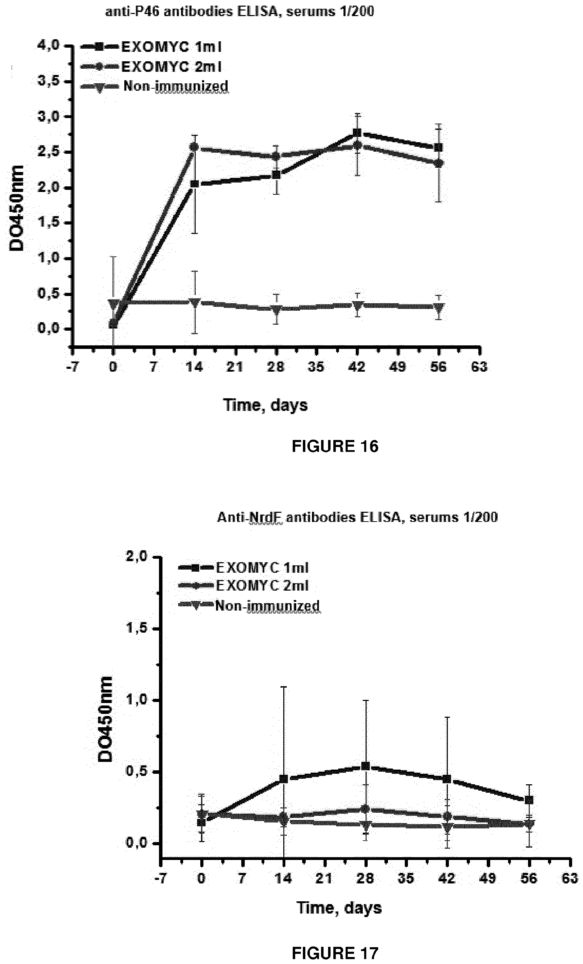

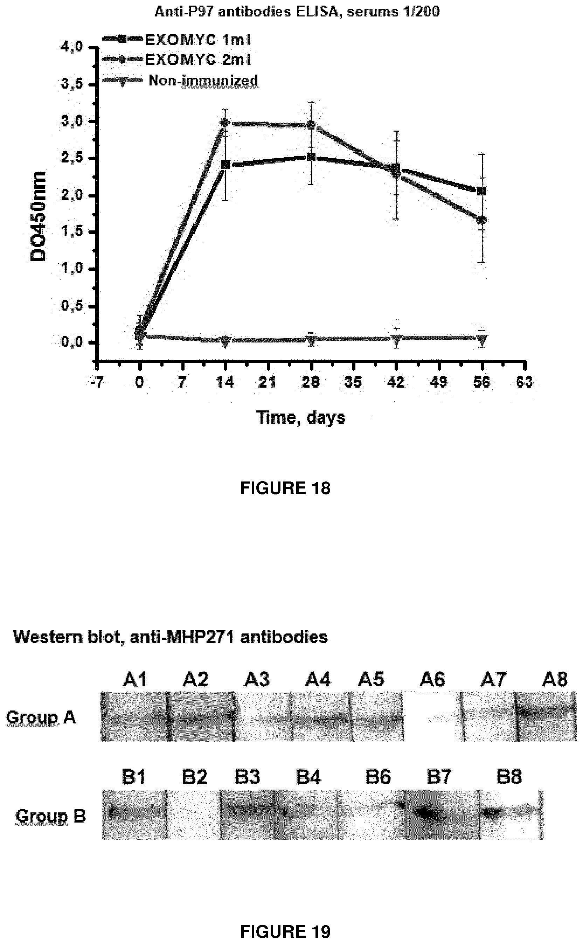

The results of the analyzes by ELISA of the sera to determine antibodies against antigens included in the Exomyc complex, performed with proteins purified in Example 2, are shown in Figure 15 (P46), Figure 16 (HSP), Figure 17 (NrdF) and Figure 18 (P97R1 R2). The response against the MHP217 protein was characterized by Western blot (Figure 19).

These results confirm the presence of the tested proteins in Exomyc complex, that is, it is truly possible to co-express, in soluble form, three polyprotein of large size and the co-purify in a single complex (Exomyc). In addition, the immunization with this complex can induce responses against each of the antigens present on it.

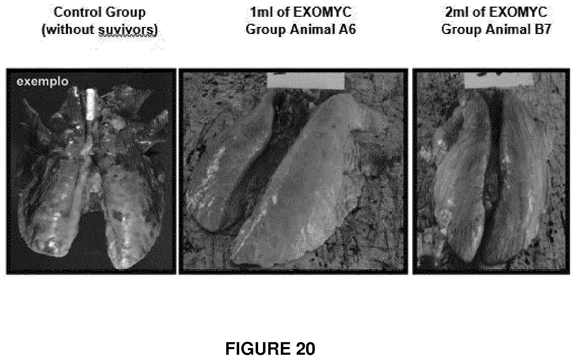

During the experiment, the animals that have not been vaccinated with any vaccine died from diseases associated with PES (Enzzotic Pneumonia of Swine) and CRS (Swine Respiratory Complex). After sacrifice of the animals immunized with 1 or 2 ml of Exomyc vaccine, few lesions were found in the lungs and liver, and some animals in Group B showed no injury (Figure 20). Thus it was proved the effectiveness of the vaccine containing the Exomyc complex.

## Claims

1. Complex of immunogenic polyproteins of *M*. *hyopneumoniae* comprising immunogenic and stable segments specific of P36, P46, HSP (P42), NrdF, P97, MHP271 and P216 antigens of *M*. *hyopneumoniae* fused to segments of *P. abyssi* exosome, wherein the major segments of amino acids derived from *M*. *hyopneumoniae* antigens are directly linked to proteins of said exosome, while smaller segments are at the C-terminal extreme of the fusions.

2. Complex of immunogenic polyproteins according to claim 1, wherein the proteins of *P. abyssi* exosome selected for fusion with the immunogenic and stable segments especific of P36, P46, HSP (P42), NrdF, P97, MHP271 and P216 antigens of *M*. *hyopneumoniae* are Rrp4, Rrp41 and Rrp42, wherein the stability of complex is reached by interaction of said proteins, thus maintaining all antigens in a single proteic complex.

3. Complex of immunogenic polyproteins of *M*. *hyopneumoniae,* comprising an amino acids sequence selected from:
(a) SEQ ID NO:26, SEQ ID NO:27 e SEQ ID NO:28;
(b) conservatively modified variants having at least 85% of identity in relation to amino acids sequences of the sequences SEQ ID NO:26, SEQ ID NO:27 and SEQ ID NO:28, and having substantially the same immunogenic activity thereof;
(c) fragments of sequences SEQ ID NO:26, SEQ ID NO:27 and SEQ ID NO:28, said fragments comprising immunogenic and stable regions of P36, P46, HSP (P42), NrdF, P97, MHP271 and P216 antigens; and
(d) a fusion protein encoded by nucleotide sequence of SEQ ID NO: 25, a conservatively modified variant of said fusion protein or a fragment of said fusion protein having the same immunogenic activity.

4. Complex of immunogenic polyproteins according to claim 3, comprising the amino acids sequences of SEQ ID NO:26, SEQ ID NO:27 and SEQ ID NO:28.

5. Complex of immunogenic polyproteins according to claim 3, comprising a fusion protein encoded by nucleotide sequence of SEQ ID NO:25.

6. Encoding synthetic gene of complex of immunogenic polyproteins of *M*. *hyopneumoniae* comprising encoding nucleotide sequences of immunogenic and stable segments specific of P36, P46, HSP (P42), NrdF, P97, MHP271 and P216 antigens of *M*. *hyopneumoniae* and encoding nucleotide sequences of segments of *P. abyssi* exosome, forming a fusion protein of said immunogenic and stable segments with said segments of *P. abyssi* exosome so that encoding nucleotide sequences of greater segments of amino acid derived from *M*. *hyopneumoniae* antigens are directly linked to nucleotide sequences encoding the proteins of said exosome, while the nucleotide sequences encoding said minor segments of amino acids derived from *M. hyopneumoniae* antigens are in the C-terminal extreme of fusions.

7. Gene according to claim 6, wherein the encoding nucleotide sequences of proteins of *P. abyssi* exosome selected for fusion with the immunogenic and stable segments specific of P36, P46, HSP (P42), NrdF, P97, MHP271 e P216 antigens of *M*. *hyopneumoniae* are the encoding nucleotide sequences of Rrp4, Rrp41 e Rrp42 proteins of *P. abyssi.*

8. Encoding gene of a complex of immunogenic polyproteins of *M*. *hyopneumoniae* comprising a nucleotide sequence selected from group consisting of:
(a) SEQ ID NO:25; e
(b) a nucleotide sequence having at least 75% of identity related to SEQ ID NO:25.

9. Gene according to claim 8, wherein said nucleotide sequence encodes the polyproteins comprising the sequences SEQ ID NO:26 SEQ ID NO:27 e SEQ ID NO:28.

10. Process for obtaining complex of immunogenic polyproteins defined in claims 1 - 5, comprising the steps of:
(i) preparation of an expression system comprising:
(a) the encoding gene of complex of polyproteins, as defined in claims 6-9;
(b) an appropriate expression system, preferably a *Escherichia coli* strain selected from BL21 (DE3) e Rosetta (DE3) strains, and
(c) an expression vector containing the phage promoter T7;
(ii) innoculation of host cells with the expression system prepared in step (i);
(iii) lysing the cells to obtain said complex of immunogenic polyproteins of *M. hyopneumoniae*; and
(iv) purification of said complex obtained in step (iii).

11. Process according to claim 10, wherein the encoding gene of step (i) (a) is formed by DNA amplification from sequences primers: SEQ ID NO:29, SEQ ID NO:30, SEQ ID NO: 31 , SEQ ID NO:32, SEQ ID NO:33, SEQ ID NO:34, SEQ ID NO:35 and SEQ ID NO:36.

12. Process according to claim 10, wherein said expression vector containing the phage promoter T7 of step (i) (c) is selected from the group consisting of plasmid of pET series and the pMRK plasmid.

13. Process according to claim 12, wherein said plasmid of pET series is pRSET.

14. Process according to claim 12, wherein said plasmid is pMRK.

15. Antigenic composition, comprising:
(a) complex of immunogenic polyproteins of M. *hyopneumoniae* as defined in any one of claims 1 -5;
(b) optionally, an adjuvant; and
(c) a pharmaceutically acceptable vehicle.

16. Antigenic composition according to claim 15, wherein said adjuvant is selected from group consisting of: incomplete Freund's adjuvant, the complete Freund's adjuvant, polydisperse acetylated mannan β-(1,4) linked, adjuvants of copolymer of polyoxiethylene-polyoxipropylene, modified lipid adjuvants, adjuvants derived from saponine, dextran sulfate, aluminum hydroxide and aluminum phosphate.

17. Antigenic composition according to claim 15, wherein said pharmaceutically acceptable vehicle includes preservatives, diluents, emulsifiers, stabilizers and other pharmaceutically acceptable ingredients.

18. Use of complex of immunogenic polyproteins of *M*. *hyopneumoniae* as defined in any one claims 1 - 5, in prophylaxis or treatment of infections caused by *Mycoplasma hyopneumoniae.*
